# EUROPEAN PATENT SPECIFICATION

(11) **EP 1 648 445 B1**
(45) Date of publication and mention of the grant of the patent: **03.06.2009**
(21) Application number: 04763610.5
(22) Date of filing: 29.07.2004
(51) Int. Cl.: A61K 31/4045, C07D 209/08, C07D 409/12, C07D 513/04, A61K 31/429, A61P 3/04

(54) **INDOL-5-YL SULFONAMIDE DERIVATIVES, THEIR PREPARATION AND THEIR USE 5-HT-6 AS MODULATORS**
INDOL-5 -SULFONAMID-DERIVATE, IHRE HERSTELLUNG UND VERWENDUNG ALS 5-HT-6 MODULATOREN
DERIVES D'INDOL-5-YL SULFONAMIDE, FABRICATION ET UTILISATION EN TANT QUE MODULATEURS DE 5-HT-6

(30) Priority: 30.07.2003 ES 200301805
(43) Date of publication of application: 26.04.2006
(73) Proprietor: LABORATORIOS DEL DR. ESTEVE, S.A., 08041 Barcelona (ES)
(72) Inventor: MERCE VIDAL, Ramon, E-08021 Barcelona (ES); CODONY SOLER, Xavier, E-08301 Mataro (ES); DORDAL ZUERAS, Alberto, E-08016 Barcelona (ES)
(74) Representative: ABG Patentes, S.L.
(86) International application number: PCT/EP2004/008511
(87) International publication number: WO 2005/013977

(56) References cited:
- EP-A- 0 471 609
- EP-A- 0 815 861
- WO-A-02/060871
- WO-A-03/042175
- US-A- 3 472 870

## Description

The present invention refers to new sulfonamide derivatives, of general formula (Ia, Ib, Ic), 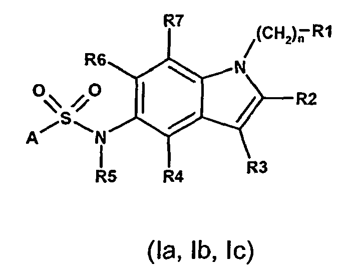 optionally in form of one of their stereoisomers, preferably enantiomers or diastereomers, their racemate, or in form of a mixture of at least two of their stereoisomers, preferably enantiomers or diastereomers, in any mixing ratio, or their salts, preferably the corresponding, physiologically acceptable salts thereof, or corresponding solvates thereof; to the processes for their preparation, to their application in medicaments in human and/or veterinary therapeutics, and to the pharmaceutical compositions containing them.

The new compounds of the present invention may be used in the pharmaceutical industry as intermediates and for preparing medicaments.

The superfamily of serotonin receptors (5-HT) comprises 7 classes (5-HT₁-5-HT₇), which cover 14 human subclasses [D. Hoyer, et al., Neuropharmacology, 1997, 36, 419]. The 5-HT₆ receptor has been the last serotonin receptor identified by molecular cloning in rats [F.J. Monsma, et al., Mol. Pharmacol., 1993, 43, 320; M. Ruat, et al., Biochem. Biophys. Res. Commun., 1993, 193, 268] as well as in humans [R. Kohen, et al., J. Neurochem., 1996, 66, 47]. The compounds with an affinity for the 5-HT₆ receptor are useful in treating different disorders of the Central Nervous System and of the Gastrointestinal system, as well as the irritable bowel syndrome. The compounds with an affinity for the 5-HT₆ receptor are useful for treating anxiety, depression and cognitive memory disorders [M. Yoshioka, et al., Ann. NYAcad. Sci., 1998, 861, 244; A. Bourson, et al., Br. J. Pharmacol., 1998, 125, 1562; D.C. Rogers, et al., Br. J. Pharmacol. Suppl., 1999, 127, 22P; A. Bourson, et al., J. Pharmacol. Exp. Ther., 1995, 274, 173; A.J. Sleight, et al., Behav. Brain Res., 1996, 73, 245; T.A. Branchek, et al., Annu. Rev. Pharmacol. Toxicol., 2000, 40, 319; C. Routledge, et al., Br. J. Pharmacol., 2000, 130, 1606]. It has been shown that the typical and atypical antipsychotics for treating schizophrenia have a high affinity for the 5-HT₆ receptors [B.L. Roth, et al., J. Pharmacol. Exp. Ther., 1994, 268, 1403; C.E. Glatt, et al., Mol. Med., 1995, 1, 398; F.J. Mosma, et al., Mol. Pharmacol., 1993, 43, 320; T. Shinkai, et al, Am. J. Med. Genet., 1999, 88, 120]. The compounds with an affinity for the 5-HT₆ receptor are useful for treating infantile hyperkinesia (ADHD, attention deficit /hyperactivity disorder) [W.D. Hirst, et al., Br. J. Pharmacol., 2000, 130, 1597; C. Gérard, et al., Brain Research, 1997, 746, 207; M.R. Pranzatelli, Drugs of Today, 1997, 33, 379].
Patent application WO 01/32646 discloses sulfonamides derived from bicycles, whereby each of the rings is 6-membered, aromatic or heteroaromatic rings with 5-HT₆ receptor antagonist activity.
Patent application EP 0 733 628 discloses sulfonamides derived from indole with 5-HT_{1F} receptor antagonist activity, useful for the treatment of migraines.

Furthermore, it has been shown that the 5-HT₆ receptor plays a role in the ingestion of food [Neuropharmacology, 41, 2001, 210-219].

Eating disorders, particularly obesity, are a serious and increasingly frequent threat for the health of persons from all age groups, since they increase the risk of developing other serious and even mortal diseases, preferably diabetes and coronary artery diseases.

Therefore, an object of the present invention was to provide new compounds, particularly suitable as active substances in medicaments, preferably in medicaments for 5-HT₆ receptor regulation, for the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes caused by obesity, for the prophylaxis and/or treatment of gastrointestinal tract disorders, preferably irritable bowel syndrome, for cognitive enhancement, for the prophylaxis and/or treatment of disorders of the central nervous system, anxiety, panic disorders, depression, bipolar disorders, cognitive memory disorders, senile dementia processes, preferably Alzheimer's disease, Parkinson's disease, Huntington's disease and Multiple Sclerosis, schizophrenia, psychosis or infantile hyperkinesia (ADHD, attention deficit / hyperactivity disorder), and other disorders mediated by the 5-HT₆ serotonin receptor in humans and/or in animals, preferably in mammals, more preferably in humans.

It has been found that the indol-5-yl sulfonamide compounds of general formulas (la, Ib, Ic) described below show an affinity for the 5-HT₆ receptor. These compounds are therefore suitable for the manufacture of a medicament for the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes caused by obesity, for the prophylaxis and/or treatment of gastrointestinal tract disorders, preferably irritable bowel syndrome, for cognitive enhancement, for the prophylaxis and/or treatment of disorders of the central nervous system, anxiety, panic disorders, depression, bipolar disorders, cognitive memory disorders, senile dementia processes, neurodegenerative disorders, preferably Alzheimer's disease, Parkinson's disease, Huntington's disease and Multiple Sclerosis, schizophrenia, psychosis or infantile hyperkinesia (ADHD, attention deficit / hyperactivity disorder) and other disorders mediated by the 5-HT₆ serotonin receptor in mammals, including humans. These compounds are also suitable for the preparation of a medicament for cognitive enhancement.

Thus, one aspect of the present invention are compounds of general formula (Ia), 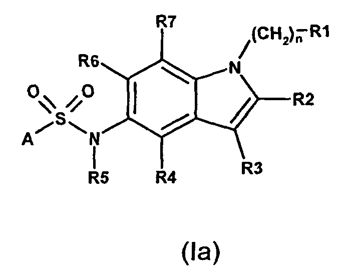 wherein
R¹ represents a -NR⁸R⁹ radical or a saturated or unsaturated, optionally at least mono-substituted cycloaliphatic radical, which may optionally contain at least one heteroatom as a ring member and/or which may be condensed with a saturated or unsaturated, optionally at least mono-substituted mono- or bicyclic cycloaliphatic ring system, which may optionally contain at least one heteroatom as a ring member,
R², R³, R⁴, R⁶ and R⁷, identical or different, each represent hydrogen, halogen, nitro, alkoxy, cyano, a saturated or unsaturated, linear or branched, optionally at least mono-substituted aliphatic radical or an optionally at least mono-substituted phenyl or an optionally at least mono-substituted heteroaryl radical,
R⁵ represents hydrogen or a saturated or unsaturated, linear or branched, optionally at least mono-substituted aliphatic radical,
R⁸ and R⁹, identical or different, each represent hydrogen or a saturated or unsaturated, linear or branched, optionally at least mono-substituted aliphatic radical,
with the proviso that R⁸ and R⁹ are not hydrogen at the same time, and if one of them, R⁸ and R⁹, represents a saturated or unsaturated, linear or branched, optionally at least mono-substituted C₁-C₄ aliphatic radical, the other one represents a saturated or unsaturated, linear or branched, optionally at least mono-substituted aliphatic radical with at least five carbon atoms, or
R⁸ and R⁹ together with the bridging nitrogen atom form a saturated or unsaturated, optionally at least mono-substituted heterocyclic ring, which may contain at least one additional heteroatom as a ring member and/or which may be condensed with a saturated or unsaturated, optionally at least mono-substituted, mono- or bicyclic cycloaliphatic ring system which may optionally contain at least one heteroatom as a ring member,
A represents an optionally at least mono-substituted mono- or polycyclic aromatic ring system, which may be bonded via an optionally at least mono-substituted alkylene, alkenylene or alkynylene group and/or which may contain at least one heteroatom as a ring member in one or more of its rings
and
n is 0, 1, 2, 3 or 4;
optionally in form of one of its stereoisomers, preferably enantiomers or diastereomers, its racemate or in form of a mixture of at least two of its stereoisomers, preferably enantiomers or diastereomers, in any mixing ratio, or a salt thereof, preferably a corresponding, physiologically acceptable salt thereof, or a corresponding solvate thereof.

Another aspect of the present invention are compounds of the general formula (Ib) 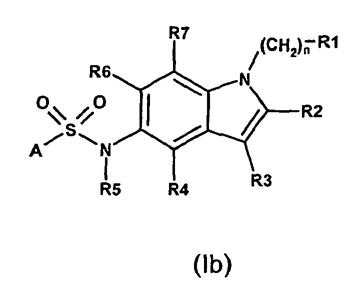 wherein
R¹ represents a -NR⁸R⁹ radical,
R², R³, R⁴, R⁶ and R⁷, identical or different, each represent hydrogen, halogen, nitro, alkoxy, cyano, a saturated or unsaturated, optionally at least mono-substituted, linear or branched aliphatic radical, or an optionally at least mono-substituted phenyl radical or an optionally at least mono-substituted heteroaryl radical,
R⁵ represents hydrogen or a saturated or unsaturated, linear or branched, optionally at least mono-substituted aliphatic radical,
R⁸ and R⁹, identical or different, each represent hydrogen or a saturated or unsaturated, linear or branched, optionally at least mono-substituted, C₁-C₄ aliphatic radical,
A represents an optionally at least mono-substituted mono- or polycyclic aromatic ring system, which may be bonded via an optionally at least mono-substituted alkylene, alkenylene or alkynylene group and/or which may contain at least one heteroatom as a ring member in one or more of its rings,
and n is 0, 1, 2, 3 or 4;
optionally in form of one of its stereoisomers, preferably enantiomers or diastereomers, its racemate or in form of a mixture of at least two of its stereoisomers, preferably enantiomers or diastereomers, in any mixing ratio, or a salt thereof, preferably a corresponding, physiologically acceptable salt thereof, or a corresponding solvate thereof.

Yet, another aspect of the present invention are compounds of general formula (Ic), 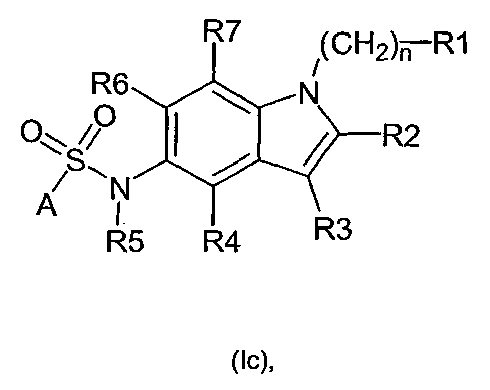 wherein
R¹ represents a -NR⁸R⁹ radical or a saturated or unsaturated, optionally at least mono-substituted, cycloaliphatic radical, which may optionally contain at least one heteroatom as a ring member and/or which may be condensed with a saturated or unsaturated, optionally at least mono-substituted mono- or bicyclic cycloaliphatic ring system, which may optionally contain at least one heteroatom as a ring member,
R², R³, R⁴, R⁶ and R⁷, identical or different, each represent hydrogen, halogen, nitro, alkoxy, cyano, a saturated or unsaturated, linear or branched, optionally at least mono-substituted aliphatic radical or an optionally at least mono-substituted phenyl or an optionally at least mono-substituted heteroaryl radical, R⁵ represents hydrogen or a saturated or unsaturated, linear or branched, optionally at least mono-substituted aliphatic radical,
R⁸ and R⁹, identical or different, each represent hydrogen or a saturated or unsaturated, linear or branched, optionally at least mono-substituted aliphatic radical,
or
R⁸ and R⁹ together with the bridging nitrogen atom form a saturated or unsaturated, optionally at least mono-substituted heterocyclic ring, which may contain at least one additional heteroatom as a ring member and/or which may be condensed with a saturated or unsaturated, optionally at least mono-substituted, mono- or bicyclic cycloaliphatic ring system which may optionally contain at least one heteroatom as a ring member,
A represents an optionally at least mono-substituted mono- or polycyclic aromatic ring system, which may be bonded via an optionally at least mono-substituted alkylene, alkenylene or alkynylene group and/or which may contain at least one heteroatom as a ring member in one or more of its rings
and
n is 0, 1, 2, 3 or 4;
optionally in form of one of its stereoisomers, preferably enantiomers or diastereomers, its racemate or in form of a mixture of at least two of its stereoisomers, preferably enantiomers or diastereomers, in any mixing ratio, or a salt thereof, preferably a corresponding, physiologically acceptable salt thereof, or a corresponding solvate thereof.

If one or more of the moieties R²-R⁹ represent a saturated or unsaturated aliphatic radical, that is, an alkyl, alkenyl or alkynyl radical which is substituted by one or more substituents, each one of these substituents may preferably be chosen, unless otherwise defined, from the group consisting of hydroxy, fluorine, chlorine, bromine and trifluoromethyl.

If R¹ is a saturated or unsaturated, optionally at least one heteroatom as a ring member containing cycloaliphatic radical, which is substituted by one or more substituents and/or is condensed with a saturated or unsaturated, optionally at least one heteroatom as a ring member containing mono- or bicyclic cycloaliphatic ring system, which is substituted by one or more substituents, each one of these substituents may preferably be chosen, unless otherwise defined, from the group consisting of hydroxy, fluorine, chlorine, bromine, linear or branched C₁-C₆ alkyl, linear or branched C₁-C₆ alkoxy, linear or branched C₁-C₆ perfluoroalkyl, linear or branched C₁-C₆ perfluoroalkoxy and benzyl, preferably from the group consisting of linear or branched C₁-C₆ alkyl and benzyl.

The heteroatoms of said cycloaliphatic radical and/or of said mono- or bicyclic cycloaliphatic ring may, independently from one another, preferably be chosen from the group consisting of nitrogen, sulphur and oxygen, more preferably nitrogen is chosen as a heteroatom.

Said cycloaliphatic radical may contain 0, 1, 2 or 3 heteroatoms chosen from the above mentioned group, preferably it contains 0, 1 or 2 heteroatoms chosen from the above mentioned group.

If R⁸ and R⁹ together with the bridging nitrogen atom form a saturated or unsaturated, optionally at least mono-substituted heterocyclic ring, which may contain at least one further heteroatom as a ring member and/or which is condensed with a saturated or unsaturated mono- or bicyclic cycloaliphatic ring system, which may contain at least one heteroatom as a ring member and/or which is substituted by one or more substituents, each one of these substituents may preferably be chosen, unless otherwise defined, from the group consisting of hydroxy, fluorine, chlorine, bromine, linear or branched C₁-C₆ alkyl, linear or branched C₁-C₆ alkoxy, linear or branched C₁-C₆ perfluoroalkyl, linear or branched C₁-C₆ perfluoroalkoxy and benzyl, preferably from the group consisting of linear or branched C₁-C₆ alkyl and benzyl.

If the heterocyclic ring contains one or more additional heteroatoms, and/or if one or both rings of the mono- or bicyclic ring system contain one or more heteroatoms, these heteroatoms may, independently from one another, preferably be chosen from the group consisting of nitrogen, sulphur and oxygen, more preferably nitrogen is chosen as a heteroatom.

Said heterocyclic ring may contain 0, 1, 2 or 3 additional heteroatoms chosen from the above mentioned group, preferably it contains 0 or 1 heteroatoms chosen from the above mentioned group.

If A is a mono- or polycyclic aromatic ring system which may be bonded via an alkylene, alkenylene or alkynylene group and/or which may contain at least one heteroatom as a ring member and/or which may be substituted by one or more substituents, each one of these substituents may preferably be chosen from the group consisting of nitro, -O-phenyl, -O-C₁₋₆ alkyl, -C(=O)-C₁₋₆ alkyl, hydroxy, halogen, linear or branched C₁-C₆ alkyl, linear or branched C₁-C₆ alkoxy, linear or branched C₁-C₆ perfluoroalkyl, linear or branched C₁-C₆ perfluoroalkoxy, an optionally at least mono-substituted phenyl radical and 5- or 6-membered heteroaryl, more preferably from the group consisting of nitro, -O-phenyl, -C(=O)-C₁₋₆ alkyl, linear or branched C₁-C₆ alkoxy, halogen, linear or branched C₁-C₆ alkyl, an optionally at least mono-substituted phenyl radical and 5- or 6-membered heteroaryl, even more preferably from the group consisting of nitro, -O-phenyl, -O-CH₃, -C(=O)-CH₃, fluorine, chlorine, bromine, linear or branched C₁-C₆ alkyl, an optionally at least mono-substituted phenyl radical and 5- or 6-membered heteroaryl.

If one or more of the rings of the mono- or polycyclic aromatic ring system contain one or more heteroatoms, these heteroatoms - like the heteroatoms of a previously mentioned 5- or 6-membered heteroaryl radical - may preferably be chosen from the group consisting of nitrogen, sulphur and oxygen.

If the previously mentioned phenyl radical is itself substituted by one or more substituents, each one of these substituents may preferably be chosen from the group consisting of fluorine, chlorine, bromine, linear or branched C₁-C₆ alkyl, linear or branched C₁-C₆ alkoxy, linear or branched C₁-C₆ alkylthio, trifluoromethyl radical, cyano radical and a -NR¹²R¹³ radical, wherein R¹² and R¹³, identical or different, represent hydrogen or a linear or branched C₁-C₆ alkyl.

If the previously mentioned alkylene, alkenylene or alkynylene group is substituted by one or more substituents, each of these substituents may preferably be chosen from the group consisting of hydroxy, halogen, linear or branched C₁-C₆ alkyl, linear or branched C₁-C₆ alkoxy, linear or branched C₁-C₆ perfluoroalkyl, linear or branched C₁-C₆ perfluoroalkoxy or an optionally at least mono-substituted phenyl radical.

If said phenyl radical is itself substituted by one or more substituents, each one of these substituents may preferably be chosen from the group consisting of fluorine, chlorine, bromine, linear or branched C₁-C₆ alkyl, linear or branched C₁-C₆ alkoxy, linear or branched C₁-C₆ alkylthio, trifluoromethyl radical, cyano radical and a -NR¹²R¹³ radical, wherein R¹² and R¹³, identical or different, represent hydrogen or a linear or branched C₁-C₆ alkyl.

If one or more of the substituents R², R³, R⁴, R⁶ and R⁷ represents an alcoxy radical, said radical may have 1 to 6, preferably 1 to 3 carbon atoms.

Those skilled in the art understand that the term "condensed" indicates that the condensed rings share more than one atom. The terms "annulated" or "fused" may also be used for this type of bonding.
Sulfonamide derivatives of general formula (Ia) are preferred, wherein R¹ represents an -NR⁸R⁹ radical or a saturated or unsaturated, optionally at least mono-substituted 5- or 6-membered cycloaliphatic radical which may optionally contain at least one heteroatom as a ring member and/or which may be condensed with a saturated or unsaturated, optionally at least mono-substituted mono- or bicyclic cycloaliphatic ring system, which may optionally contain at least one heteroatom as a ring member, whereby the rings of the ring system are 5- or 6-membered,
more preferably R¹ represents an -NR⁸R⁹ radical or a radical chosen from the group consisting of 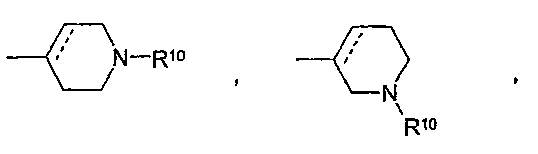 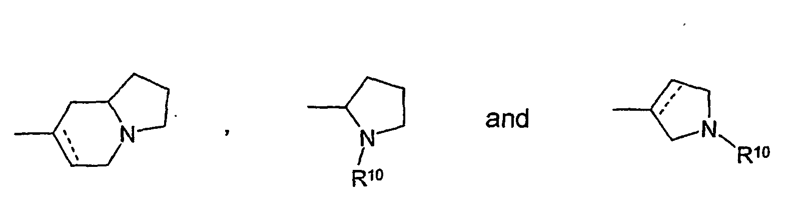 wherein, if present, the dotted line is an optional chemical bond, and R¹⁰ represents hydrogen, a linear or branched C₁-C₆ alkyl radical or a benzyl radical, preferably hydrogen or a C₁-C₂ alkyl radical and R² to R⁹, A and n are defined as above.

Sulfonamide derivatives of general formula (Ia) are also preferred, wherein R², R³, R⁴, R⁶ and R⁷, identical or different, each represent hydrogen, a linear or branched, optionally at least mono-substituted C₁-C₆ alkyl radical, a linear or branched, optionally at least mono-substituted C₂-C₆ alkenyl radical or a linear or branched, optionally at least mono-substituted C₂-C₆ alkynyl radical,
more preferably R², R³, R⁴, R⁶ and R⁷, identical or different, each represent hydrogen or a linear or branched, optionally at least mono-substituted C₁₋C₆ alkyl radical,
even more preferably R², R³, R⁴, R⁶ and R⁷ each represent hydrogen or a C₁₋₂ alkyl radical and R¹, R⁵, R⁸, R⁹, A and n are defined as above.

The use of sulfonamide derivatives of general formula (Ia) is also preferred, wherein R⁵ represents hydrogen, a linear or branched, optionally at least mono-substituted C₁-C₆ alkyl radical, a linear or branched, optionally at least mono-substituted C₂-C₆ alkenyl radical, a linear or branched, optionally at least mono-substituted C₂-C₆ alkynyl radical,
more preferably R⁵ represents hydrogen or a linear or branched, optionally at least mono-substituted C₁-C₆ alkyl radical,
even more preferably R⁵ represents hydrogen or a C₁-C₂ alkyl radical and R¹-R⁴, R⁶-R⁹, A and n are defined as above.

Furthermore, sulfonamide derivatives of general formula (Ia) are also preferred, wherein R⁸ and R⁹, identical or different, each represent a linear or branched, optionally at least mono-substituted C₁-C₁₀ alkyl radical, a linear or branched, optionally at least mono-substituted C₂-C₁₀ alkenyl radical, a linear or branched, optionally at least mono-substituted C₂-C₁₀ alkynyl radical,

with the proviso that R⁸ and R⁹ do not represent hydrogen at the same time, and if one of them, R⁸ and R⁹, represents a saturated or unsaturated, linear or branched, optionally at least mono-substituted C₁-C₄ aliphatic radical, the other one represents a saturated or unsaturated, linear or branched, optionally at least mono-substituted aliphatic radical, with at least five carbon atoms, or
R⁸ and R⁹ together with the bridging nitrogen atom form a saturated or unsaturated, optionally at least mono-substituted 5- or 6-membered heterocyclic ring which may contain at least one additional heteroatom as a ring member and/or which may be condensed with a saturated or unsaturated, optionally at least mono-substituted mono- or bicyclic cycloaliphatic ring system, which may optionally contain at least one heteroatom as a ring member, whereby the rings of the ring system are 5- 6- or 7-membered and R¹-R⁷, A and n are defined as above.

Particularly preferred is the use of sulfonamide derivatives of general formula (Ia), wherein R⁸ and R⁹, identical or different, each represent hydrogen or a linear or branched C₁-C₁₀ alkyl radical,
with the proviso that R⁸ and R⁹ do not represent hydrogen at the same time, and if one of them, R⁸ and R⁹, represents a saturated or unsaturated, linear or branched, optionally at least mono-substituted C₁-C₄ aliphatic radical, the other one represents a saturated or unsaturated, linear or branched, optionally at least mono-substituted aliphatic radical, with at least five carbon atoms, or
R⁸ and R⁹ together with the nitrogen atom bridge form a radical chosen from the group consisting of 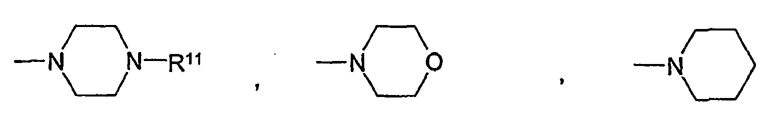 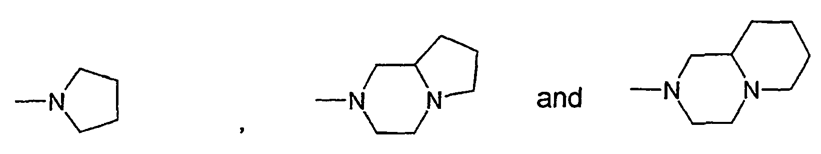 wherein R¹¹, if present, represents hydrogen, a linear or branched C₁-C₆ alkyl radical or a benzyl radical, preferably hydrogen or a C₁-C₂ alkyl radical, and R¹-R⁹, A and n are defined as above.

Furthermore, sulfonamide derivatives of general formula (Ia) are preferred, wherein A represents an optionally at least mono-substituted mono- or polycyclic aromatic ring system, wherein the ring(s) is/are 5- or 6-membered, which may be bonded via an optionally at least mono-substituted C₁₋C₆ alkylene group, an optionally at least mono-substituted C₂₋C₆ alkenylene group or an optionally at least mono-substituted C₂₋C₆ alkynylene group and/or wherein the ring(s) may contain at least one heteroatom as a ring member,
preferably A represents an optionally at least mono-substituted mono- or polycyclic aromatic ring system, wherein the ring(s) is/are 5- or 6-membered and wherein one or more of the rings contain at least one heteroatom,
or a radical chosen from the group consisting of 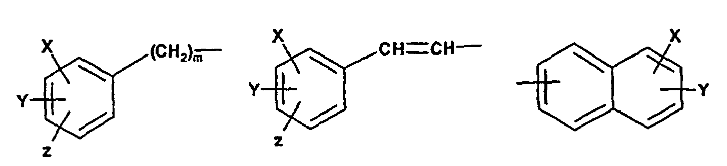 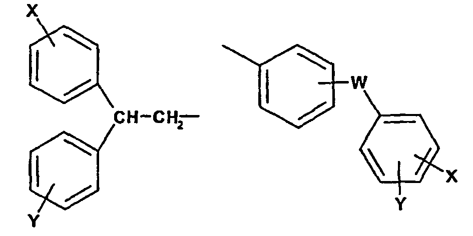 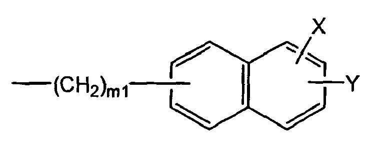 wherein X, Y, Z, independently from one another, each represent a radical selected from the group consisting of hydrogen, fluorine, chlorine, bromine, nitro, acetyl, linear or branched C₁-C₆ alkyl, linear or branched C₁-C₆ alkoxy, linear or branched C₁-C₆ alkylthio, a trifluoromethyl radical, a cyano radical and a -NR¹²R¹³ radical,
wherein R¹² and R¹³, identical or different, each represent hydrogen or linear or branched C₁-C₆ alkyl,
W represents a single chemical bond between the two rings, a CH₂, O, S group or a NR¹⁴ radical,
wherein R¹⁴ is hydrogen or a linear or branched C₁-C₆ alkyl,
m is 0, 1, 2, 3 or 4 and
m1 is 1 or 2, preferably 2, and R¹-R⁹ and n are defined as above.

Furthermore, sulfonamide derivatives of general formula (Ia) are preferred, wherein A represents an optionally at least mono-substituted mono- or polycyclic aromatic ring system, wherein the ring(s) is/are 5- or 6-membered, which may be bonded via an optionally at least mono-substituted C₁₋C₆ alkylene group, an optionally at least mono-substituted C₂₋C₆ alkenylene group or an optionally at least mono-substituted C₂₋C₆ alkynylene group, and/or wherein the ring(s) may contain at least one heteroatom as a ring member,
more preferably A represents an optionally at least mono-substituted mono- or polycyclic aromatic ring system, wherein the ring(s) is/are 5- or 6-membered and wherein one or more of the rings contain at least one heteroatom, or a radical chosen from the group consisting of 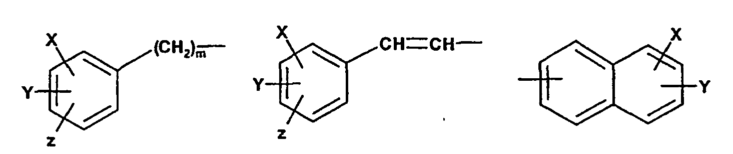 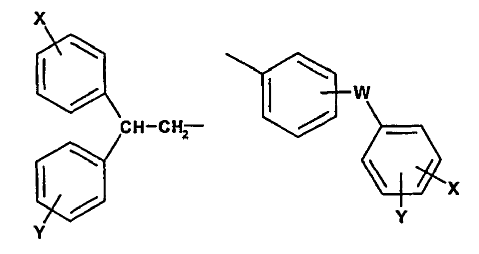 wherein X, Y, Z, independently from one another, each represent a radical selected from the group consisting of hydrogen, fluorine, chlorine, bromine, linear or branched C₁-C₆ alkyl, linear or branched C₁-C₆ alkoxy, linear or branched C₁-C₆ alkylthio, a trifluoromethyl radical, a cyano radical and a - NR¹²R¹³ radical,
wherein R¹² and R¹³, identical or different, each represent hydrogen or linear or branched C₁-C₆ alkyl,
W represents a single chemical bond between the two rings, a CH₂, O, S group or a NR¹⁴ radical,
wherein R¹⁴ is hydrogen or a linear or branched C₁-C₆ alkyl,
and
m is 0, 1, 2, 3 or 4.
and R¹-R⁹ and n are defined as above.

Furthermore, sulfonamide derivatives of general formula (Ia) are preferred, wherein A represents a heteroaryl radical selected from the group consisting of quinolinyl, benzo[b]thiophenyl, benzo[1,2,5]thiadiazolyl, thiophenyl and imidazo[2,1-b]thiazolyl which may be substituted by 1, 2 or 3 substituents selected from the group consisting of fluorine, bromine, chlorine, methyl, phenyl, nitro, -C(=O)-CH₃, -O-CH₃ and -O-phenyl and/or which may be bonded via a C₁₋₂ alkylene group or a C₂ alkenylene group,
or a radical chosen from the group consisting of 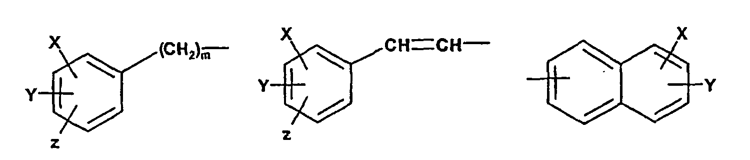 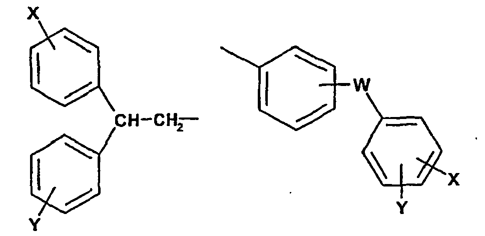 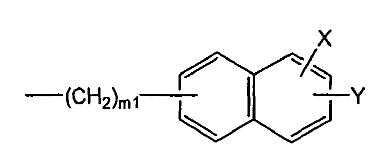 wherein X, Y, Z, independently from one another, each represent a radical selected from the group consisting of hydrogen, fluorine, chlorine, bromine, nitro, acetyl, linear or branched C₁-C₆ alkyl, linear or branched C₁-C₆ alkoxy, linear or branched C₁-C₆ alkylthio, a trifluoromethyl radical, a cyano radical and a -NR¹²R¹³ radical,
wherein R¹² and R¹³, identical or different, each represent hydrogen or linear or branched C₁-C₆ alkyl,
W represents a single chemical bond between the two rings, a CH₂, O, S group or a NR¹⁴ radical,
wherein R¹⁴ is hydrogen or a linear or branched C₁-C₆ alkyl,
m is 0, 1, 2, 3 or 4 and
m1 is 1 or 2, preferably 2, and R¹-R⁹ and n are defined as above.

Furthermore sulfonamide derivatives of general formula (Ia) are preferred, wherein n is 0, 1, 2, 3 or 4; preferably n is 1, 2 or 3; more preferably n is 2 or 3 and R¹ to R⁹ and A are defined as above.

Those most preferred compounds of general formula (Ia) are selected from the group consisting of
[16] N-[1-(2-pyrrolidine-1-yl-ethyl)-1H-indole-5-yl]-naphthalene-2-sulfonamide,
[17] N-[1-(2-pyrrolidine-1-yl-ethyl)-1H-indole-5-yl]-naphthalene-1-sulfonamide,
[18] N-[1-(2-pyrrolidine-1-yl-ethyl)-1H-indole-5-yl]-5-chloro-3-methylbenzo[b]thiophene-2-sulfonamide,
[28] N-[1-(2-pyrrolidine-1-yl-ethyl)-1H-indole-5-yl]-]-6-chloroimidazo[2,1-b]thiazole-5-sulfonamide,
[43] 5-chloro-3-methyl-N-(1-(3-(piperidin-1-yl)propyl)-1H-indol-5-yl)benzo[b]thiophene-2-sulfonamide,
[44] N-(1-(3-(piperidin-1-yl)propyl)-1H-indol-5-yl)naphthalene-2-suffonamide,
[45] N-(1-(3-(piperidin-1-yl)propyl)-1H-indol-5-yl)naphthalene-1-sulfonamide,
[46] 6-chloro-N-(1-(3-piperidin-1-yl)propyl)-1H-indol-5-yl)imidazo[2,1-b]thiazole-5-sulfonamide,
[47] 4-phenyl-N-(1-(3-(piperidin-1-yl)propyl)-1H-indol-5-yl)benzenesulfonamide,
[48] 2-(naphth-1-yl)-N-(1-(3-(piperidin-1-yl)propyl)-1H-indol-5-yl)ethanesulfonamide,
[49] 4-phenoxy-N-(1-(3-(piperidin-1-yl)propyl)-1H-indol-5-yl)benzenesulfonamide,
[50] 3,5-dichloro-N-(1-(3-(piperidin-1-yl)propyl)-1H-indol-5-yl)benzenesulfonylamide,
[51] 4,5-dichloro-N-(1-(3-(piperidin-1-yl)propyl)-1H-indol-5-yl)thiophene-2-sulfonamide and
[52] 5-chloro-N-(1-(3-(piperidin-1-yl)propyl)-1H-indol-5-yl)naphthalene-1-sulfonamide,
and their corresponding salts and solvates.

Furthermore, sulfonamide derivatives of general formula (Ib) are also preferred, wherein R², R³, R⁴, R⁶ and R⁷, identical or different, each represent hydrogen, a linear or branched, optionally at least mono-substituted C₁₋C₆ alkyl radical, a linear or branched, optionally at least mono-substituted C₂₋C₆ alkenyl radical, or a linear or branched, optionally at least mono-substituted C₂₋C₆ alkynyl radical,
more preferably R², R³, R⁴, R⁶ and R⁷, identical or different, each represent hydrogen or a linear or branched, optionally at least mono-substituted C₁₋C₆ alkyl radical,
even more preferably R², R³, R⁴, R⁶ and R⁷ each represent hydrogen or an C₁₋₂ alkyl radical and R¹, R⁵, R⁸, R⁹, A and n are defined as above.

Sulfonamide derivatives of general formula (Ib) are also preferred, wherein R⁵ hydrogen, a linear or branched, optionally at least mono-substituted C₁-C₆ alkyl radical, a linear or branched, optionally at least mono-substituted C₂-C₆ alkenyl radical or a linear or branched, optionally at least mono-substituted C₂-C₆ alkynyl radical,
preferably that R⁵ represents hydrogen or a linear or branched, optionally at least mono-substituted C₁-C₆ alkyl radical,
even more preferably R⁵ represents hydrogen or a C₁-C₂ alkyl radical and R¹-R⁴, R⁶-R⁹, A and n are defined as above.

Furthermore, sulfonamide derivatives of general formula (Ib) are also preferred, wherein R⁸ and R⁹, identical or different, each represent hydrogen or a linear or branched, optionally at least mono-substituted C₁-C₄ alkyl radical,
with the proviso that R⁸ and R⁹ are not hydrogen at the same time and R¹-R⁷, A and n are defined as above.

Particularly preferred are sulfonamide derivatives of general formula (Ib) wherein R⁸ and R⁹, identical or different, each represent hydrogen or a C₁-C₂ alkyl radical,
with the proviso that R⁸ and R⁹ are not hydrogen at the same time, and R¹-R⁷, A and n are defined as above.

Furthermore, sulfonamide derivatives of general formula (Ib) are preferred, wherein A represents an optionally at least mono-substituted mono- or polycyclic aromatic ring system, wherein the ring(s) is/are 5- or 6-membered, which may be bonded via an optionally at least mono-substituted C₁₋C₆ alkylene group, an optionally at least mono-substituted C₂₋C₆ alkenylene group or an optionally at least mono-substituted C₂₋C₆ alkynylene group and/or wherein the ring(s) may contain at least one heteroatom as a ring member,
preferably A represents an optionally at least mono-substituted mono- or polycyclic aromatic ring system, wherein the ring(s) is/are 5- or 6-membered and wherein one or more of the rings contain at least one heteroatom,
or a radical chosen from the group consisting of 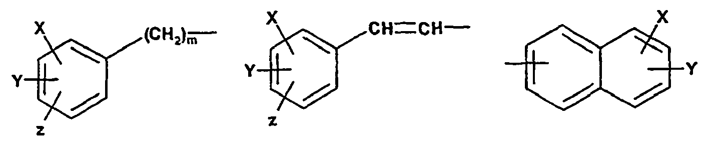 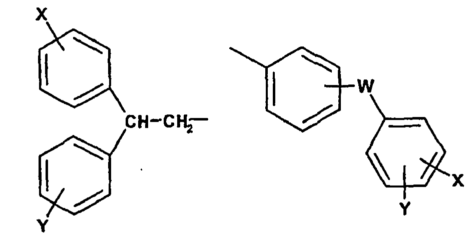 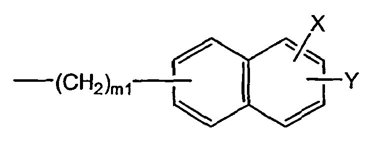 wherein X, Y, Z, independently from one another, each represent a radical selected from the group consisting of hydrogen, fluorine, chlorine, bromine, nitro, acetyl, linear or branched C₁-C₆ alkyl, linear or branched C₁-C₆ alkoxy, linear or branched C₁-C₆ alkylthio, a trifluoromethyl radical, a cyano radical and a -NR¹²R¹³ radical,
wherein R¹² and R¹³, identical or different, each represent hydrogen or linear or branched C₁-C₆ alkyl,
W represents a single chemical bond between the two rings, a CH₂, O, S group or a NR¹⁴ radical,
wherein R¹⁴ is hydrogen or a linear or branched C₁-C₆ alkyl,
m is 0, 1, 2, 3 or 4 and
m1 is 1 or 2, preferably 2, and R¹-R⁹ and n are defined as above.

Furthermore, sulfonamide derivatives of general formula (Ib) are preferred, wherein A represents an optionally at least mono-substituted mono- or polycyclic aromatic ring system, wherein the ring(s) is/are 5- or 6-membered, which may be bonded via an optionally at least mono-substituted C₁₋C₆ alkylene group, an optionally at least mono-substituted C₂₋C₆ alkenylene group or an optionally at least mono-substituted C₂₋C₆ alkynylene group, and/or wherein the ring(s) may contain at least one heteroatom as a ring member,
more preferably A represents an optionally at least mono-substituted mono- or polycyclic aromatic ring system, wherein the ring(s) is/are 5- or 6-membered and wherein one or more of the rings contain at least one heteroatom, or a radical chosen from the group consisting of 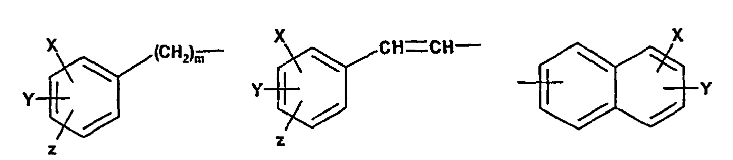 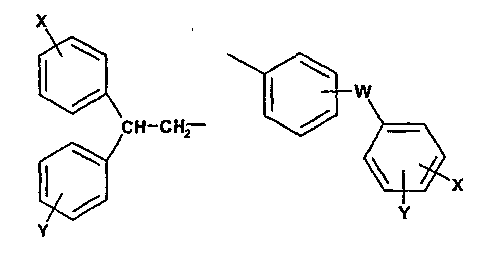 wherein X, Y, Z, independently from one another, each represent a radical selected from the group consisting of hydrogen, fluorine, chlorine, bromine, linear or branched C₁-C₆ alkyl, linear or branched C₁-C₆ alkoxy, linear or branched C₁-C₆ alkylthio, a trifluoromethyl radical, a cyano radical and a - NR¹²R¹³ radical,
wherein R¹² and R¹³, identical or different, each represent hydrogen or linear or branched C₁-C₆ alkyl,
W represents a single chemical bond between the two rings, a CH₂, O, S group or a NR¹⁴ radical,
wherein R¹⁴ is hydrogen or a linear or branched C₁-C₆ alkyl,
and
m is 0, 1, 2, 3 or 4.
and R¹-R⁹ and n are defined as above.

Furthermore, sulfonamide derivatives of general formula (Ib) are preferred, wherein A represents a heteroaryl radical selected from the group consisting of quinolinyl, benzo[b]thiophenyl, benzo[1,2,5]thiadiazolyl, thiophenyl and imidazo[2,1-b]thiazolyl which may be substituted by 1, 2 or 3 substituents selected from the group consisting of fluorine, bromine, chlorine, methyl, phenyl, nitro, -C(=O)-CH₃, -O-CH₃ and -O-phenyl and/or which may be bonded via a C₁₋ ₂ alkylene group or a C₂ alkenylene group,
or a radical chosen from the group consisting of 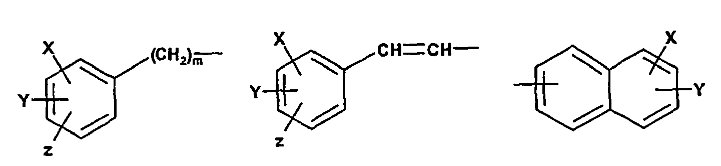 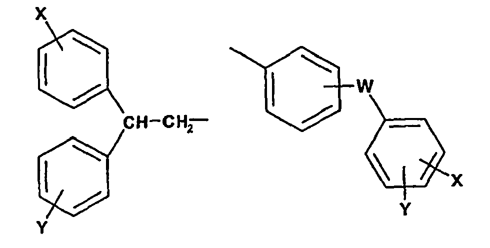 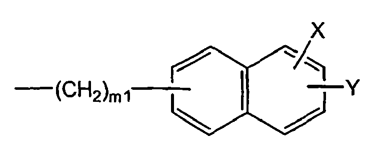 wherein X, Y, Z, independently from one another, each represent a radical selected from the group consisting of hydrogen, fluorine, chlorine, bromine, nitro, acetyl, linear or branched C₁-C₆ alkyl, linear or branched C₁-C₆ alkoxy, linear or branched C₁-C₆ alkylthio, a trifluoromethyl radical, a cyano radical and a -NR¹²R¹³ radical, wherein R¹² and R¹³, identical or different, each represent hydrogen or linear or branched C₁-C₆ alkyl,
W represents a single chemical bond between the two rings, a CH₂, O, S group or a NR¹⁴ radical,
wherein R¹⁴ is hydrogen or a linear or branched C₁-C₆ alkyl,
m is 0, 1, 2, 3 or 4 and
m1 is 1 or 2, preferably 2,
and R¹-R⁹ and n are defined as above.

Furthermore sulfonamide derivatives of general formula (Ib) are preferred, wherein n is 0, 1, 2, 3 or 4; preferably n is 1, 2 or 3; more preferably n is 2 or 3 and R¹ to R⁹ and A are defined as above.

Those most preferred compounds of general formula (Ib) are selected from the group consisting of
[1] N-[1-(2-dimethylaminoethyl)-1H-indole-5-yl]-5-chloro-3-methylbenzo[b]thiophene-2-sulfonamide,
[2] N-[1-(2-dimethylaminoethyl)-1H-indole-5-yl]-naphthalene-2-sulfonamide,
[3] N-[1-(2-dimethylaminoethyl)-1H-indole-5-yl]-naphthalene-1-sulfonamide,
[4] N-[1-(2-dimethylaminoethyl)-1H-indole-5-yl]-5-chloronaphthalene-1-sulfonamide,
[5] N-[1-(2-dimethylaminoethyl)-1H-indole-5-yl]-benzenesulfonamide,
[6] N-[1-(2-dimethylaminoethyl)-1H-indole-5-yl]-quinoline-8-sulfonamide,
[7] N-[1-(2-dimethylaminoethyl)-1H-indole-5-yl]-4-phenoxybenzenesulfonamide,
[8] N-[1-(2-dimethylaminoethyl)-1H-indole-5-yl]-4-methylbenzenesulfonamide,
[9] N-[1-(2-dimethylaminoethyl)-1H-indole-5-yl]-5-chlorothiophene-2-sulfonamide,
[10] N-[1-(2-dimethylaminoethyl)-1H-indole-5-yl]-benzo[1,2,5]thiadiazole-4-sulfonamide,
[11] N-[1-(2-dimethylaminoethyl)-1H-indole-5-yl]-6-chloroimidazo[2,1-b]thiazole-5-sulfonamide,
[12] N-[1-(2-dimethylaminoethyl)-1H-indole-5-yl]-3,5-dichlorobenzenesulfonamide,
[13] N-[1-(2-dimethylaminoethyl)-1H-indole-5-yl]-3-bromobenzenesulfonamide,
[14] N-[1-(2-dimethylaminoethyl)-1H-indole-5-yl]-3-nitrobenzenesulfonamide,
[15] N-[1-(2-dimethylaminoethyl)-1H-indole-5-yl]-1-phenylmethanesulfonamide,
[19] *trans*-N-[1-(2-dimethylaminoethyl)-1H-indole-5-yl]-2-phenylethenesulfonamide,
[20] N-[1-(2-dimethylaminoethyl)-1H-indole-5-yl]-4,5-dichlorothiophene-2-sulfonamide,
[21] N-[1-(2-dimethylaminoethyl)-1H-indole-5-yl]-4-acetylbenzenesulfonamide,
[22] N-[1-(2-dimethylaminoethyl)-1H-indole-5-yl]-4-bromobenzenesulfonamide,
[23] N-[1-(2-dimethylaminoethyl)-1H-indole-5-yl]-4-methoxybenzenesulfonamide,
[24] N-[3-(2-diethylaminoethyl)-1H-indole-5-yl]-5-chloro-3-methylbenzo[b]thiophene-2-sulfonamide,
[25] N-[1-(2-dimethylaminoethyl)-1H-indole-5-yl]-4-nitrobenzenesulfonamide,
[26] N-[1-(2-dimethylaminoethyl)-1H-indole-5-yl]-4-fluorobenzenesulfonamide,
[27] N-[1-(2-diethylaminoethyl)-1H-indole-5-yl]-6-chloroimidazo[2,1-b]thiazole-5-sulfonamide
[29] N-(1-(2-(diethylamino)ethyl)-1H-indol-5-yi)-naphthalene-2-sulfonamide,
[30] N-(1-(2-(diethylamino)ethyl)-1H-indol-5-yl)-naphthalene-1-sulfonamide,
[31] N-(1-(2-(diethylamino)ethyl)-1H-indol-5-yl)-4-phenylbenzenesulfonamide,
[32] 5-chloro-N-(1-(2-(dimethylamino)ethyl)-2-methyl-1H-indol-5-yl)-3-methylbenzo[b]thiophene-2-sulfonamide,
[33] N-(1-(2-(dimethylamino)ethyl)-2-methyl-1H-indol-5-yl)-naphthalene-2-sulfonamide,
[34] N-(1-(2-(dimethylamino)ethyl)-2-methyl-1H-indol-5-yl)-naphthalene-1-sulfonamide,
[35] 6-chloro-N-(1-(2-(dimethylamino)ethyl)-2-methyl-1H-indol-5-yl)imidazo[2,1-b]thiazole-5-sulfonamide,
[36] N-(1-(2-(dimethylamino)ethyl)-2-methyl-1H-indol-5-yl)-4-phenylbenzenesulfonamide,
[37] N-(1-(2-dimethylamino)ethyl)-2-methyl-1H-indol-5-yl)-2-(naphth-1-yl)-ethanesulfonamide,
[38] N-(1-(2-(dimethylamino)ethyl)-2-methyl-1H-indol-5-yl)-4-phenoxybenzenesulfonamide,
[39] 3,5-dichloro-N-(1-(2-(dimethylamino)ethyl)-2-methyl-1H-indol-5-yl)-benzenesulfonamide,
[40] N-(1-(2-(dimethylamino)ethyl)-2-methyl-1H-indol-5-yl)benzo[b]thiophene-3-sulfonamide,
[41] N-(1-(2-(diethylamino)ethyl)-1H-indol-5-yl)benzo[b]thiophene-3-sulfonamide and
[42] N-(1-(2-(dimethylamino)ethyl)-1H-indol-5-yl)benzo[b]thiophene-3-sulfonamide,
and their corresponding salts and solvates.

Sulfonamide derivatives of general formula (Ic) are preferred, wherein R¹ represents an -NR⁸R⁹ radical or a saturated or unsaturated, optionally at least mono-substituted 5- or 6-membered cycloaliphatic radical which may optionally contain at least one heteroatom as a ring member and/or which may be condensed with a saturated or unsaturated, optionally at least mono-substituted mono- or bicyclic cycloaliphatic ring system, which may optionally contain at least one heteroatom as a ring member, whereby the rings of the ring system are 5- or 6-membered,
more preferably R¹ represents an -NR⁸R⁹ radical or a radical chosen from the group consisting of 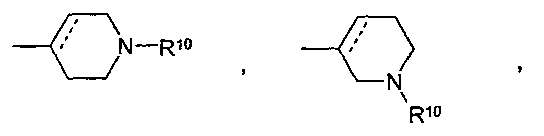 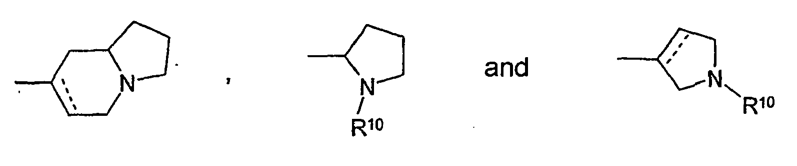 wherein, if present, the dotted line is an optional chemical bond, and R¹⁰ represents hydrogen, a linear or branched C₁-C₆ alkyl radical or a benzyl radical, preferably hydrogen or a C₁-C₂ alkyl radical and R² to R⁹, A and n are defined as above.

Sulfonamide derivatives of general formula (Ic) are also preferred, wherein R², R³, R⁴, R⁶ and R⁷, identical or different, each represent hydrogen, a linear or branched, optionally at least mono-substituted C₁₋C₆ alkyl radical, a linear or branched, optionally at least mono-substituted C₂₋C₆ alkenyl radical or a linear or branched, optionally at least mono-substituted C₂₋C₆ alkynyl radical,
more preferably R², R³, R⁴, R⁶ and R⁷, identical or different, each represent hydrogen or a linear or branched, optionally at least mono-substituted C₁₋C₆ alkyl radical,
even more preferably R², R³, R⁴, R⁶ and R⁷ each represent hydrogen or a C1-2 alkyl radical and R¹, R⁵, R⁸, R⁹, A and n are defined as above.

Sulfonamide derivatives of general formula (Ic) are also preferred, wherein R⁵ represents hydrogen, a linear or branched, optionally at least mono-substituted C₁-C₆ alkyl radical, a linear or branched, optionally at least mono-substituted C₂-C₆ alkenyl radical, a linear or branched, optionally at least mono-substituted C₂-C₆ alkynyl radical,
more preferably R⁵ represents hydrogen or a linear or branched, optionally at least mono-substituted C₁-C₆ alkyl radical,
even more preferably R⁵ represents hydrogen or a C₁-C₂ alkyl radical and R¹-R⁴, R⁶-R⁹, A and n are defined as above.

Furthermore, sulfonamide derivatives of general formula (Ic) are also preferred, wherein R⁸ and R⁹, identical or different, each represent a linear or branched, optionally at least mono-substituted C₁-C₁₀ alkyl radical, a linear or branched, optionally at least mono-substituted C₂₋C₁₀ alkenyl radical, a linear or branched, optionally at least mono-substituted C₂-C₁₀ alkynyl radical, or R⁸ and R⁹ together with the bridging nitrogen atom form a saturated or unsaturated, optionally at least mono-substituted 5- or 6-membered heterocyclic ring which may contain at least one additional heteroatom as a ring member and/or which may be condensed with a saturated or unsaturated, optionally at least mono-substituted mono- or bicyclic cycloaliphatic ring system, which may optionally contain at least one heteroatom as a ring member, whereby the rings of the ring system are 5- 6- or 7-membered and R¹-R⁷, A and n are defined as above.

Particularly preferred are sulfonamide derivatives of general formula (Ic), wherein R⁸ and R⁹, identical or different, each represent hydrogen or a linear or branched C₁-C₁₀ alkyl radical, or
R⁸ and R⁹ together with the nitrogen atom bridge form a radical chosen from the group consisting of 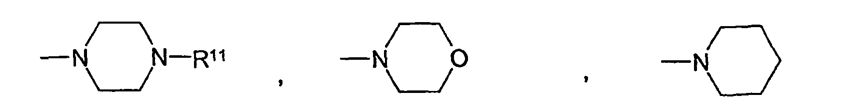 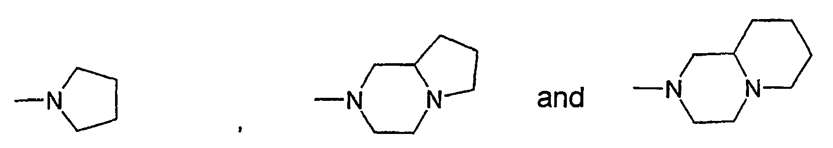 wherein R¹¹, if present, represents hydrogen, a linear or branched C₁-C₆ alkyl radical or a benzyl radical, preferably hydrogen or a C₁-C₂ alkyl radical, and R¹-R⁹, A and n are defined as above.

Furthermore, sulfonamide derivatives of general formula (Ic) are preferred, wherein A represents an optionally at least mono-substituted mono- or polycyclic aromatic ring system, wherein the ring(s) is/are 5- or 6-membered, which may be bonded via an optionally at least mono-substituted C₁₋C₆ alkylene group, an optionally at least mono-substituted C₂₋C₆ alkenylene group or an optionally at least mono-substituted C₂₋C₆ alkynylene group and/or wherein the ring(s) may contain at least one heteroatom as a ring member,
preferably A represents an optionally at least mono-substituted mono- or polycyclic aromatic ring system, wherein the ring(s) is/are 5- or 6-membered and wherein one or more of the rings contain at least one heteroatom,
or a radical chosen from the group consisting of 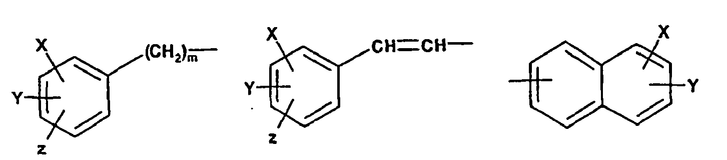 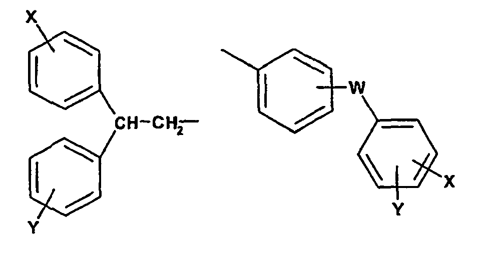 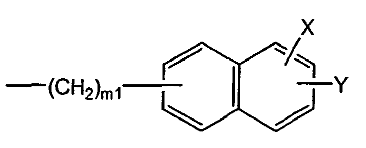 wherein X, Y, Z, independently from one another, each represent a radical selected from the group consisting of hydrogen, fluorine, chlorine, bromine, nitro, acetyl, linear or branched C₁-C₆ alkyl, linear or branched C₁-C₆ alkoxy, linear or branched C₁-C₆ alkylthio, a trifluoromethyl radical, a cyano radical and a -NR¹²R¹³ radical,
wherein R¹² and R¹³, identical or different, each represent hydrogen or linear or branched C₁-C₆ alkyl,
W represents a single chemical bond between the two rings, a CH₂, O, S group or a NR¹⁴ radical,
wherein R¹⁴ is hydrogen or a linear or branched C₁-C₆ alkyl,
m is 0, 1, 2, 3 or 4 and
m1 is 1 or 2, preferably 2, and R¹-R⁹ and n are defined as above.

Furthermore, sulfonamide derivatives of general formula (Ic) are preferred, wherein A represents an optionally at least mono-substituted mono- or polycyclic aromatic ring system, wherein the ring(s) is/are 5- or 6-membered, which may be bonded via an optionally at least mono-substituted C₁₋C₆ alkylene group, an optionally at least mono-substituted C₂₋C₆ alkenylene group or an optionally at least mono-substituted C₂₋C₆ alkynylene group, and/or wherein the ring(s) may contain at least one heteroatom as a ring member,
more preferably A represents an optionally at least mono-substituted mono- or polycyclic aromatic ring system, wherein the ring(s) is/are 5- or 6-membered and wherein one or more of the rings contain at least one heteroatom, or a radical chosen from the group consisting of 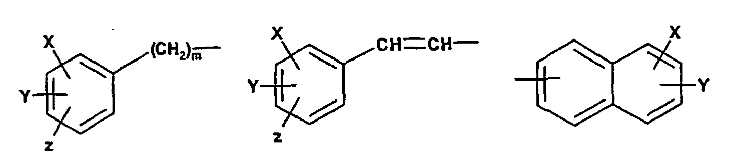 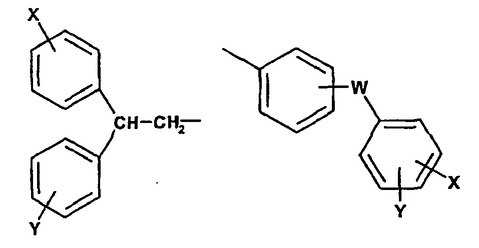 wherein X, Y, Z, independently from one another, each represent a radical selected from the group consisting of hydrogen, fluorine, chlorine, bromine, linear or branched C₁-C₆ alkyl, linear or branched C₁-C₆ alkoxy, linear or branched C₁-C₆ alkylthio, a trifluoromethyl radical, a cyano radical and a - NR¹²R¹³ radical,
wherein R¹² and R¹³, identical or different, each represent hydrogen or linear or branched C₁-C₆ alkyl,
W represents a single chemical bond between the two rings, a CH₂, O, S group or a NR¹⁴ radical,
wherein R¹⁴ is hydrogen or a linear or branched C₁-C₆ alkyl,
and
m is 0, 1, 2, 3 or 4.
and R¹-R⁹ and n are defined as above.

Furthermore, sulfonamide derivatives of general formula (Ic) are preferred, wherein A represents a heteroaryl radical selected from the group consisting of quinolinyl, benzo[b]thiophenyl, benzo[1,2,5]thiadiazolyl, thiophenyl and imidazo[2,1-b]thiazolyl which may be substituted by 1, 2 or 3 substituents selected from the group consisting of fluorine, bromine, chlorine, methyl, phenyl, nitro, -C(=O)-CH₃, -O-CH₃ and -O-phenyl and/or which may be bonded via a C₁₋₂ alkylene group or a C₂ alkenylene group,
or a radical chosen from the group consisting of 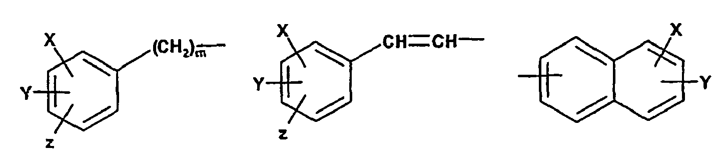 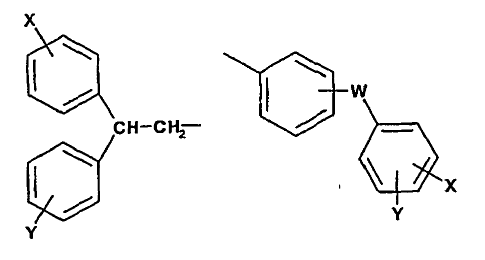 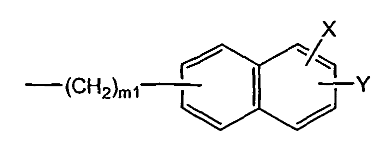 wherein X, Y, Z, independently from one another, each represent a radical selected from the group consisting of hydrogen, fluorine, chlorine, bromine, nitro, acetyl, linear or branched C₁-C₆ alkyl, linear or branched C₁-C₆ alkoxy, linear or branched C₁-C₆ alkylthio, a trifluoromethyl radical, a cyano radical and a -NR¹²R¹³ radical,
wherein R¹² and R¹³, identical or different, each represent hydrogen or linear or branched C₁-C₆ alkyl,
W represents a single chemical bond between the two rings, a CH₂, O, S group or a NR¹⁴ radical,
wherein R¹⁴ is hydrogen or a linear or branched C₁-C₆ alkyl,
m is 0, 1, 2, 3 or 4 and
m1 is 1 or 2, preferably 2, and R¹-R⁹ and n are defined as above.

Furthermore sulfonamide derivatives of general formula (Ic) are preferred, wherein n is 0, 1, 2, 3 or 4; preferably n is 1, 2 or 3; more preferably n is 2 or 3 and R¹ to R⁹ and A are defined as above.

Another aspect of the present invention are compounds of general formula (Ic), 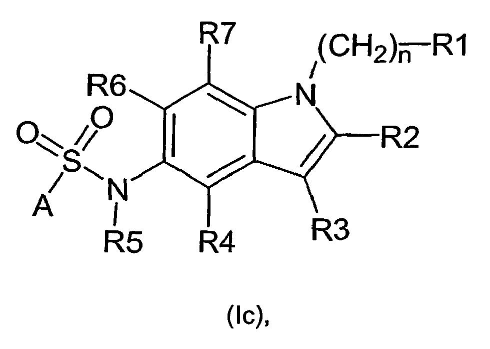 wherein
R¹ represents a -NR⁸R⁹ radical,
R² represents hydrogen or an alkyl radical selected from the group consisting of methyl, ethyl, n-propyl and iso-propyl, more preferably hydrogen or methyl,
R³, R⁴, R⁶ and R⁷ each represent hydrogen,
R⁵ represents hydrogen,
R⁸ and R⁹, identical or different, each represent methyl, ethyl, n-propyl or iso-propyl, more preferably methyl or ethyl,
or
R⁸ and R⁹ together with the bridging nitrogen form a 5- or 6-membered heterocyclic ring, more preferably form pyrrolidine or piperidine,
A represents an aryl or heteroaryl radical selected from the group consisting of phenyl, naphthyl, quinolinyl, benzo[b]thiophenyl, benzo[1,2,5]thiadiazolyl, thiophenyl and imidazo[2,1-b]thiazolyl which may be substituted by 1, 2 or 3 substituents selected from the group consisting of fluorine, bromine, chlorine, methyl, phenyl, nitro, -C(=O)-CH₃, -O-CH₃ and -O-phenyl and/or which may be bonded via a C₁-₂ alkylene group or a C₂ alkenylene group,
and
n is 2 or 3,
optionally in form of one of its stereoisomers, preferably enantiomers or diastereomers, its racemate or in form of a mixture of at least two of its stereoisomers, preferably enantiomers or diastereomers, in any mixing ratio, or a salt thereof, preferably a corresponding, physiologically acceptable salt thereof, or a corresponding solvate thereof.

The most preferred compounds of general formula (Ic) are selected from the group consisting of
[1] N-[1-(2-dimethylaminoethyl)-1H-indole-5-yl]-5-chloro-3-methylbenzo[b]thiophene-2-sulfonamide,
[2] N-[1-(2-dimethylaminoethyl)-1H-indole-5-yl]-naphthalene-2-sulfonamide,
[3] N-[1-(2-dimethylaminoethyl)-1H-indole-5-yl]-naphthalene-1-sulfonamide,
[4] N-[1-(2-dimethylaminoethyl)-1H-indole-5-yl]-5-chloronaphthalene-1-sulfonamide,
[5] N-[1-(2-dimethylaminoethyl)-1H-indole-5-yl]-benzenesulfonamide,
[6] N-[1-(2-dimethylaminoethyl)-1H-indole-5-yl]-quinoline-8- sulfonamide,
[7] N-[1-(2-dimethylaminoethyl)-1H-indole-5-yl]-4-phenoxybenzenesulfonamide,
[8] N-[1-(2-dimethylaminoethyl)-1H-indole-5-yl]-4-methylbenzenesulfonamide,
[9] N-[1-(2-dimethylaminoethyl)-1H-indole-5-yl]-5-chlorothiophene-2-sulfonamide,
[10] N-[1-(2-dimethylaminoethyl)-1H-indole-5-yl]-benzo[1,2,5]thiadiazole-4-sulfonamide,
[11] N-[1-(2-dimethylaminoethyl)-1H-indole-5-yl]-6-chloroimidazo[2,1-b]thiazole-5-sulfonamide,
[12] N-[1-(2-dimethylaminoethyl)-1H-indole-5-yl]-3,5-dichlorobenzenesulfonamide,
[13] N-[1-(2-dimethylaminoethyl)-1H-indole-5-yl]-3-bromobenzenesulfonamide,
[14] N-[1-(2-dimethylaminoethyl)-1H-indole-5-yl]-3-nitrobenzenesulfonamide,
[15] N-[1-(2-dimethylaminoethyl)-1H-indole-5-yl]-1-phenylmethanesulfonamide,
[16] N-[1-(2-pyrrolidine-1-yl-ethyl)-1H-indole-5-yl]-naphthalene-2-sulfonamide,
[17] N-[1-(2-pyrrolidine-1-yl-ethyl)-1H-indole-5-yl]-naphthalene-1-sulfonamide,
[18] N-[1-(2-pyrrolidine-1-yl-ethyl)-1H-indole-5-yl]-5-chloro-3-methylbenzo[b]thiophene-2-sulfonamide,
[19] *trans*-N-[1-(2-dimethylaminoethyl)-1H-indole-5-yl]-2-phenylethenesulfonamide,
[20] N-[1-(2-dimethylaminoethyl)-1H-indole-5-yl]-4,5-dichlorothiophene-2-sulfonamide,
[21] N-[1-(2-dimethylaminoethyl)-1 H-indole-5-yl]-4-acetylbenzenesulfonamide,
[22] N-[1-(2-dimethylaminoethyl)-1H-indole-5-yl]-4-bromobenzenesulfonamide,
[23] N-[1-(2-dimethylaminoethyl)-1H-indole-5-yl]-4-methoxybenzenesulfonamide,
[24] N-[3-(2-diethylaminoethyl)-1H-indole-5-yl]-5-chloro-3-methylbenzo[b]thiophene-2-sulfonamide,
[25] N-[1-(2-dimethylaminoethyl)-1H-indole-5-yl]-4-nitrobenzenesulfonamide,
[26] N-[1-(2-dimethylaminoethyl)-1H-indole-5-yl]-4-fluorobenzenesulfonamide,
[27] N-[1-(2-diethylaminoethyl)-1H-indole-5-yl]-6-chloroimidazo[2,1-b]thiazole-5-sulfonamide,
[28] N-[1-(2-pyrrolidine-1-yl-ethyl)-1H-indole-5-yl]- ]-6-chloroimidazo[2,1-b]thiazole-5-sulfonamide,
[29] N-(1-(2-(diethylamino)ethyl)-1H-indol-5-yl)-naphthalene-2-sulfonamide,
[30] N-(1-(2-(diethylamino)ethyl)-1H-indol-5-yl)-naphthalene-1-sulfonamide,
[31] N-(1-(2-(diethylamino)ethyl)-1H-indol-5-yl)-4-phenylbenzenesulfonamide,
[32] 5-chloro-N-(1-(2-(dimethylamino)ethyl)-2-methyl-1H-indol-5-yl)-3-methylbenzo[b]thiophene-2-sulfonamide,
[33] N-(1-(2-(dimethylamino)ethyl)-2-methyl-1H-indol-5-yl)-naphthalene-2-sulfonamide,
[34] N-(1-(2-(dimethylamino)ethyl)-2-methyl-1H-indol-5-yl)-naphthalene-1-sulfonamide,
[35] 6-chloro-N-(1-(2-(dimethylamino)ethyl)-2-methyl-1H-indol-5-yl)imidazo[2,1-b]thiazole-5-sulfonamide,
[36] N-(1-(2-(dimethylamino)ethyl)-2-methyl-1H-indol-5-yl)-4-phenylbenzenesulfonamide,
[37] N-(1-(2-dimethylamino)ethyl)-2-methyl-1H-indol-5-yl)-2-(naphth-1-yl)-ethanesulfonamide,
[38] N-(1-(2-(dimethylamino)ethyl)-2-methyl-1H-indol-5-yl)-4-phenoxybenzenesulfonamide,
[39] 3,5-dichloro-N-(1-(2-(dimethylamino)ethyl)-2-methyl-1H-indol-5-yl)-benzenesulfonamide,
[40] N-(1-(2-(dimethylamino)ethyl)-2-methyl-1H-indol-5-yl)benzo[b]thiophene-3-sulfonamide,
[41] N-(1-(2-(diethylamino)ethyl)-1H-indol-5-yl)benzo[b]thiophene-3-sulfonamide and
[42] N-(1-(2-(dimethylamino)ethyl)-1H-indol-5-yl)benzo[b]thiophene-3-sulfonamide,
[43] 5-chloro-3-methyl-N-(1-(3-(piperidin-1-yl)propyl)-1H-indol-5-yl)benzo[b]thiophene-2-sulfonamide,
[44] N-(1-(3-(piperidin-1-yl)propyl)-1H-indol-5-yl)naphthalene-2-sulfonamide,
[45] N-(1-(3-(piperidin-1-yl)propyl)-1H-indol-5-yl)naphthalene-1-sulfonamide,
[46] 6-chloro-N-(1-(3-piperidin-1-yl)propyl)-1H-indol-5-yl)imidazo[2,1-b]thiazole-5-sulfonamide,
[47] 4-phenyl-N-(1-(3-(piperidin-1-yl)propyl)-1H-indol-5-yl)benzenesulfonamide,
[48] 2-(naphth-1-yl)-N-(1-(3-(piperidin-1-yl)propyl)-1H-indol-5-yl)ethanesulfonamide,
[49] 4-phenoxy-N-(1-(3-(piperidin-1-yl)propyl)-1H-indol-5-yl)benzenesulfonamide,
[50] 3,5-dichloro-N-(1-(3-(piperidin-1-yl)propyl)-1H-indol-5-yl)benzenesulfonylamide,
[51] 4,5-dichloro-N-(1-(3-(piperidin-1-yl)propyl)-1H-indol-5-yl)thiophene-2-sulfonamide and
[52] 5-chloro-N-(1-(3-(piperidin-1-yl)propyl)-1H-indol-5-yl)naphthalene-1-sulfonamide,
and their corresponding salts and solvates.

The present invention likewise refers to the salts, preferably the physiologically acceptable salts of the compounds of general formula (Ia) and/or (Ib) and/or of general formula (Ic), preferably the addition salts of mineral acids, more preferably of hydrochloric acid, hydrobromic acid acid, phosphoric acid, sulphuric acid, nitric acid, and the salts of organic acids, more preferably of citric acid, maleic acid acid, fumaric acid, tartaric acid or their derivatives, p-toluenesulphonic acid, methanesulphonic acid, camphorsulphonic acid, etc.

Below, the expression sulfonamide derivatives of the general formula (I), refers to one or more compounds of general formula (Ia) and/or to one or more compounds of general formula (Ib) and/or to one or more compounds of general formula (Ic), respectively, and optionally in form of one of their stereoisomers, preferably enantiomers or diastereomers, their racemate, or in form of a mixture of at least two of their stereoisomers, preferably enantiomers or diastereomers, in any mixing ratio, or a salt thereof, preferably a corresponding physiologically acceptable salt thereof, or a corresponding solvate thereof.

Another aspect of the present invention consists of a process for preparing the new derivatives of general formula (I), wherein R¹-R⁹, n and A have the previously indicated meaning, according to which at least one compound of general formula (II), 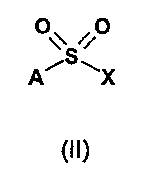 wherein A has the previously mentioned meaning, and X is an acceptable leaving group, preferably an halogen atom, more preferably chlorine; reacts with at least one substituted 5-aminoindole of general formula (III) 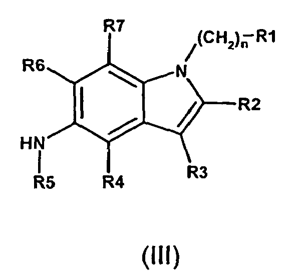 wherein R¹-R⁷ and n have the previously indicated meaning, or one of their suitable protected derivatives, and, if necessary, the protective groups are removed in order to obtain the corresponding sulfonamide derivative of formula (I), which may be purified and/or isolated by means of conventional methods known in the prior art.

The reaction between the compounds of general formula (II) and (III) is usually carried out in the presence of an organic reaction medium, preferably in the presence of dialkyl ether, more preferably diethyl ether or a cyclic ether, more preferably tetrahydrofuran or dioxane, an halogenated organic hydrocarbon, more preferably methylene chloride or chloroform, an alcohol, more preferably methanol or ethanol, a dipolar aprotic solvent, more preferably acetonitrile, pyridine or dimethylformamide, or any other suitable reaction medium. Naturally, mixtures of at least two of the classes of the mentioned compounds or at least two compounds of one class may also be used.

The reaction is preferably carried out in the presence of a suitable base, for example, an inorganic base, more preferably alkaline metal hydroxides and alkaline metal carbonates, or in the presence of an organic base, more preferably triethylamine, N-ethyldiisopropylamine or pyridine.

The most suitable reaction temperatures range from 0°C to room temperature, that is, approximately 25°C, and the reaction time preferably comprises from 5 minutes to 24 hours.

The resulting sulfonamide derivative of general formula (I) may be purified and/or isolated according to conventional methods known in the prior art.

Preferably, the sulfonamide derivatives of general formula (I) may be isolated by evaporating the reaction medium, adding water and, if necessary, adjusting the pH so that a solid which may be isolated by filtration is obtained; or the sulfonamide derivative may be extracted with a water immiscible solvent, preferably chloroform, and be purified by chromatography or recrystallization in a suitable solvent.
The compounds of general formula (II) are commercially available, or they may be prepared according to standard methods known in the prior art, for example by methods similar to those described in the literature [E.E.Gilbert, Synthesis, 1969, 1, 3]. The compounds of general formula (III) may also be prepared according to standard methods known in the prior art, for example by methods similar to those described in the literature: Pigerol, Charles; De Cointet de Fillain, Paul; Eymard, Pierre; Werbenec, Jean Pierre; Broil, Madeleine. (Labaz S. A., Fr.). Ger. Offen. (1977). DE 2727047 19771229. Schwink, Lothar; Stengelin, Siegfried; Gossel, Matthias. Preparation of indol-5-ylureas and related compounds for the treatment of obesity and type II diabetes. WO 0315769 A1 20030227. One of them consists of nitro group reduction of derivatives of general formula (IV) by methods known in the prior art, as for example: BRATTON, L. D.; ROTH, B. D.; TRIVEDI, B. K.; UNANGST, P. C.; J Heterocycl Chem , 2000, 37 (5), 1103-1108. FANGHAENEL, E.; CHTCHEGLOV, D.; J Prakt Chem/Chem-Ztg, 1996, 338 (8), 731-737. KUYPER, L. F.; BACMAYARI, D. P.; JONES, M. L.; HUNTER, R. N.; TANSIK, R. L.; JOYNER, S. S.; BOYTOS, C. M.; RUDOLPH, S. K.; KNICK, V.; WILSON, H. R.; CADDELL, J. M.; FRIEDMAN, H. S.; ET AL.; J Med Chem, 1996, 39 (4), 892-903. 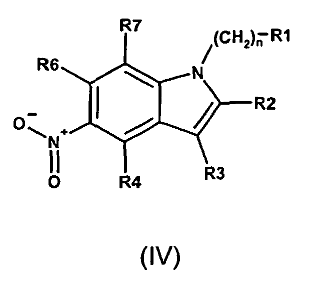 wherein R¹-R⁷ and n have the previously indicated meaning, or one of their suitably protected derivatives, and, if necessary, the protective groups are removed in order to obtain the corresponding amine of general formula (III), which may be purified and/or isolated by means of conventional methods known in the prior art.

The compounds of general formula (IV) may also be prepared according to standard methods known in the prior art, for example by methods similar to those described in the literature: Journal of Heterocyclic Chemistry, 37(5), 1103-1108; 2000; Schwink, Lothar; Stengelin, Siegfried; Gossel, Matthias. Preparation of indol-5-ylureas and relate compounds for the treatment of obesity and type II diabetes WO 0315769 A1 20030227; Baxter, Andrew; Brough, Stephen; Mcinally, Thomas; Mortimore, Michael; Cladingboel, David. Preparation of N-aryl-1-adamantaneacetamides and analogs as purinergic P2Z receptor antagonists WO 9929660 A1 19990617 ; Pigerol, Charles; De Cointet de Fillain, Paul; Eymard, Pierre; Werbenec, Jean Pierre; Broil, Madeleine. Indole derivatives. Ger. Offen. (1977), DE 2727047 19771229.

One of them consists of the alkylation of nitro derivatives of general formula (V) by methods known in the art, as for example: BHAGWAT, S. S.; GUDE, C.; Tetrahedron Lett, 1994, 35 (12), 1847-1850. BRATTON, L. D.; ROTH, B. D.; TRIVEDI, B. K.; UNANGST, P. C.; J Heterocycl Chem, 2000, 37 (5), 1103-1108 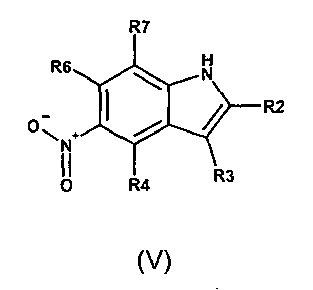 wherein R²-R⁷ and n have the previously mentioned meaning, or one of their suitably protected derivatives, and, if necessary, the protective groups are removed in order to obtain the corresponding amine of general formula (III), which may be purified and/or isolated by means of conventional methods known in the prior art.

The compounds of general formula (V) are commercially available or may also be prepared according to standard methods known in the prior art, as for example YAMASHKIN, S. A.; YUROVSKAYA, M. A.; Chem Heterocycl Compd (N Y) 1999, 35 (12), 1426-1432. OTTONI, O.; CRUZ, R.; KRAMMER, N. H.; Tetrahedron Lett ,1999, 40 (6), 1117-1120. EZQUERRA, J.; PEDREGAL, C.; LAMAS, C.; BARLUENGA, J.; PEREZ, M.; GARCIA-MARTIN, M. A.; GONZALEZ, J. M.; J Org Chem ,1996, 61 (17), 5804-5812. FADDA, A. A.; Indian J Chem, Sect B: Org Chem Incl Med Chem , 1990, 29 (11),1017-1019. KATRITZKY, A. R.; RACHWAL, S.; BAYYUK, S.; Org Prep Proced Int, 1991, 23 (3), 357-363. Inada, A.; Nakamura, Y.; Morita, Y.; Chem Lett , 1980, 1287.

The respective literature descriptions are incorporated by reference and form part of the disclosure.

Another aspect of the present invention consists of a process for preparing the new sulfonamide derivatives of general formula (I), wherein R¹-R⁴, R⁶-R⁹, A, n and A have the previously indicated meaning and R⁵ is an alkyl radical, preferably a linear or branched, optionally at least mono-substituted C₁-C₆alkyl radical, by alkylation of a sulfonamide derivative of general formula (I),
wherein R¹-R⁴, R⁶-R⁹, n and A have the previously indicated meaning, and R⁵ is a hydrogen atom, with an alkyl halogenide or dialkyl sulfate.

The alkylation reaction is carried out preferably in the presence of a suitable base, more preferably in the presence of alkaline metal hydroxides and alkaline metal carbonates, metal hydrides, metal alkoxides, even more preferably sodium methoxide or potassium tert-butoxide, organometallic compounds, even more preferably butyllithium or tert-butyllithium, in the presence of an organic reaction medium, more preferably dialkyl ether, even more preferably diethyl ether, or a cyclic ether, even more preferably tetrahydrofuran or dioxane, an hydrocarbon, even more preferably toluene, an alcohol, even more preferably methanol or ethanol, a dipolar aprotic solvent, even more preferably acetonitrile, pyridine or dimethylformamide, or any other suitable reaction medium. Naturally, mixtures of at least two of the classes of the mentioned compounds or at least two compounds of one class may also be used.

The most suitable reaction temperatures range from 0°C to the boiling temperature of the reaction medium, and the reaction times preferably comprise from 1 to 24 hours.

Preferably, the resulting sulfonamide derivative of general formula (I) may be isolated by filtration, concentrating the filtrate under reduced pressure, adding water and, if necessary, adjusting the pH so that a solid which may be isolated by filtration is obtained; or the sulfonamide derivative may be extracted with a water immiscible solvent, preferably chloroform, and be purified by chromatography or recrystallization of a suitable solvent.

The salts, preferably pharmaceutically acceptable salts of the compounds of general formula (I), may be prepared by means of conventional methods known in the prior art, preferably by reaction with a mineral acid, more preferably by reaction with hydrochloric acid, hydrobromic acid, phosphoric acid acid, sulphuric acid or nitric acid, or by reaction with organic acids, more preferably by reaction with citric acid, maleic acid, fumaric acid acid, tartaric acid, or their derivatives, *p*-toluenesulphonic acid, methanesulphonic acid, camphorsulphonic acid, etc., in a suitable solvent, preferably methanol, ethanol, diethyl ether, ethyl acetate, acetonitrile or acetone, and obtaining the resulting salts by using the usual techniques for the precipitation or crystallization of the corresponding salts.

The preferred physiologically acceptable salts of the sulfonamide derivatives of general formula (I) are the addition salts of mineral acids, more preferably of hydrochloric acid, hydrobromic acid, phosphoric acid, sulphuric acid acid or nitric acid, and the addition salts of organic acids, more preferably citric acid, maleic acid, fumaric acid, tartaric acid, or their derivatives, p-toluenesulphonic acid, methanesulphonic acid, camphorsulphonic acid, etc.

The solvates, preferably the physiologically acceptable solvates, more preferably hydrates, of the sulfonamide derivatives of general formula (I) or of the corresponding physiologically acceptable salts, may be prepared by methods known in the prior art.

During some of the synthetic sequences described or in the preparation of the suitable reagents used, it may be necessary and/or desirable to protect sensitive or reactive groups in some of the molecules used. This may be carried out by means of the use of conventional protective groups preferably those described in the literature [Protective groups in Organic Chemistry, ed. J.F.W. McOmie, Plenum Press, 1973; T.W. Greene & P.G.M. Wuts, Protective Groups in Organic Chemistry, John Wiley & Sons, 1991]. The protective groups may be removed in the suitable subsequent stage by methods known in the prior art. The respective literature descriptions are incorporated by reference and form part of the disclosure.

If the sulfonamide derivatives of general formula (I) are obtained in form of a mixture of stereoisomers, preferably enantiomers or diastereomers, said mixtures may be separated by means of standard processes known in the prior art, for example chromatographic methods or crystallization with chiral agents.

Another aspect of the present invention is a medicament comprising at least one indol-5-yl sulfonamide derivative of general formula (I), optionally in form of one of its stereoisomers, preferably enantiomers or diastereomers, its racemate, or in form of a mixture of at least two of its stereoisomers, preferably enantiomers or diastereomers, in any mixing ratio, or a corresponding physiologically acceptable salt thereof or a corresponding solvate thereof, and optionally one or more pharmaceutically acceptable adjuvants.

This medicament is suitable for 5-HT₆ receptor regulation, for the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes caused by obesity, for the prophylaxis and/or treatment of gastrointestinal tract disorders, preferably irritable bowel syndrome, for cognitive enhancement, for the prophylaxis and/or treatment of disorders of the central nervous system, anxiety, panic disorders, depression, bipolar disorders, cognitive memory disorders, senile dementia processes, neurodegenerative disorders, preferably Alzheimer's disease, Parkinson's disease, Huntington's disease and Multiple Sclerosis, schizophrenia, psychosis or infantile hyperkinesia (ADHD, attention deficit / hyperactivity disorder), and other disorders mediated by the 5-HT₆ serotonin receptor in humans and/or in animals, preferably in mammals, more preferably in humans.

Another aspect of the present invention is a medicament comprising at least one indol-5-yl sulfonamide derivative of general formula (Ia), optionally in form of one of its stereoisomers, preferably enantiomers or diastereomers, its racemate, or in form of a mixture of at least two of its stereoisomers, preferably enantiomers or diastereomers, in any mixing ratio, or a corresponding physiologically acceptable salt thereof or a corresponding solvate thereof, and optionally one or more pharmaceutically acceptable adjuvants.

This medicament is suitable for 5-HT₆ receptor regulation, for the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes caused by obesity, for the prophylaxis and/or treatment of gastrointestinal tract disorders, preferably irritable bowel syndrome, for cognitive enhancement, for the prophylaxis and/or treatment of disorders of the central nervous system, anxiety, panic disorders, depression, bipolar disorders, cognitive memory disorders, senile dementia processes, neurodegenerative disorders, preferably Alzheimer's disease, Parkinson's disease, Huntington's disease and Multiple Sclerosis, schizophrenia, psychosis or infantile hyperkinesia (ADHD, attention deficit / hyperactivity disorder) and other disorders mediated by the 5-HT₆ serotonin receptor in humans and/or in animals, preferably in mammals, more preferably in humans,
more suitable for 5-HT₆ receptor regulation, for the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes caused by obesity, for the prophylaxis and/or treatment of gastrointestinal tract disorders, preferably irritable bowel syndrome in humans and/or in animals, preferably in mammals, more preferably in humans.

Another aspect of the present invention is a medicament comprising at least one indol-5-yl sulfonamide derivative of general formula (Ib), optionally in form of one of its stereoisomers, preferably enantiomers or diastereomers, its racemate, or in form of a mixture of at least two of its stereoisomers, preferably enantiomers or diastereomers, in any mixing ratio, or a corresponding physiologically acceptable salt thereof or a corresponding solvate thereof, and optionally one or more pharmaceutically acceptable adjuvants.

This medicament is suitable for 5-HT₆ receptor regulation, for the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes caused by obesity, for the prophylaxis and/or treatment of gastrointestinal tract disorders, preferably irritable bowel syndrome, for cognitive enhancement, for the prophylaxis and/or treatment of disorders of the central nervous system, anxiety, panic disorders, depression, bipolar disorders, cognitive memory disorders, senile dementia processes, neurodegenerative disorders, preferably Alzheimer's disease, Parkinson's disease, Huntington's disease and Multiple Sclerosis, schizophrenia, psychosis or infantile hyperkinesia (ADHD, attention deficit / hyperactivity disorder) and other disorders mediated by the 5-HT₆ serotonin receptor in humans and/or in animals, preferably in mammals, more preferably in humans,
more suitable for cognitive enhancement, for the prophylaxis and/or treatment of disorders of the central nervous system, anxiety, panic disorders, depression, bipolar disorders, cognitive memory disorders, senile dementia processes, neurodegenerative disorders, preferably Alzheimer's disease, Parkinson's disease, Huntington's disease and Multiple Sclerosis, schizophrenia, psychosis or infantile hyperkinesia (ADHD, attention deficit /hyperactivity disorder) improving cognition, for preventing and/or treating Central Nervous System Disorders, anxiety, panic disorders, depression, bipolar disorders, cognitive memory disorders, senile dementia processes, neurodegenerative disorders, preferably Alzheimer's disease, Parkinson's disease, Huntington's disease and Multiple Sclerosis, schizophrenia, psychosis, infantile hyperkinesia (ADHD, attention deficit / hyperactivity disorder) in humans and/or in animals, preferably in mammals, more preferably in humans.

Another aspect of the present invention is a medicament composed of at least one indol-5-yl sulfonamide derivative of general formula (Ic), optionally in form of one of its stereoisomers, preferably enantiomers or diastereomers, its racemate, or in form of a mixture of at least two of its stereoisomers, preferably enantiomers or diastereomers, in any mixing ratio, or a corresponding physiologically acceptable salt thereof or a corresponding solvate thereof, and optionally one or more pharmaceutically acceptable adjuvants.

This medicament is suitable for 5-HT₆ receptor regulation, for the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes caused by obesity, for the prophylaxis and/or treatment of gastrointestinal tract disorders, preferably irritable bowel syndrome, for cognitive enhancement, for the prophylaxis and/or treatment of disorders of the central nervous system, anxiety, panic disorders, depression, bipolar disorders, cognitive memory disorders, senile dementia processes, neurodegenerative disorders, preferably Alzheimer's disease, Parkinson's disease, Huntington's disease and Multiple Sclerosis, schizophrenia, psychosis or infantile hyperkinesia (ADHD, attention deficit / hyperactivity disorder) and other disorders mediated by the 5-HT₆ serotonin receptor in humans and/or in animals, preferably in mammals, more preferably in humans.

The medicament obtained according to the present invention is particularly suitable for the administration to mammals, including man. The medicament may preferably be administered to all age groups, namely, children, adolescents and adults.

Another aspect of the present invention is the use of at least one sulfonamide derivative of general formula (I), optionally in form of one of its stereoisomers, preferably enantiomers or diastereomers, its racemate, or in form of a mixture of at least two of its stereoisomers, preferably enantiomers or diastereomers, in any mixing ratio, or a corresponding physiologically acceptable salt thereof or a corresponding solvate thereof, for the manufacture of a medicament for 5-HT₆ receptor regulation, for the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes caused by obesity, for the prophylaxis and/or treatment of gastrointestinal tract disorders, preferably irritable bowel syndrome, for cognitive enhancement, for the prophylaxis and/or treatment of disorders of the central nervous system, anxiety, panic disorders, depression, bipolar disorders, cognitive memory disorders, senile dementia processes, neurodegenerative disorders, preferably Alzheimer's disease, Parkinson's disease, Huntington's disease and Multiple Sclerosis, schizophrenia, psychosis or infantile hyperkinesia (ADHD, attention deficit / hyperactivity disorder) and other disorders mediated by the 5-HT₆ serotonin receptor in humans and/or in animals, preferably in mammals, more preferably in humans.

Another aspect of the present invention is the use of at least one sulfonamide derivative of the previous general formula (Ia), optionally in form of one of its stereoisomers, preferably enantiomers or diastereomers, its racemate, or in form of a mixture of at least two of its stereoisomers, preferably enantiomers or diastereomers, in any mixing ratio, or a corresponding physiologically acceptable salt thereof or a corresponding solvate thereof, for the manufacture of a medicament for 5-HT₆ receptor regulation, for the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes caused by obesity, for the prophylaxis and/or treatment of gastrointestinal tract disorders, preferably irritable bowel syndrome, for cognitive enhancement, for the prophylaxis and/or treatment of disorders of the central nervous system, anxiety, panic disorders, depression, bipolar disorders, cognitive memory disorders, senile dementia processes, neurodegenerative disorders, preferably Alzheimer's disease, Parkinson's disease, Huntington's disease and Multiple Sclerosis, schizophrenia, psychosis or infantile hyperkinesia (ADHD, attention deficit / hyperactivity disorder) in humans and/or in animals, preferably in mammals, more preferably in humans,
preferably for 5-HT₆ receptor regulation, for the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes caused by obesity, for the prophylaxis and/or treatment of gastrointestinal tract disorders, preferably irritable bowel syndrome in humans and/or in animals, preferably in mammals, more preferably in humans.

Another aspect of the present invention is the use of at least one sulfonamide derivative of the previous general formula (Ib), optionally in form of one of its stereoisomers, preferably enantiomers or diastereomers, its racemate, or in form of a mixture of at least two of its stereoisomers, preferably enantiomers or diastereomers, in any mixing ratio, or a corresponding physiologically acceptable salt thereof or a corresponding solvate thereof, for the manufacture of a medicament for 5-HT₆ receptor regulation, for the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes caused by obesity, for the prophylaxis and/or treatment of gastrointestinal tract disorders, preferably irritable bowel syndrome, for cognitive enhancement, for the prophylaxis and/or treatment of disorders of the central nervous system, anxiety, panic disorders, depression, bipolar disorders, cognitive memory disorders, senile dementia processes, neurodegenerative disorders, preferably Alzheimer's disease, Parkinson's disease, Huntington's disease and Multiple Sclerosis, schizophrenia, psychosis or infantile hyperkinesia (ADHD, attention deficit / hyperactivity disorder) and other disorders mediated by the 5-HT₆ serotonin receptor in humans and/or in animals, preferably in mammals, more preferably in humans,
preferably for cognitive enhancement, for the prophylaxis and/or treatment of disorders of the central nervous system, anxiety, panic disorders, depression, bipolar disorders, cognitive memory disorders, senile dementia processes, neurodegenerative disorders, preferably Alzheimer's disease, Parkinson's disease, Huntington's disease and Multiple Sclerosis, schizophrenia, psychosis or infantile hyperkinesia (ADHD, attention deficit /hyperactivity disorder) in humans and/or in animals, preferably in mammals, more preferably in humans.

Another aspect of the present invention is the use of at least one sulfonamide derivative of the previous general formula (Ic), optionally in form of one of its stereoisomers, preferably enantiomers or diastereomers, its racemate, or in form of a mixture of at least two of its stereoisomers, preferably enantiomers or diastereomers, in any mixing ratio, or a corresponding physiologically acceptable salt thereof or a corresponding solvate thereof, for the manufacture of a medicament for 5-HT₆ receptor regulation, for the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes caused by obesity, for the prophylaxis and/or treatment of gastrointestinal tract disorders, preferably irritable bowel syndrome, for cognitive enhancement, for the prophylaxis and/or treatment of disorders of the central nervous system, anxiety, panic disorders, depression, bipolar disorders, cognitive memory disorders, senile dementia processes, neurodegenerative disorders, preferably Alzheimer's disease, Parkinson's disease, Huntington's disease and Multiple Sclerosis, schizophrenia, psychosis or infantile hyperkinesia (ADHD, attention deficit / hyperactivity disorder) and other disorders mediated by the 5-HT₆ serotonin receptor in humans and/or in animals, preferably in mammals, more preferably in humans.

The preparation of the corresponding pharmaceutical compositions as well as of the formulated medicaments may be carried out by means of conventional methods known in the prior art, for example, based on the indices of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan, J.C. (Eds.), Marcel Dekker, Inc. New York (2002); "Modern Pharmaceutics", Fourth Edition, Banker G.S. and Rhodes C.T. (Eds.) Marcel Dekker, Inc. New York (2002), and "The Theory and Practice of Industrial Pharmacy", Lachman L., Lieberman H. and Kanig J. (Eds.), Lea & Febiger, Philadelphia (1986). The respective literature descriptions are incorporated as a reference and are part of this disclosure.

The pharmaceutical compositions, as well as the formulated medicaments prepared according to the present invention, may, in addition to at least one sulfonamide derivative of general formula (I), optionally in form of one of its stereoisomers, preferably enantiomers or diastereomers, its racemate, or in form of a mixture of at least two of its stereoisomers, preferably enantiomers or diastereomers, in any mixing ratio, or a corresponding physiologically acceptable salt thereof or a corresponding solvate thereof, as excipients, fillers, solvents, diluents, dyes, coating agents, matrix forming agents and/or binders. As the skilled person in the art also knows, the choice of the auxiliary substances and the amounts thereof depend on the intended administration route, for example, rectal, intravenous, intraperitoneal, intramuscular, intranasal, oral, buccal or topical.

Medicaments suitable for oral administration are, for example, tablets, coated tablets, capsules or multiparticulates, preferably granules or pellets, optionally subjected to compression in tablets, filled in capsules or suspended in solutions, suspensions or suitable liquids.

Medicaments suitable for parenteral, topical or inhalatory administration may preferably be chosen from the group consisting of solutions, suspensions, quickly reconstitutable dry preparations and also sprays.

Medicaments suitable for oral or percutaneous use may release the sulfonamide compounds of general formula (I) in a sustained manner, the preparation of these sustained release medicaments generally being known in the prior art.

Suitable sustained release forms, as well as the materials and methods for the preparation thereof, are known in the art, for example from the indices of "Modified-Release Drug Delivery Technology", Rathbone, J.Jl, Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York (2000); "Controlled Drug Delivery", Vol. I, Basic Concepts, Bruck, S.D. (Ed.), CRD Press, Inc., Boca Raton (1983), and by Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective literature references are incorporated by reference and form part of the disclosure.

The medicament of the present invention may also have at least one enteric coating, which dissolves according to the pH. As a result of this coating, the medicament may pass through the stomach without dissolving, and the compounds of general formula I are only released in the intestinal tract. The enteric coating preferably dissolves at a pH of between 5 and 7.5. The materials and methods suitable for preparing enteric coatings are also known in the prior art.

Typically, the pharmaceutical compositions and the medicaments comprise from 1 to 60% by weight of one or more sulfonamide derivatives of general formula (I), and from 40 to 99% by weight of one or more excipients.

The active substance amount to be administered to the patient varies according to the patient's weight, the administration route, the indication and the severity of the disorder. Usually from 1 mg to 2 g of at least one sulfonamide derivative of general formula (I) are administered per patient per day. The total daily dose may be administered to the patient in one or more doses.

### Pharmaceutical Methods:

### BINDING TO THE 5HT₆ SEROTONIN RECEPTOR

HEK-293 cell membranes expressing the recombinant human 5HT₆ receptor were supplied by Receptor Biology. The receptor concentration in said membranes is 2.18 pmol/mg of protein and the protein concentration is 9.17 mg/ml. The experimental protocol follows the method of B.L. Roth et al. [B.L. Roth, S.C. Craigo, M.S. Choudhary, A. Uluer, F.J. Monsma, Y. Shen, H.Y. Meltzer, D.R. Sibley: Binding of Typical and Atypical Antipshychotic Agents to 5-Hydroxytryptamine-6 and Hydroxytryptamine-7 Receptors. The Journal of Pharmacology and Experimental Therapeutics, 1994, 268, 1403], with slight modifications. The commercial membrane is diluted (1:40 dilution) with the binding buffer: 50 mM Tris-HCl, 10 mM MgCl₂, 0.5 mM EDTA (pH 7.4). The radioligand used is [³H]-LSD at a concentration of 2.7 nM, the final volume being 200 µl. Incubation begins by adding 100 µl of the membrane suspension (≈ 22.9 µg of membrane protein), and is prolonged for 60 minutes at a temperature of 37°C. Incubation ends by quick filtration in a Harvester Brandel Cell through fiberglass filters of the Schleicher & Schuell GF 3362 trademark, pretreated with a 0.5% polyethyleneimine solution. The filters are washed three times with three milliliters of 50 mM Tris HCl buffer, pH 7.4. The filters are transferred to vials and 5 ml of Ecoscint H. liquid scintillation cocktail are added to each vial. The vials are left to equilibrate for several hours prior to their counting in a 1414 Wallac Winspectral scintillation counter. The non-specific binding is determined in the presence of 100 µM of serotonin. The assays are carried out in triplicate. The inhibition constants (Kᵢ, nM) are calculated by nonlinear regression analysis using the EBDA/LIGAND program [Munson and Rodbard, Analytical Biochemistry, 1980, 107, 220].

The respective literature descriptions are incorporated by reference and form part of the disclosure.

### MEASUREMENTS OF FOOD INGESTION (BEHAVIOURAL MODEL)

Male W rats (200-270 g) from Harlan, S.A. are used. The animals are acclimatized to the housings during at least 5 days prior to being subjected to any treatment. During this period, the animals are housed (in groups of five) in translucent cages and have free access to water and food. The animals are housed in individual cages at least 24 hours prior to starting the treatment.

The acute effect of the sulfonamide derivatives of formula (I) used inventively on food ingestion in rats in fasting conditions is then determined as follows:

The rats are kept in fasting conditions for 23 hours in their individual cages. After this period, the rats are orally or intraperitoneally treated with a dose of a composition containing a sulfonamide derivative of general formula (I) or a corresponding composition (vehicle) without said sulfonamide derivative. Immediately after this, the rat is left with pre-weighed food and the accumulated food intake is measured after 1, 2, 4 and 6 hours.

This food ingestion measuring method is also described in publications of Kask et al., European Journal of Pharmacology 414 (2001), 215-224, and Turnbull et al., Diabetes, Vol. 51, August, 2002. The respective bibliographic descriptions are incorporated as a reference and they form part of the disclosure.

The preparation of new compounds according to the invention is indicated in the following examples. The affinity for the 5HT₆ serotonin receptor, as well as the galenic formulas applicable to the compounds of the invention, is also described. The examples indicated below, given as an illustrative example, should in no way limit the scope of the invention.

### Examples:

### Example 2.- Preparation of N-[1-(2-dimethylaminoethyl)-1H-indole-5-yl]-naphthalene-2-sulfonamide

150 mg (0.66 mmol) of naphthalene-2-sulfonyl chloride were added to a solution of 122 mg (0.6 mMol) of 5-amino-1-(2-dimethylaminoethyl)-1 H-indole in 3 ml of dimethylformamide and 116 mg of N-ethyldiisopropylamine. The reaction mixture was stirred at the room temperature for 12 hours. Then it was evaporated to dryness, slightly alkalinized with sodium bicarbonate solution and extracted with chloroform. The organic phase was repeatedly washed with water and saturated solution of sodium bicarbonate, it was separated and dried with anhydrous sodium sulfate. The organic solution was evaporated to dryness and the resulting solid was purified by chromatography, obtaining 187 mg (80%) of N-[1-(2-dimethylaminoethyl)-1H-indole-5-yl]-naphthalene-2-sulfonamide.

### Example 10.- Preparation of N-[1-(2-dimethylaminoethyl)-1H-indole-5-yl]-benzo-[1,2,5]thiadiazole-4-sulfonamide 4-sulfonamide

116 mg (0.66 mMol) of benzo-[1,2,5]thiadiazole-4-sulfonyl chloride were added to a solution of 168 mg (0.6 mmol) of 5-amino-1-(2-dimethylaminoethyl)-1H-indole in 5 ml of pyridine and 311 mg of N-ethyldiisopropylamine. The reaction mixture was stirred at the room temperature for 2 hours. Then it was evaporated to dryness, slightly alkalinized with sodium bicarbonate solution and extracted with chloroform. The organic phase was repeatedly washed with water and saturated solution of sodium bicarbonate, it was separated and dried with anhydrous sodium sulfate. The organic solution was evaporated to dryness and the resulting solid was treated with diethyl ether obtaining 183 mg (76%) of N-[1-(2-dimethylaminoethyl)-1H-indole-5-yl]-benzo-[1,2,5]thiadiazole-4-sulfonamide 4-sulfonamide.

### Example 17.- Preparation of N-[1-(2-pyrrolidine-1-yl-ethyl)-1H-indole-5-yl]-naphthalene-1-sulfonamide

199 mg (0.88 mMol) of naphthalene-1-suifonyl chloride were added to a solution of 335 mg (0.8 mMol) of 5-amino-1-(2- pyrrolidine-1-yl-ethyl)-1H-indole in 10 ml of methylene chloride and 0,44 mg of triethylamine. The reaction mixture was stirred at the room temperature for 12 hours. Then it was slightly alkalinized with sodium bicarbonate solution and extracted with methylene chloride. The organic phase was repeatedly washed with water and saturated solution of sodium bicarbonate, it was separated and dried with anhydrous sodium sulfate. The organic solution was evaporated to dryness and the resulting solid was treated with diethyl ether obtaining 264 mg (79%) of N-[1-(2-pyrrolidine-1-yl-ethyl)-1H-indole-5-yl]-naphthalene-1-sulfonamide as a solid.

### Example 29. Preparation of N-(1-(2-(diethylamino)ethyl)-1H-indol-5-yl)-naphthalene-2-sulfonamide

The reaction was carried out according to the procedure given in Example 1. 139 mg (0.6 mMol) of 5-amino-1-(2-(diethylamino)ethyl)-1H-indole and 150 mg (0.66 mMol) of 2-naphthyl-sulfonyl chloride were reacted to give 115 mg (45 %) of the desired compound as a solid.

### Example 30. Preparation of N-(1-(2-(diethylamino)ethyl)-1H-indol-5-yl)-naphthalene-1-sulfonamide

The reaction was carried out according to the procedure given in Example 1. 139 mg (0.6 mMol) of 5-amino-1-(2-(diethylamino)ethyl)-1H-indole and 150 mg (0.66 mMol) of 2-naphthyl-sulfonyl chloride were reacted to give 160 mg (63 %) of the desired compound as a solid.

### Example 31. Preparation of N-(1-(2-(diethylamino)ethyl)-1H-indol-5-yl)-4-phenylbenzenesulfonamide

The reaction was carried out according to the procedure given in Example 1. 139 mg (0.6 mMol) of 5-amino-1-(2-(diethylamino)ethyl)-1H-indole and 167 mg (0.66 mMol) of 4-phenylbenzenesulfonyl chloride were reacted to give 181 mg (68 %) of the desired compound as an oil.

### Example 32. Preparation of 5-chloro-N-(1-(2-(dimethylamino)ethyl)-2-methyl-1 H-indol-5-yl)-3-methylbenzo[b]thiophene-2-sulfonamide

The reaction was carried out according to the procedure given in Example 1. 130 mg (0.6 mMol) of 5-amino-1-(2-(dimethylamino)ethyl)-2-methyl-1H-indole and 186 mg (0.66 mMol) of 5-chloro-2-methylbenzo[b]thiophene-2-sulfonyl chloride were reacted to give 127 mg (46 %) of the desired compound as a solid.

### Example 33. Preparation of N-(1-(2-(dimethylamino)ethyl)-2-methyl-1H-indol-5-yl)-naphthalene-2-sulfonamide

The reaction was carried out according to the procedure given in Example 1. 130 mg (0.6 mMol) of 5-amino-1-(2-(dimethylamino)ethyl)-2-methyl-1H-indole and 150 mg (0.66 mMol) of naphthyl-2-sulfonyl chloride were reacted to give 142 mg (58 %) of the desired compound as a solid.

### Example 34. Preparation of N-(1-(2-(dimethylamino)ethyl)-2-methyl-1H-indol-5-yl)-naphthalene-1-sulfonamide

The reaction was carried out according to the procedure given in Example 1. 130 mg (0.6 mMol) of 5-amino-1-(2-(dimethylamino)ethyl)-2-methyl-1H-indole and 150 mg (0.66 mMol) of naphthyl-1-sulfonyl chloride were reacted to give 81 mg (33 %) of the desired compound as a solid.

### Example 35. Preparation of 6-chloro-N-(1-(2-(dimethylamino)ethyl)-2-methyl-1 H-indol-5-yl)imidazo[2,1-b]thiazole-5-sulfonamide

The reaction was carried out according to the procedure given in Example 1. 130 mg (0.6 mMol) of 5-amino-1-(2-(dimethylamino)ethyl)-2-methyl-1H-indole and 170 mg (0.66 mMol) of 6-chloro-imidazo[2,1-b]thiazole-5-sulfonyl chloride were reacted to give 96 mg (37 %) of the desired compound as a solid.

### Example 36. Preparation of N-(1-(2-(dimethylamino)ethyl)-2-methyl-1H-indol-5-yl)-4-phenylbenzenesulfonamide

The reaction was carried out according to the procedure given in Example 1. 130 mg (0.6 mMol) of 5-amino-1-(2-(dimethylamino)ethyl)-2-methyl-1H-indole and 167 mg (0.66 mMol) of 4-phenylbenzenesulfonyl chloride were reacted to give 160 mg (62 %) of the desired compound as a solid.

### Example 37. Preparation of N-(1-(2-dimethylamino)ethyl)-2-methyl-1H-indol-5-yl)-2-(naphth-1-yl)-ethanesulfonamide

The reaction was carried out according to the procedure given in Example 1. 130 mg (0.6 mMol) of 5-amino-1-(2-(dimethylamino)ethyl)-2-methyl-1H-indole and 168 mg (0.66 mMol) of 2-(naphth-1-yl)-ethanesulfonyl chloride were reacted to give 108 mg (41 %) of the desired compound as a solid.

### Example 38. Preparation of N-(1-(2-(dimethylamino)ethyl)-2-methyl-1H-indol-5-yl)-4-phenoxy-benzenesulfonamide

The reaction was carried out according to the procedure given in Example 1. 130 mg (0.6 mMol) of 5-amino-1-(2-(dimethylamino)ethyl)-2-methyl-1H-indole and 177 mg (0.66 mMol) of 4-phenoxy-benzenesulfonyl chloride were reacted to give 89 mg (33 %) of the desired compound as a solid.

### Example 39. Preparation of 3,5-dichloro-N-(1-(2-(dimethylamino)ethyl)-2-methyl-1 H-indol-5-yl)-benzenesulfonamide

The reaction was carried out according to the procedure given in Example 1. 130 mg (0.6 mMol) of 5-amino-1-(2-(dimethylamino)ethyl)-2-methyl-1H-indole and 162mg (0.66 mMol) of 3,5-dichloro-benzenesulfonyl chloride were reacted to give 81 mg (32 %) of the desired compound as a solid.

### Example 40. Preparation of N-(1-(2-(dimethylamino)ethyl)-2-methyl-1H-indol-5-yl)benzo[b]thiophene-3-sulfonamide

The reaction was carried out according to the procedure given in Example 1. 108 mg (0.5 mMol) of 5-amino-1-(2-(dimethylamino)ethyl)-2-methyl-1H-indole and 128 mg (0.55 mMol) of benzo[b]thiophene-3-sulfonyl chloride were reacted to give 82 mg (39 %) of the desired compound as a solid.

### Example 41. Preparation of N-(1-(2-(diethylamino)ethyl)-1H-indol-5-yl)benzo[b]thiophene-3-sulfonamide

The reaction was carried out according to the procedure given in Example 1. 115 mg (0.5 mMol) of 5-amino-1-(2-(diethylamino)ethyl)-1H-indole and 128 mg (0.55 mMol) of benzo[b]thiophene-3-sulfonyl chloride were reacted to give 91 mg (43 %) of the desired compound as a solid.

### Example 42. Preparation of N-(1-(2-(dimethylamino)ethyl)-1H-indol-5-yl)benzo[b]thiophene-3-sulfonamide

The reaction was carried out according to the procedure given in Example 1. 102 mg (0.5 mMol) of 5-amino-1-(2-(diethylamino)ethyl)-1H-indole and 128 mg (0.55 mMol) of benzo[b]thiophene-3-sulfonyl chloride were reacted to give 91 mg (43 %) of the desired compound as a solid.

### Example 43. Preparation of 5-chloro-3-methyl-N-(1-(3-(piperidin-1-yl)propyl)-1H-indol-5-yl)benzo[b]thiophene-2-sulfonamide

The reaction was carried out according to the procedure given in Example 1. 118 mg (0.46 mMol) of 5-amino-1-(3-(piperidin-1-yl)propyl))-1H-indole and 143 mg (0.51 mMol) of 5-chloro-3-methyl-benzo[b]thiophene-2-sulfonyl chloride were reacted to give 89 mg (38 %) of the desired compound as a solid.

### Example 44. Preparation of N-(1-(3-(piperidin-1-yl)propyl)-1H-indol-5-yl)naphthalene-2-sulfonamide

The reaction was carried out according to the procedure given in Example 1. 118 mg (0.46 mMol) of 5-amino-1-(3-(piperidin-1-yl)propyl))-1H-indole and 116 mg (0.51 mMol) of naphthyl-2-sulfonyl chloride were reacted to give 75 mg (37 %) of the desired compound as a solid.

### Example 45. Preparation of N-(1-(3-(piperidin-1-yl)propyl)-1H-indol-5-yl)naphthalene-1-sulfonamide

The reaction was carried out according to the procedure given in Example 1. 118 mg (0.46 mMol) of 5-amino-1-(3-(piperidin-1-yl)propyl))-1H-indole and 116 mg (0.51 mMol) of naphthyl-2-sulfonyl chloride were reacted to give 91 mg (44 %) of the desired compound as a solid.

### Example 46. Preparation of 6-chloro-N-(1-(3-piperidin-1-yl)propyl)-1H-indol-5-yl)imidazo[2,1-b]thiazole-5-sulfonamide

The reaction was carried out according to the procedure given in Example 1. 118 mg (0.46 mMol) of 5-amino-1-(3-(piperidin-1-yl)propyl))-1H-indole and 131 mg (0.51 mMol) of 6-chloro-imidazo[2,1-b]thiazole-5-sulfonyl chloride were reacted to give 91 mg (44 %) of the desired compound as a solid.

### Example 47. Preparation of 4-phenyl-N-(1-(3-(piperidin-1-yl)propyl)-1H-indol-5-yl)benzenesulfonamide

The reaction was carried out according to the procedure given in Example 1. 118 mg (0.46 mMol) of 5-amino-1-(3-(piperidin-1-yl)propyl))-1H-indole and 129 mg (0.51 mMol) of 4-phenylbenzenesulfonyl chloride were reacted to give 106 mg (49 %) of the desired compound as a solid.

### Example 48. Preparation of 2-(naphth-1-yl)-N-(1-(3-(piperidin-1-yl)propyl)-1H-indol-5-yl)ethanesulfonamide

The reaction was carried out according to the procedure given in Example 1. 118 mg (0.46 mMol) of 5-amino-1-(3-(piperidin-1-yl)propyl))-1H-indole and 130 mg (0.51 mMol) of 2-(naphth-1-yl)ethanesulfonyl chloride were reacted to give 68 mg (31 %) of the desired compound as a solid.

### Example 49. Preparation of 4-phenoxy-N-(1-(3-(piperidin-1-yl)propyl)-1H-indol-5-yl)benzenesulfonamide

The reaction was carried out according to the procedure given in Example 1. 118 mg (0.46 mMol) of 5-amino-1-(3-(piperidin-1-yl)propyl))-1H-indole and 137 mg (0.51 mMol) of 4-phenoxybenzenesulfonyl chloride were reacted to give 86 mg (38 %) of the desired compound as a solid.

### Example 50. Preparation of 3,5-dichloro-N-(1-(3-(piperidin-1-yl)propyl)-1H-indol-5-yl)benzenesulfonylamide

The reaction was carried out according to the procedure given in Example 1. 118 mg (0.46 mMol) of 5-amino-1-(3-(piperidin-1-yl)propyl))-1H-indole and 125 mg (0.51 mMol) of 3,5-dichlorobenzenesulfonyl chloride were reacted to give 79 mg (37 %) of the desired compound as a solid.

### Example 51. Preparation of 4,5-dichloro-N-(1-(3-(piperidin-1-yl)propyl)-1H-indol-5-yl)thiophene-2-sulfonamide

The reaction was carried out according to the procedure given in Example 1. 118 mg (0.46 mMol) of 5-amino-1-(3-(piperidin-1-yl)propyl))-1H-indole and 128 mg (0.51 mMol) of 4,5-dichlorothiophene-2-sulfonyl chloride were reacted to give 68 mg (31 %) of the desired compound as a solid.

### Example 52. Preparation of 5-chloro-N-(1-(3-(piperidin-1-yl)propyl)-1H-indol-5-yl)naphthalene-1-sulfonamide

The reaction was carried out according to the procedure given in Example 1. 118 mg (0.46 mMol) of 5-amino-1-(3-(piperidin-1-yl)propyl))-1H-indole and 133 mg (0.51 mMol) of 5-chloro-napthyl-1-sulfonyl chloride were reacted to give 81 mg (37 %) of the desired compound as a solid.

The yields are indicative and no added effort was made to improve them.

The melting point and spectroscopic data for identifying some of the compounds of the present invention are indicated in the following table.

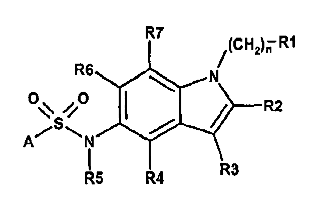

| Ex | R1 | R2 | R3 | R4 | R5 | R6 | R7 | n | A | m.p. °C | IR cm⁻¹ | ¹H-NMR (300 MHz),δ (solvent) |
|---|---|---|---|---|---|---|---|---|---|---|---|---|
| 1 | (CH₃)₂N- | H | H | H | H | H | H | 2 | 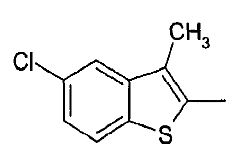 | 71-73 | 2950, 1334, 1160, 1080,862, 652, 560. | 2,11(s, 6H); 2,36(s, 3H); 2,51 (m, 2H); 4,14(t, 2H, J=6,6 Hz); 6,30(d, 1H, J=3 Hz); 7,32 (m, 2H); 7,50(dd, 1H, J=8,7 Hz, J'=2,0 Hz); 7,93(d, 1H, J=2,0 Hz); 7.99(d, 1H, J=8.7 Hz). (DMSO-d6) |
| 2 | (CH₃)₂N- | H | H | H | H | H | H | 2 | 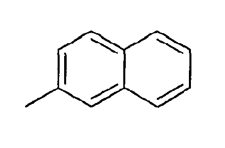 | 54-57 | 3254, 3049, 2945, 1463, 1330, 1160, 1074, 658, 550. | 2,26(s, 6H); 2,63(t, 2H, J=7,1 Hz); 4,14(t, 2H, J=7,1 Hz); 6,35(d, 1H, J=3,1 Hz); 6,88 (dd, 1H, J=8,6 Hz, J'=2,0 Hz); 7,10(d, 1H, 3,1 Hz); 7,15(d, 1H, J=8,6 Hz); 7,31 (d, 1H, J=2,0Hz); 7,50-7,63(m, 2H); 7,69(dd, 1H, J=8,7 Hz, J'=1,8 Hz); 7,84(m, 3H); 8,29(s, 1H). (CDCl₃) |
| 3 | (CH₃)₂N- | H | H | H | H | H | H | 2 | 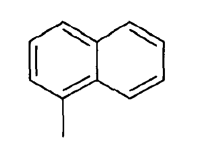 | 179-181 | 3106, 2783, 1491, 1318, 1159, 1130, 763, 586, 503. | 2,25(s, 6H); 2,63(t, 2H, J=7,0 Hz); 4,11(t, 2H, J=7,0 Hz); 6,28(d, 1H, J=3,1 Hz); 6,68 (dd, 1H, J=8,6 Hz, J'=2,0 Hz); 7,03-7,11(m, 3H); 7,37(m, 1H); 7,58-7,70(m, 2H); 7,94(d, 1H, J=8,7 Hz); 8,00(d, 1H, J=7,9 Hz); 8,06(d, 1H, J=7,3 Hz); 8,73(d, 1H, J=8,7 Hz). (CDCl₃) |
| 4 | (CH₃)₂N- | H | H | H | H | H | H | 2 | 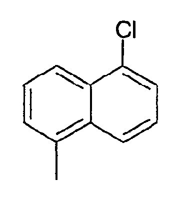 | 172-174 | 3257, 2935, 2768, 1488, 1334, 1167, 1138, 1013, 790, 606. | 2,26(s, 6H); 2,63(t, 2H, J=7,1 Hz); 4,13(t, 2H, J=7,1 Hz); 6,30(d, 1H, J=3,1 Hz); 6,66 (dd, 1H, J=8,6 Hz, J'=2,0 Hz); 7,05(d, 1H, J=8,7 Hz); 7,08(d, 1 H, J=3,1 Hz); 7,11(d, 1H, J=2,0 Hz); 7,46-7,58(m, 2H); 7,69(d, 1H, J=7,5 Hz); 8,13(d, 1H, J=7,5 Hz); 8,50(d, 1H, J=8,6 Hz); 8,69(d, 1H, J=8,8 Hz). (CDCl₃) |
| 5 | (CH₃)₂N- | H | H | H | H | H | H | 2 | 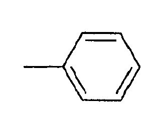 | 139-141 | 1463, 1334, 1306, 1164, 1090, 725, 589. | 2,28(s, 6H); 2,66(t, 2H, J=7,1 Hz); 4,17(t, 2H, J=7,1 Hz); 6,38(d, 1H, J=3,1 Hz); 6,88 (dd, 1H, J=8,6 Hz, J'=2,0 Hz); 7,13(d, 1H, J=3,1 Hz); 7,18(d, 1H, J=8,6 Hz); 7,27(m, 1 H); 7,39(m, 2H); 7,48(m, 1 H); 7,69(m, 2H). (CDCl₃) |
| 6 | (CH₃)₂N- | H | H | H | H | H | H | 2 | 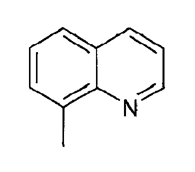 | 161-164 | 3095, 02821, 2776, 1492, 1459, 1322, 1158, 1141, 782, 736, 596, 507. | 2,23(s, 6H); 2,58(t, 2H, J=7,1 Hz); 4,08(t, 2H, J=7,1 Hz); 6,24(d, 1H, J=3,1 Hz); 6,88 (dd, 1H, J=8,8, J'=2,0 Hz); 7,03(d, 1H, J=3,1 Hz); 7,07(d, 1H, J=8,8 Hz); 7,10(d, 1H, J=2,0 Hz); 7,51(t, 1H, J=7,8 Hz); 7,64(dd, 1 H, J=8,5, J'=4,3 Hz); 8,00(d, 1H, J=8,2 Hz); 8,26(m, 1H); 8,30(dd, 1 H, J=8,2, J'=1,5 Hz); 8,40(s, 1 H); 9,20(dd, 1H, J=4,1 J'=1,4 Hz). (CDCl₃) |
| 7 | (CH₃)₂N- | H | H | H | H | H | H | 2 | 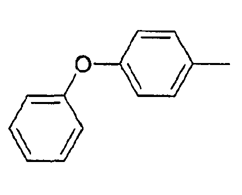 | 138-140 | 3255, 2951, 1583, 1488, 1332, 1245, 1156, 1092,866, 695,569. | 2,28(s, 6H); 2,67(t, 2H, J=7,1 Hz); 4,18(t, 2H, J=7,1 Hz); 6,40(d, 1H, J=3,1 Hz); 6,92 (m, 3H); 7,02(d, 2H, J=7,7 Hz); 7,14(d, 1H, J=3,1 Hz); 7,20(d, 2H, J=8,5 Hz); 7,28(d, 1H, J=1,9 Hz); 7,37(m, 2H); 7,64(d, 2H, J=8,6Hz). (CDCl₃) |
| 8 | (CH₃)₂N- | H | H | H | H | H | H | 2 | 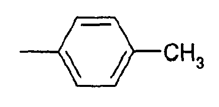 | 126-128 | 1474, 1287, 1156, 1088, 973, 730, 654, 554, 538. | 2,31(s, 6H); 2,36(s, 3H); 2,72(t, 2H, J=7,1 Hz); 4,20(t, 2H, J=7,1 Hz); 6,39(d, 1H, J=3,1 Hz); 6,90 (dd, 1H, J=8,6 Hz, J'=1,6 Hz); 7,13(d, 1H, J=3,1 Hz); 7,16-7,20(m, 3H); 7,26(m, 1 H); 7,57(d, 2H, J=8,3 Hz). (CDCl₃) |
| 9 | (CH₃)₂N- | H | H | H | H | H | H | 2 | 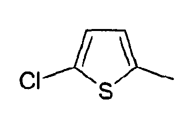 | 145-147 | 3095, 2951, 1416, 1319, 1148, 989, 730, 605, 537. | 2,33(s, 6H); 2,74(m, 2H); 4,24(t, 2H, J=7,1 Hz); 6,44(d, 1H, J=3,1 Hz); 6,79 (d, 1 H, J=4,0 Hz); 6,95(dd, 1 H, J=8,7 Hz, J'=2,0 Hz); 7,15(d, 1H, J=4,0 Hz); 7,17(d, 1 H, J=3,1 Hz); 7,24(d, 1H, J=8,7 Hz); 7,35(d, 1H, J=2,0 Hz). (DMSO-d6) |
| 10 | (CH₃)₂N- | H | H | H | H | H | H | 2 | 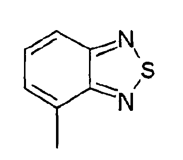 | 166-168 | 3103,2783 , 1526, 1488, 1331, 1154, 1140,973, 734, 607. | 2,26(s, 6H); 2,63(t, 2H, J=7,1 Hz); 4,12(t, 2H, J=7,1 Hz); 6,29(d, 1H, J=3,1 Hz); 6,80 (dd, 1H, J=8,7 Hz, J'=2,0 Hz); 7,07(m, 2H); 7,15(d, 1H, J=1,5 Hz); 7,57(dd, 1H, J=8,8 Hz, J'=7,1Hz); 8,10(d, 1H, J=7,1 Hz); 8,16(d, 1H, J=8,8 Hz). (CDCl₃) |
| 11 | (CH₃)₂N- | H | H | H | H | H | H | 2 | 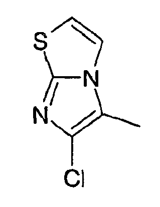 | 50-52 | 3103, 2943, 1457, 1336, 1326, 1244, 1177, 1142, 727 628, 528. | 2,26(s, 6H); 2,64(t, 2H, J=6,4 Hz); 4,16(t, 2H, J=6,4 Hz); 6,39(m, 1H); 6,78 (d, 1H, J=4,0 Hz); 6,94(d, 1H, J=8,4 Hz); 7,15(m, 2H); 7,39(s, 1H); 7,55(d, 1H, J=4,0 Hz). (CDCl₃) |
| 12 | (CH₃)₂N- | H | H | H | H | H | H | 2 | 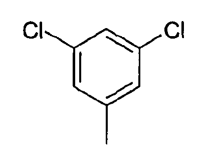 | 124-126 | 3064, 2935, 1333, 1166, 1136, 596, 587. | 2,28(s, 6H); 2,67(t, 2H, J=7,0 Hz); 4,19(t, 2H, J=7,0 Hz); 6,43(d, 1H, J=3,1 Hz); 6,85 (dd, 1 H, J=8,6 Hz, J'=2,0 Hz); 7,17(d, 1H, J=3,1 Hz); 7,22(d, 1H, J=8,6 Hz); 7,31(d, 1H, J=2,0 Hz); 7,48(t, 1H, J=1,8 Hz); 7,56(d, 2H, J=1,8 Hz). (CDCl₃) |
| 13 | (CH₃)₂N- | H | H | H | H | H | H | 2 | 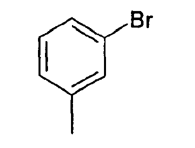 | 114-116 | 1464, 1335, 1286, 1161,722 584. | 2,28(s, 6H); 2,67(t, 2H, J=7,0 Hz); 4,19(t, 2H, J=7,0 Hz); 6,41 (d, 1H, J=2,9 Hz); 6,87 (d, 1H, J=8,8 Hz); 7,15(d, 1H, J=2,9 Hz); 7.19-7.29(m, 3H); 7,56(d, 1H, J=7,8 Hz); 7,63(d, 1H, J=7,9 Hz); 7,88(s,1H), (CDCl₃) |
| 14 | (CH₃)₂N- | H | H | H | H | H | H | 2 | 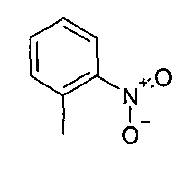 | 138-140 | 1541, 1481, 1365, 1330, 1235, 1150, 1124, 736, 580. , | 2,28(s, 6H); 2,66(t, 2H, J=7,1 Hz); 4,17(t, 2H, J=7,1 Hz); 6,40(d, 1H, J=2,9 Hz); 7,03 (dd, 1H, J=8,7 Hz, J'=1,8 Hz); 7,15(d, 1H, J=2,9 Hz); 7,21 (d, 1H, J=8,7 Hz); 7,39(d, 1H, J=1,8Hz); 7,48(m, 1 H); 7,65(m, 1 H); 7,71(d, 1H, J=8,8 Hz); 7,86(d,1H, J=7,8Hz). (CDCl₃) |
| 15 | (CH₃)₂N- | H | H | H | H | H | H | 2 | 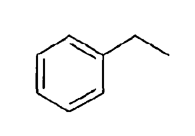 | 163-166 | 1329, 1288, 1153, 1126, 694, 545, 509. | 2,30(s, 6H); 2,70(t, 2H, J=7,1 Hz); 4,22(t, 2H, J=7,1 Hz); 4,29(s, 2H); 6,48(d, 1 H, J=3,1 Hz); 7,04 (dd, 1 H, J=8,8 Hz, J'=2,2 Hz); 7,19(d, 1H, J=3,1 Hz); 7,31(d, 1H, J=8,8 Hz); 7,33-7,40(m, 5H); 7,49(d, 1H, J=2,2 Hz). (CDCl₃) |
| 16 | 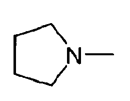 | H | H | H | H | H | H | 2 | 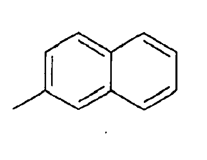 | 138-140 | 2960, 1481, 1323, 1161, 1151, 1074, 659, 549,480. | 1,57(m, 4H); 2,37(m, 4H); 2,66(t, 2H, J=6,8 Hz); 4,12(t, 2H, J=6,8 Hz); 6,25(d, 1 H, J=3,1 Hz); 6,82 (dd, 1H, J=8,8 Hz, J'=2,0 Hz); 7,22(d, 1H, 2,0 Hz); 7,25(d, 1H, J=8,6 Hz); 7,29(d, 1 H, J=3,1Hz); 7,54-7,66(m, 2H); 7,74(dd, 1H, J=8,7 Hz, J'=1,8 Hz); 7,94(m, 1 H); 8,03(m, 2H); 8,28(s, 1H). (CDCl₃) |
| 17 | 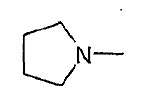 | H | H | H | H | H | H | 2 | 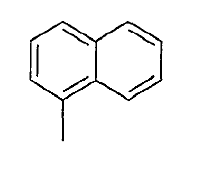 | 186-189 | 2814, 1491, 1291, 1158, 1128, 763, 585. | 1,59(m, 4H); 2,39(m, 4H); 2,67(t, 2H, J=6,8 Hz); 4,11(t, 2H, J=6,8 Hz); 6,21(d, 1H, J=3,1 Hz); 6,70 (dd, 1H, J=8,8 Hz, J'=1,8 Hz); 7,10(d, 1H, 1,8 Hz); 7,20(d, 1H, J=8,8 Hz); 7,27(d, 1 H, J=3,1 Hz); 7,50(m, 1 H); 7,60-7,74(m, 2H); 8,03(m 2H); 8,11(d, 1H, J=8,1 Hz); 8,76(d, 1H, J=8,6 Hz). (CDCl₃) |
| 18 | 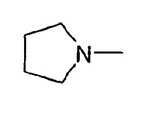 | H | H | H | H | H | H | 2 | 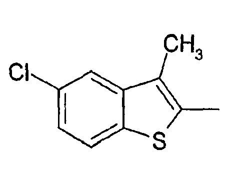 | 156-158 | 2950, 2803, 1491, 1325, 1156, 1078, 650, 564. | 1,59(m, 4H); 2,36(m, 4H); 2,69(t, 2H, J=6,6 Hz); 4,11(t, 2H, J=6,6 Hz); 6,30(d, 1H, J=3,1 Hz); 6,83 (dd, 1H, J=8,7 Hz, J'=1,9 Hz); 7,25(d, 1H, 1,9 Hz); 7,32(m, 2H); 7,50(dd, 1 H, J=8,6Hz, J'=2,0 Hz); 7,92(d, 1H, J=2,0 Hz); 7,98(d, 1H, J=8,8 Hz). (CDCl₃) |
| 19 | (CH₃)₂N- | H | H | H | H | H | H | 2 | 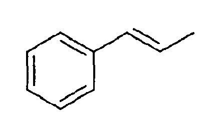 | 108-111 | 2943, 2821, 1516, 1139, 752, 728, 542, 531. | 2,28(s, 6H); 2,68(t, 2H, J=7,1 Hz); 4,19(t, 2H, J=7,1 Hz); 6.43(d, 1H, J=3,1 Hz); 6,82(d, 1H, J=16,6 Hz); 7,09(dd, 1H, J=8,6, J'=2,0Hz); 7,15(d, 1H, J=3,3 Hz); 7,25(m, 1 H); 7,33-7,44(m, 6H); 7,49(d, 1H, J=2,0 Hz). (CDCl₃) |
| 20 | (CH₃)₂N- | H | H | H | H | H | H | 2 | 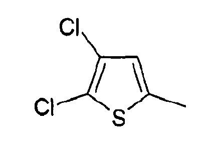 | 120-123 | 3095, 2943, 1421, 1325, 1148, 1020, 730, 609, 534. | 2,30(s, 6H); 2,69(t, 2H, J=7,1 Hz); 4,21(t, 2H, J=7,1 Hz); 6.46(d, 1H, J=3,1 Hz); 6,93(dd, 1H, J=8,6, J'=2,0Hz); 7,17(s, 1H); 7,18(d, 1 H, J=3,3 Hz); 7,25(m, 1H); 7,39(d, 1 H, J=2,0 Hz). (CDCl₃) |
| 21 | (CH₃)₂N- | H | H | H | H | H | H | 2 | 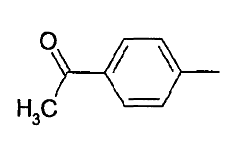 | 175 (decompo sition) | 1686, 1164, 1094, 637, 534, 475. | 2,12(s, 6H); 2,53(t, 2H, J=6,6 Hz); 4,15(t, 2H, J=6,6 Hz); 6.30(d, 1H, J=3,1 Hz); 6,80 (dd, 1H, J=8,6, J'=2,2 Hz); 7,19(d, 1H, J=2,0 Hz); 7,30-7,34(m, 2H); 7,57(AB sist., 2H, J=8,4Hz); 7,70(AB sist., 2H, J=8,4Hz). (DMSO-d6). |
| 22 | (CH₃)₂N- | H | H | H | H | H | H | 2 | 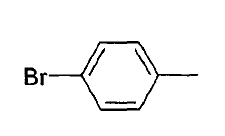 | 114-117 | 2943, 1573, 1469, 1321, 1161, 1088, 737, 630, 538. | 2,13(s, 6H); 2,50-2,55(m, 5H); 4,14(t, 2H, J=6,3 Hz); 6.29(d, 1H, J=3,0 Hz); 6,82 (d, 1H, J=8,2 Hz); 7,20(s, 1 H); 7,29-7,32(m, 2H); 7,79(AB sist., 2H, J=8,4Hz); 8,02(AB sist., 2H, J=8,4Hz). (DMSO-d6). |
| 23 | (CH₃)₂N- | H | H | H | H | H | H | 2 | 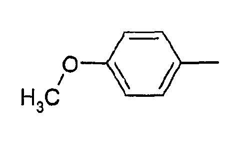 | 141-144 | 2935, 1598, 1497, 1333, 1258, 1159, 1093, 1018,836, 568, 560. | 2,28(s, 6H); 2,67(t, 2H, J=7,1 Hz); 4,18(t, 2H, J=7,1 Hz); 6.38(d, 1H, J=3,1 Hz); 6,84 (AB Sist., 2H, J=9,0 Hz); 6,90(dd, 1H, J=8,8, J'=2,0 Hz); 7,13(d, 1H, J=3,3 Hz); 7,19(d, 1H, J=8,8 Hz); 7,27(d, 1H, J=2,0 Hz); 7,62(AB sist., 2H, J=9 Hz). (CDCl₃) |
| 24 | (CH₃CH₂)₂N- | H | H | H | H | H | H | 2 | 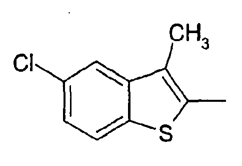 | 53-56 | 2969, 1486, 1334, 1161, 114 1080, 862, 729, 652, 560. | 0,78(m, 6H); 2,32(s, 3H); 2,42(m, 4H); 2,65(m, 2H); 4,12(m, 2H); 6.30(d, 1 H, J=3,0 Hz); 6,82 (d, 1 H, J=8,6 Hz); 7,25(d, 1H, 1,7 Hz); 7,32(m, 2H); 7,50(dd, 1H, J=8,7Hz, J'=1,9 Hz); 7,91 (d, 1H, J=1,7 Hz); 7,99(d, 1 H, J=8,6 Hz). (CDCl₃) |
| 25 | (CH₃)₂N- | H | H | H | H | H | H | 2 | 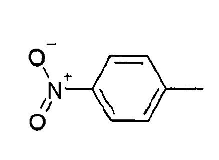 | 60-64 | 3095, 2768, 1529, 1349, 1165, 1090, 736 | 2,29(s, 6H); 2,67(t, 2H, J=7,0 Hz); 4,18(t, 2H, J=7,0 Hz); 6.40(d, 1H, J=3,1 Hz); 6,85 (dd, 1H, J=8,6 Hz, J'=2,0 Hz); 7,16(d, 1H, J=3,1 Hz); 7,18(d, 1H, J=8,6 Hz); 7,29(d, 1H, J=2,0 Hz) 7,85(AB sys, J=8.8 Hz, 2 H); 8,21 (AB sys, J=8.8 Hz, 2 H). (CDCl₃) |
| 26 | (CH₃)₂N- | H | H | H | H | H | H | 2 | 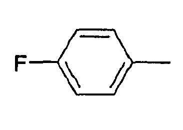 | 138-140 | 3103, 2951, 1587, 1491, 1335, 1166, 1089,557, 542. | 2,35(s, 6H); 2,83(m, 2H); 4,28(t, 2H, J=6,7 Hz); 6.40(d, 1H, J=3,0 Hz); 6,83 (dd, 1H, J=8,6 Hz, J'=2,0 Hz); 7,20(d, 1H, J=1,9 Hz); 7,30-7,38(m, 4H); 7,70-7,75(m, 2H). (DMSO-d6). |
| 27 | (CH₃CH₂)₂N- | H | H | H | H | H | H | 2 | 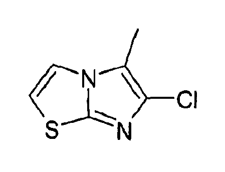 | 68-70 | 3110, 2969, 1458, 1271, 1249, 1179, 1140,727, 651. | 1,00(t, 6H, J=7,0 Hz); 2,60(q, 4H, J=7,0 Hz); 2,81(t, 2H, J=6,7 Hz); 4,21(t, 2H, J=6,7 Hz); 6.38(d, 1H J=3,0 Hz); 6,79 (d, 1H, J=4,5 Hz); 6,96(dd, 1H, J=8,6, J'=1,7 Hz); 7,14(d, 1H, 3,0 Hz); 7,19(d, 1H, J=8,8 Hz); 7,40(d, 1H, J=1,5 Hz); 7,59(d, 1 H, J=4,4 Hz). (CDCl₃) |
| 28 | 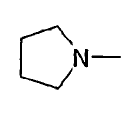 | H | H | H | H | H | H | 2 | 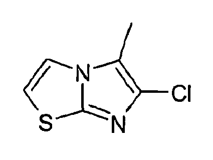 | 81-84 | 3119, 2951, 2798, 1458, 1271, 1248, 1178, 1140,727, 623. | 1,85(m, 6H); 2,68(m, 4H); 3,00(m, 2H); 4,38(m, 2H); 6.40(d, 1H J=3,1 Hz); 6,82 (d, 1H, J=4,5 Hz); 6,96(d, 1H, J=8,6 Hz); 7,19(d, 1H, 2,7 Hz); 7,22(m, 1H); 7,41(m, 1H); 7,64(d, 1H, J=4,5 Hz). (CDCl₃) |
| 29 | (CH₃CH₂)₂N- | H | H | H | H | H | H | 2 | 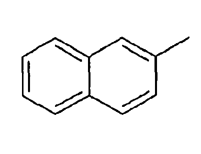 | 97-104 | | 0,95(t, 6H, J=7,1 Hz); 2,54(q, 4H, J=7,0 Hz); 2,76(t, 2H, J=6,7 Hz); 4,07(t, 2H, J=6,7 Hz); 6,66(dd, 1H, J=8,5 J'=1,7 Hz); 6,91 (s, 1H); 6,97(s, 1H); 7,01 (d, 1H, J=8,8 Hz); 7,22(dd, 1H, J=8,6 , J'=1,6 Hz); 7,26(s, 1H); 7,42-7,55(m, 3H); 7,63(d, 1H, J=8,1 Hz); 7,70(d, 1H, J=8,2 Hz); 8,03(s, 1H); 9,95(s, 1H). (CDCl3) |
| 30 | (CH₃CH₂)₂N- | H | H | H | H | H | H | 2 | 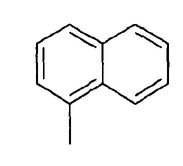 | 133-135 | | 0,87(m, 6H); 2,58(m, 4H); 2,76(m, 2H); 4,14(m, 2H); 6,24(s, 1H); 6,73(d, 1H, J=8,8 Hz); 7,11(s, 1H); 7,21 (d, 1H, J=8,0 Hz); 7,29(s, 1H); 7,50(t, 1H, J=7,8 Hz); 7,63-7,71(m, 2H); 8,04(d, 2H, J=7,5 Hz); 8,13(d, 1H, J=8,2 Hz); 8,76(d, 1H, J=8,2 Hz); 10,21 (s, 1 H). (DMSO-d6) |
| 31 | (CH₃CH₂)₂N- | H | H | H | H | H | H | 2 | 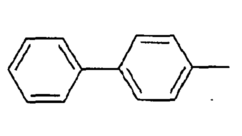 | Oil | | 0,83(m, 6H); 2,50(m, 4H); 2,70(m, 2H); 4,13(m, 2H); 6,30(d, 1H, J=2,6 Hz); 6,87(d, 1H, J=8,6 Hz); 7,24(s, 1 H); 7,30(m, 2H); 7,44(m, 3H); 7,66(d, 2H, J=7,2 Hz); 7,72(AB sys, 2H, J=8,5 Hz); 7,78(AB sys, 2H, J=8,5 Hz); 9,91 (s, 1H). (DMSO-d6) |
| 32 | (CH₃)₂N- | CH₃ | H | H | H | H | H | 2 | 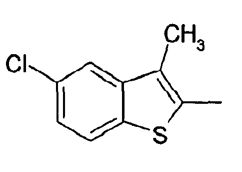 | 203-205 | | 2,13(s, 6H); 2,33(s, 6H); 2,39(t, 2H, J=7,0 Hz); 4,07(t, 2H, J=6,8 Hz); 6,08(s, 1H); 6,76(dd, 1H, J=8,6 , J'=2,0 Hz); 7,13(d, 1H, J=2,0 Hz); 7,20(d, 1H, J=8,6 Hz); 7,51 (dd, 1H, J=8,7 , J'=2,0 Hz); 7,93(d, 1H, J=2,0 Hz); 8,00(d, 1H, J=8,8 Hz); 10,20(s, 1H). (DMSO-d6) |
| 33 | (CH₃)₂N- | CH₃ | H | H | H | H | H | 2 | 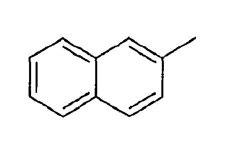 | 199-200 | | 2,11(s, 6H); 2,30(s, 3H); 2,35(t, 2H, J=7,0 Hz); 4,03(t, 2H, J=7,0 Hz); 6,03(s, 1H); 6,75(dd, 1H, J=8,6, J'=2,0 Hz); 7,10(d, 1H, J=2,0 Hz); 7,13(d, 1H, J=8,6 Hz); 7,54-7,67(m, 2H); 7,73(dd, 1H, J=8,6 , J'=1,8 Hz); 7,95(d, 1H, J=7,9 Hz); 8,02(d, 2H, J=8,6 Hz); 8,27(d, 1H, J=1,5 Hz); 9,89(s, 1H). (DMSO-d6) |
| 34 | (CH₃)₂N- | CH₃ | H | H | H | H | H | 2 | 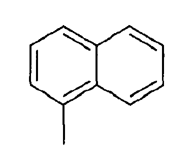 | 183-184 | | 2,12(s, 6H); 2,29(s, 3H); 2,35(t, 2H, J=7,0 Hz); 4,01 (t, 2H, J=7,0 Hz); 5,98(s, 1H); 6,62(dd, 1H, J=8,7 , J'=1,9 Hz); 6,98(d, 1H, J=2,0 Hz); 7,07(d, 1H, J=8,6 Hz); 7,49(m, 1H); 7,63(m, 1H); 7,70(m, 1H); 8,02(d, 2H, J=7,5 Hz); 8,12(d, 1H, J=8,0 Hz); 8,75(d, 1H, J=8,4 Hz); 10,15(s, 1H). (DMSO-d6) |
| 35 | (CH₃)₂N- | CH₃ | H | H | H | H | H | 2 | 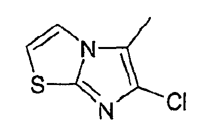 | 182-183 | | 2,14(s, 6H); 2,34(s, 3H); 2,39(m, 2H); 4,01(m, 2H); 6,09(s, 1H); 6,70(dd, 1H, J=8,5 J' 1,8 Hz); 7,08(d, 1H, J=1,8 Hz); 7,21 (d, 1H, J=8,5 Hz); 7,51 (d, 1H, J=4,5 Hz); 7,80(d, 1H, J=4,5 Hz). (DMSO-d6) |
| 36 | (CH₃)₂N- | CH₃ | H | H | H | H | H | 2 | 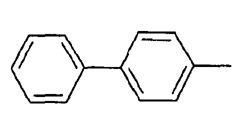 | 176-177 | | 2,14(s, 6H); 2,33(s, 3H); 2,39(t, 2H, J=7,1 Hz); 4,06(t, 2H, J=7,1 Hz); 6,07(s, 1H); 6,79(dd, 1H, J=8,8 , J'=2,0 Hz); 7,11 (d, 1H, J=1,8 Hz); 7,19(d, 1H, J=8,8 Hz); 7,36-7,48(m, 3H); 7,66(m, 2H); 7,72(AB sys, 2H, J=8,8 Hz); 7,79(AB sys, 2H, J=8,8 Hz); 9,85(s, 1H). (DMSO-d6) |
| 37 | (CH₃)₂N- | CH₃ | H | H | H | H | H | 2 | 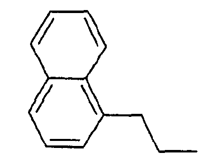 | 135-137 | | 2,17(s, 6H); 2,38(s, 3H); 2,45(m, 2H); 3,30-3,45(m, 4H); 4,14(t, 2H, J=6,7 Hz); 6,15(s, 1H); 7,04(d, 1H, J=8,5 Hz); 7,26(m, 1H); 7,30-7,38(m, 4H); 7,44(m, 1H); 7,65(d, 1H, J=8,2 Hz); 7,62(m, 1H); 7,87(d, 1H, J=8,2 Hz); 9,56(s, 1H). (DMSO-d6) |
| 38 | (CH₃)₂N- | CH₃ | H | H | H | H | H | 2 | 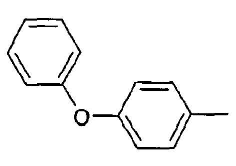 | 147-149 | | 2,20(s, 6H); 2,34(s, 3H); 2,45(m, 2H); 4,10(t, 2H, J=7,1 Hz); 6,08(s, 1 H); 6,76(dd, 1H, J=8,6 , J'=2,0 Hz); 6,99(d, 2H, J=8,8 Hz); 7,03-7,08(m, 3H); 7,17-7,24(m, 2H); 7,41(t, 2H, J=7,8 Hz); 7,63(d, 2H, J=8,8 Hz); 9,73(s, 1H). (DMSO-d6) |
| 39 | (CH₃)₂N- | CH₃ | H | H | H | H | H | 2 | 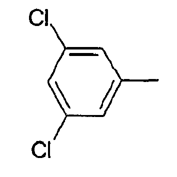 | 147-149 | | 2,15(s, 6H); 2,35(s, 3H); 2,41(t, 2H, J=6,7 Hz); 4,10(t, 2H, J=7,1 Hz); 6,12(s, 1H); 6,71(dd, 1H, J=8,6, J'=2,0 Hz); 7,09(d, 1H, J=1,8 Hz); 7,24(d, 1H, J=9,0 Hz); 7,58(d, 2H, J=1,9 Hz); 7,90(t, 1H, J=1,9 Hz). (DMSO-d6) |
| 40 | (CH₃)₂N- | CH₃ | H | H | H | H | H | 2 | 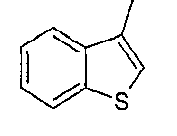 | 167-169 | | 2,14(s, 6H); 2,31(s, 3H); 2,38(m, 2H); 4,04(t, 2H, J=7,1 Hz); 6,02(s, 1 H); 6,66(dd, 1H, J=8,4, J'=1,8 Hz); 7,04(d, 1H, J=1,6 Hz); 7,12(d, 1H, J=8,2 Hz); 7,40-7,51(m, 2H); 8,03(d, 1H, J=7,6 Hz); 8,21(d, 1H, J=7,9 Hz); 8,31(s, 1H); 10,08(s, 1H). (DMSO-d6) |
| 41 | (CH₃CH₂)₂N- | H | H | H | H | H | H | 2 | 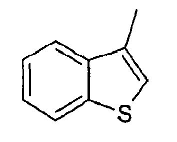 | 62-75 | | 0,98(m, 6H); 2,54(m, 4H); 2,70(m, 2H); 4,18(m, 2H); 6,29(s, 1H); 6,77(d, 1H, J=8,5 Hz); 7,18(s, 1H); 7,34(m, 2H); 7,39-7,52(m, 2H); 8,03(d, 1H, J=7,9 Hz); 8,22(d, 1H, J=7,5 Hz); 8,34(s, 1H); 10,18(s, 1H). (CDCl3) |
| 42 | (CH₃)₂N- | H | H | H | H | H | H | 2 | 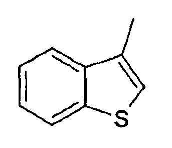 | 61-72 | | 2,12(s, 6H); 2,50(m, 2H); 4,12(t, 2H, J=6,7 Hz); 6,25(d, 1H, J=3,1 Hz); 6,75(dd, 1H, J=8,7, J'=2,1 Hz); 7,17(d, 1H, J=1,9 Hz); 7,25(d, 1H, J=8,9 Hz); 7,30(d, 1H, J=3,2 Hz); 7,48(m, 2H); 8,04(d, 1H, J=7,0 Hz); 8,24(d, 1H, J=7,4 Hz); 8,34(s, 1H); 10,14(s, 1H). (CDCl3) |
| 43 | 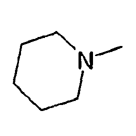 | H | H | H | H | H | H | 3 | 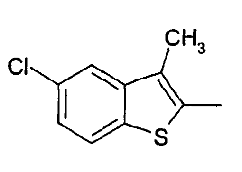 | 82-92 | | 1,20-1,55(m, 6H); 1,88(m, 2H); 2,33(s, 3H); 2,16-2,60(m, 6H); 4,10(t, 2H, J=6,6 Hz); 6,34(d, 1H, J=3,2 Hz); 6,82(d, 1H, J=9,9 Hz); 7,27-7,35(m, 3H); 7,50(dd, 1H, J=8,7, J'=2,0 Hz); 7,91 (d, 1H, J=2,2 Hz); 7,99(d, 1H, J=8,6 Hz); 10,20(bs, 1H). (DMSO-d6) |
| 44 | 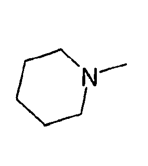 | H | H | H | H | H | H | 3 | 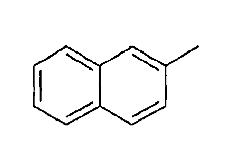 | 92-108 | | 1,20-1,55(m, 6H); 1,87(m, 2H); 2,22-2,62(m, 6H); 4,06(t, 2H, J=6,6 Hz); 6,28(d, 1H, J=2,9 Hz); 6,83(dd, 1H, J=8,7, J'=2,0 Hz); 7,24(d, 1H, J=2,0 Hz); 7,27 (m, 2H); 7,59(m, 1H); 7,64(m, 1H); 7,75(dd, 1H, J=8,8, J'=1,9 Hz); 7,95(d, 1H, J=7,5 Hz); 8,03( d, 2H, J=8,5 Hz); 8,28(s, 1H); 9,97(s, 1H). (DMSO-d6) |
| 45 | 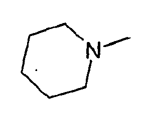 | H | H | H | H | H | H | 3 | 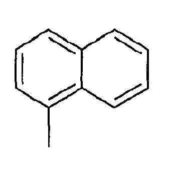 | 105-107 | | 1,25-1,55(m, 6H); 1,81(m, 2H); 2,03-2,60(m, 6H); 4,03(t, 2H, J=6,4 Hz); 6,23(d, 1H, J=3,1 Hz); 6,70(d, 1H, J=8,9 Hz); 7,11 (d, 1H, J=1,8 Hz); 7,20(d, 1H, J=8,9 Hz); 7,24(d, 1H, J=3,1 Hz); 7,49(dd, 1H, J=8,1, J'=7,4 Hz); 7,59-7,66(m, 1H); 7,66-7,73(m, 1H); 8-8,05(m, 2H); 8,12(d, 1H, J=8,2 Hz); 8,75(d, 1H, J=7,8 Hz); 10,20(bs, 1H). (DMSO-d6) |
| 46 | 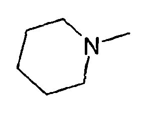 | H | H | H | H | H | H | 3 | 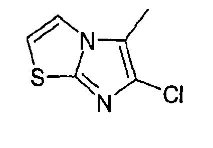 | 85-86 | | 1,36 (m, 2H); 1,49(m, 4H); 1,86(m, 2H); 2,15-2,44(m, 6H); 4,10(t, 2H, J=6,7 Hz); 6,33(d, 1H, J=3,1 Hz); 6,79(dd, 1H, J=8,7, J'=2,0 Hz); 7,21 (d, 1H, J=2,0 Hz); 7,30-7,36(m, 2H); 7,52(d, 1H, J=4,4 Hz); 7,83(d, 1 H, J=4,4 Hz); 10,25(bs, 1H). (DMSO-d6) |
| 47 | 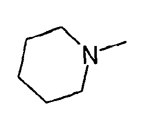 | H | H | H | H | H | H | 3 | 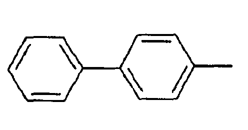 | 148-150 | | 1,34 (m, 2H); 1,47(m, 4H); 1,86(m, 2H); 2,03-2,55(m, 6H); 4,09(t, 2H, J=6,6 Hz); 6,32(d, 1H, J=2,8 Hz); 6,87(dd, 1H, J=8,9, J'=1,8 Hz); 7,26(d, 1H, J=1,9 Hz); 7,28-7,34(m, 2H); 7,36-7,49(m, 3H); 7,66(m, 2H); 7,73(AB sys, 2H, J=8,8 Hz); 7,79(AB sys, 2H, J=8,8 Hz); 9,91 (s, 1H). (DMSO-d6) |
| 48 | 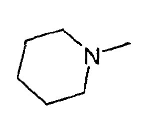 | H | H | H | H | H | H | 3 | 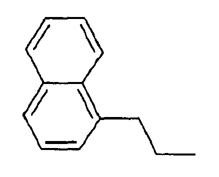 | 59-61 | | 1,20-1,56(m, 6H); 1,89(m, 2H); 2,12-2,50(m, 6H); 3,26-3,47(m, 4H); 4,16(t, 2H, J=6,2 Hz); 6,40(d, 1H, J=2,3 Hz); 7,13(d, 1H, J=8,6 Hz); 7,24(t, 1H, J=7,5 Hz); 7,34-7,50(m, 6H); 7,64(d, 1H, J=8,4 Hz); 7,76(m, 1H); 7,87(d, 1H, J=8,2 Hz); 9,65(s, 1 H). (DMSO-d6) |
| 49 | 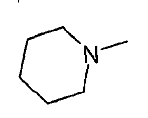 | H | H | H | H | H | H | 3 | 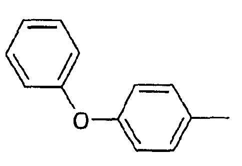 | 64-66 | | 1,20-1,58(m, 6H); 1,90(m, 2H); 2,20-2,55(m, 6H); 4,09(t, 2H, J=6,4 Hz); 6,33(s, 1H); 6,84(dd, 1H, J=8,4 Hz); 6,96-7,02(m, 2H); 7,04(d, 2H, J=7,9 Hz); 7,17-7,24(m, 2H); 7,32(m, 2H); 7,41 (m, 2H); 7,64(d, 2H, J=8,6 Hz); 9,79(s, 1H). (DMSO-d6) |
| 50 | 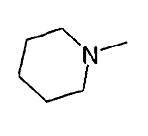 | H | H | H | H | H | H | 3 | 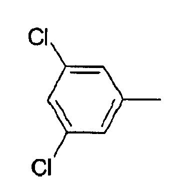 | 56-58 | | 1,35(m, 2H); 1,46(m, 4H); 1,83(m, 2H); 2,10(m, 2H); 2,24(m, 4H); 4,11(t, 2H, J=6,4 Hz); 6,36(d, 1H, J=2,6 Hz); 6,78(dd, 1H, J=8,8, J'=1,8 Hz); 7,22(d, 1H, J=1,90 Hz); 7,33(d, 1H, J=2,9 Hz); 7,37(d, 1H, J=8,8 Hz); 7,58(d, 2H, J=8,6 Hz); 7,89(t, 1H, J=1,9 Hz); 9,99(s, 1H). (DMSO-d6) |
| 51 | 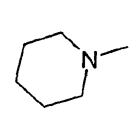 | H | H | H | H | H | H | 3 | 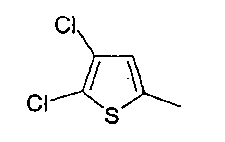 | 70-72 | | 1,38(m, 2H); 1,52(m, 4H); 1,91 (m, 2H); 2,24(m, 2H); 2,37(m, 4H); 4,16(t, 2H, J=6,6 Hz); 6,42(d, 1H, J=2,5 Hz); 6,89(d, 1H, J=8,6 Hz); 7,32(s, 1H); 7,37(d, 1H, J=2,8 Hz); 7,43(d, 1H, J=8,6 Hz); 7,48(s, 1H). (DMSO-d6) |
| 52 | 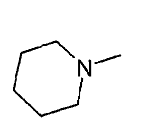 | H | H | H | H | H | H | 3 | 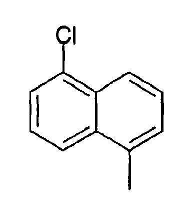 | 92-96 | | 1,13-1,70(m, 6H); 2,00(m, 2H); 2,68(m, 2H); 2,84(m, 2H); 3,26(m, 2H); 4,05(m, 2H); 6,22(d, 1H, J=3,1 Hz); 6,71(m, 1H); 7,10-7,18(m, 3H); 7,52-7,53(m, 2H); 7,72(m, 1H); 8,15(d, 1H, J=7,3 Hz); 8,37(d, 1H, J=8,5 Hz); 8,77(d, 1H, J=8,5 Hz); 8,97(bs, 1H); 10,23(bs, 1H). (DMSO-d6 + TFA) |

### Pharmaceutical particulars:

Binding of the new compounds of general formula (Ia) and (Ib) **and (Ic)** to the 5-HT₆ receptor was determined as previously described.

The binding results for some of the compounds of the present invention are indicated in the following table:

**Table**

| **Example** | **Kᵢ (nM)** |
|---|---|
| 3 | 94,2 |
| 4 | 112,4 |
| 11 | 1,89 |
| 12 | 104,6 |
| 13 | 82,5 |
| 20 | 84,8 |

The daily posology in human medicine is comprised between 1 milligram and 2 grams of medicinal product which may be administered in one or several doses. The compositions are prepared under forms that are compatible with the administration route used, preferably tablets, coated tablets, capsules, suppositories, solutions or suspensions. These compositions are prepared by means of known methods and comprise from 1 to 60% by weight of the active substance (compound of general formula I), and 40 to 99% by weight of the suitable pharmaceutical vehicle compatible with the active substance and the physical form of the composition used.

The formula of a tablet containing a product of the invention is provided by way of example:
Example of formula per tablet:

| | |
|---|---|
| Example 1 | 5 mg |
| Lactose | 60 mg |
| Crystalline cellulose | 25 mg |
| Povidone K 90 | 5 mg |
| Pregelatinized starch | 3 mg |
| Colloidal silicon dioxide | 1 mg |
| Magnesium stearate | 1 mg |
| Total weight per tablet | 100 mg |

## Claims

1. A sulfonamide compound of general formula (Ia) 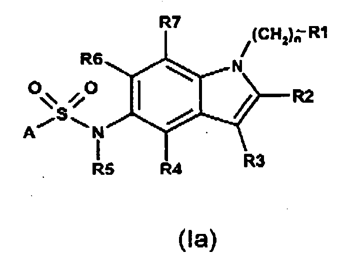 wherein
R¹ represents an -NR⁸R⁹ radical or a saturated or unsaturated, optionally at least mono-substituted, cycloaliphatic radical, which may optionally contain at least one heteroatom as a ring member and/or which may be condensed with a saturated or unsaturated, optionally at least mono-substituted mono- or bicyclic cycloaliphatic ring system, which may optionally contain at least one heteroatom as a ring member,
R², R³, R⁴, R⁶ and R⁷, identical or different, each represent hydrogen, halogen, nitro, alkoxy, cyano, a saturated or unsaturated, linear or branched, optionally at least mono-substituted aliphatic radical or an optionally at least mono-substituted phenyl radical or an optionally at least mono-substituted heteroaryl radical,
R⁵ represents hydrogen or a saturated or unsaturated, linear or branched, optionally at least mono-substituted aliphatic radical,
R⁸ and R⁹, identical or different, each represent hydrogen or a saturated or unsaturated, linear or branched, optionally at least mono-substituted aliphatic radical,
with the proviso that R⁸ and R⁹ are not hydrogen at the same time, and if one of them, R⁸ and R⁹, represents a saturated or unsaturated, linear or branched, optionally at least mono-substituted C₁-C₄ aliphatic radical, the other one represents a saturated or unsaturated, linear or branched, optionally at least mono-substituted aliphatic radical with at least five carbon atoms, or
R⁸ and R⁹ together with the bridging nitrogen atom form a saturated or unsaturated, optionally at least mono-substituted heterocyclic ring, which may contain at least one additional heteroatom as a ring member and/or which may be condensed with a saturated or unsaturated, optionally at least mono-substituted, mono- or bicyclic cycloaliphatic ring system which may optionally contain at least one heteroatom as a ring member
A represents an optionally at least mono-substituted mono- or polycyclic aromatic ring system, which may be bonded via an optionally at least mono-substituted alkylene, alkenylene or alkynylene group and/or which may contain at least one heteroatom as a ring member in one or more of its rings,
and
n is 0, 1, 2, 3 or 4;
optionally in form of one of its stereoisomers, preferably enantiomers or diastereomers, its racemate or in form of a mixture of at least two of its stereoisomers, preferably enantiomers or diastereomers, in any mixing ratio, or a salt thereof, preferably a corresponding, physiologically acceptable salt thereof, or a corresponding solvate thereof.

2. A compound according to claim 1, **characterized in that** R¹ represents an -NR⁸R⁹ radical or a saturated or unsaturated, optionally at least mono-substituted 5- or 6-membered cycloaliphatic radical which may optionally contain at least one heteroatom as a ring member and/or which may be condensed with a saturated or unsaturated, optionally at least mono-substituted mono- or bicyclic cycloaliphatic ring system, which may optionally contain at least one heteroatom as a ring member, whereby the rings of the ring system are 5- or 6-membered.

3. A compound according to claim 1 or 2, **characterized in that** R², R³, R⁴, R⁶ and R⁷, identical or different, each represent hydrogen, a linear or branched, optionally at least mono-substituted C₁-C₆ alkyl radical, a linear or branched, optionally at least mono-substituted C₂-C₆ alkenyl radical or a linear or branched, optionally at least mono-substituted C₂-C₆ alkynyl radical.

4. A compound according to one or more of claims 1 to 3, **characterized in that** R⁵ represents hydrogen, a linear or branched, optionally at least mono-substituted C₁-C₆ alkyl radical, a linear or branched, optionally at least mono-substituted C₂-C₆ alkenyl radical, or a linear or branched, optionally at least mono-substituted C₂-C₆ alkynyl radical.

5. A compound according to one or more of claims 1 to 4, **characterized in that** R⁸ and R⁹, identical or different, each represent hydrogen, a linear or branched, optionally at least mono-substituted C₁-C₁₀ alkyl radical, a linear or branched, optionally at least mono-substituted C₂-C₁₀ alkenyl radical, a linear or branched, optionally at least mono-substituted C₂-C₁₀ alkynyl radical,
or
R⁸ and R⁹ together with the bridging nitrogen atom form a saturated or unsaturated, optionally at least mono-substituted 5- or 6-membered heterocyclic ring which may contain at least one additional heteroatom as a ring member and/or which may be condensed with a saturated or unsaturated, optionally at least mono-substituted mono- or bicyclic cycloaliphatic ring system, which may optionally contain at least one heteroatom as a ring member, whereby the rings of the ring system are 5- 6- or 7-membered.

6. A compound according to claim 5, **characterized in that** R⁸ and R⁹, identical or different, each represent hydrogen or a linear or branched C₁-C₁₀ alkyl radical, or
R⁸ and R⁹ together with the bridging nitrogen atom form a radical chosen from the group consisting of 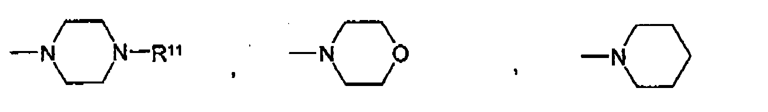 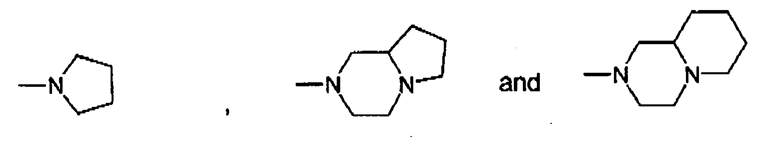 wherein R¹¹, if present, represents hydrogen, a linear or branched C₁-C₆ alkyl radical or a benzyl radical.

7. A compound according to one or more of claims 1 to 6, **characterized in that** A represents an optionally at least mono-substituted mono- or polycyclic aromatic ring system, wherein the ring(s) is/are 5- or 6- membered, which may be bonded via an optionally at least mono-substituted C₁-C₆ alkylene group, an optionally at least mono-substituted C₂-C₆ alkenylene group or an optionally at least mono-substituted C₂-C₆ alkynylene group and/or wherein the ring(s) may contain at least one heteroatom as a ring member.

8. A compound according to one or more of claims 1 to 7 chosen from the group consisting of
[16] N-[1-(2-pyrrolidine-1-yl-ethyl)-1H-indole-5-yl]-naphthalene-2-sulfonamide,
[17] N-[1-(2-pyrrolidine-1-yl-ethyl)-1H-indole-5-yl]-naphthalene-1-sulfonamide,
[18] N-[1-(2-pyrrolidine-1-yl-ethyl)-1H-indole-5-yl]-5-chloro-3-methylbenzo[b]thiophene-2-sulfonamide,
[28] N-[1-(2-pyrrolidine-1-yl-ethyl)-1H-indole-5-yl]-]-6-chloroimidazo[2,1-b]thiazole-5-sulfonamide,
[43] 5-chloro-3-methyl-N-(1-(3-(piperidin-1-yl)propyl)-1H-indol-5-yl)benzo[b]thiophene-2-sulfonamide,
[44] N-(1-(3-(piperidin-1-yl)propyl)-1H-indol-5-yl)naphthalene-2-sulfonamide,
[45] N-(1-(3-(piperidin-1-yl)propyl)-1H-indol-5-yl)naphthalene-1-sulfonamide,
[46] 6-chloro-N-(1-(3-piperidin-1-yl)propyl)-1H-indol-5-yl)imidazo[2,1-b]thiazole-5-sulfonamide,
[47] 4-phenyl-N-(1-(3-(piperidin-1-yl)propyl)-1H-indol-5-yl)benzenesulfonamide,
[48] 2-(naphth-1-yl)-N-(1-(3-(piperidin-1-yl)propyl)-1H-indol-5-yl)ethanesulfonamide,
[49] 4-phenoxy-N-(1-(3-(piperidin-1-yl)propyl)-1H-indol-5-yl)benzenesulfonamide,
[50] 3,5-dichloro-N-(1-(3-(piperidin-1-yl)propyl)-1H-indol-5-yl)benzenesulfonylamide,
[51] 4,5-dichloro-N-(1-(3-(piperidin-1-yl)propyl)-1H-indol-5-yl)thiophene-2-sulfonamide and
[52] 5-chloro-N-(1-(3-(piperidin-1-yl)propyl)-1H-indol-5-yl)naphthalene-1-sulfonamide,
optionally in form of their corresponding salts or their corresponding solvates.

9. A sulfonamide compound of general formula (Ib) 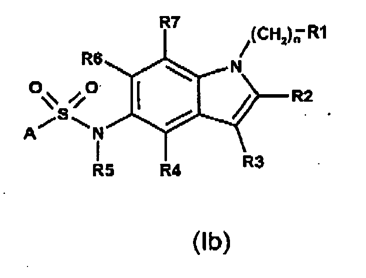 wherein
R¹ represents a -NR⁸R⁹ radical,
R², R³, R⁴, R⁶ and R⁷, identical or different, each represent hydrogen, halogen, nitro, alkoxy, cyano, a saturated or unsaturated, optionally at least mono-substituted, linear or branched aliphatic radical, or an optionally at least mono-substituted phenyl or an optionally at least mono-substituted heteroaryl radical,
R⁵ represents hydrogen or a saturated or unsaturated, linear or branched, optionally at least mono-substituted aliphatic radical,
R⁸ and R⁹, identical or different, each represent hydrogen or a saturated or unsaturated, linear or branched, optionally at least mono-substituted, C₁-C₄ aliphatic radical,
A represents an optionally at least mono-substituted mono- or polycyclic aromatic ring system, which may be bonded via an optionally at least mono-substituted alkylene, alkenylene or alkynylene group and/or which may contain at least one heteroatom as a ring member in one or more of its rings,
and n is 0, 1, 2, 3 or 4;
optionally in form of one of its stereoisomers, preferably enantiomers or diastereomers, its racemate or in form of a mixture of at least two of its stereoisomers, preferably enantiomers or diastereomers, in any mixing ratio, or a salt therof, preferably a corresponding, physiologically acceptable salt thereof, or a corresponding solvate thereof.

10. A compound according to claim 9, **characterized in that** R², R³, R⁴, R⁶ and R⁷, identical or different, each represent hydrogen, a linear or branched, optionally at least mono-substituted C₁-C₆ alkyl radical, a linear or branched, optionally at least mono-substituted C₂-C₆ alkenyl radical, or a linear or branched, optionally at least mono-substituted C₂-C₆ alkynyl radical.

11. A compound according to claim 9 or 10, **characterized in that** R⁵ represents hydrogen, a linear or branched, optionally at least mono-substituted C₁-C₆ alkyl radical, a linear or branched, optionally at least mono-substituted C₂-C₆ alkenyl radical or a linear or branched, optionally at least mono-substituted C₂-C₆ alkynyl radical.

12. A compound according to one or more of claims 9 to 11, **characterized in that** R⁸ and R⁹, identical or different, each represent hydrogen or a linear or branched, optionally at least mono-substituted C₁-C₄ alkyl radical, with the proviso that R⁸ and R⁹ are not hydrogen at the same time.

13. A compound according to one or more of claims 9 to 12, **characterized in that** A represents an optionally at least mono-substituted mono- or polycyclic aromatic ring system, wherein the ring(s) is/are 5- or 6-membered, which may be bonded via an optionally at least mono-substituted C₁-C₆ alkylene group, an optionally at least mono-substituted C₂-C₆ alkenylene group or an optionally at least mono-substituted C₂-C₆ alkynylene group and/or wherein the ring(s) may contain at least one heteroatom as a ring member.

14. A compound according to one or more of claims 9 to 13 selected from the group consisting of
[1] N-[1-(2-dimethylaminoethyl)-1H-indole-5-yl]-5-chloro-3-methylbenzo[b]thiophene-2-sulfonamide,
[2] N-[1-(2-dimethylaminoethyl)-1H-indole-5-yl]-naphthalene-2-sulfonamide,
[3] N-[1-(2-dimethylaminoethyl)-1H-indole-5-yl]-naphthalene-1-sulfonamide,
[4] N-[1-(2-dimethylaminoethyl)-1H-indole-5-yl]-5-chloronaphthalene-1-sulfonamide,
[5] N-[1-(2-dimethylaminoethyl)-1H-indole-5-yl]-benzenesulfonamide,
[6] N-[1-(2-dimethylaminoethyl)-1H-indole-5-yl]-quinoline-8-sulfonamide,
[7] N-[1-(2-dimethylaminoethyl)-1H-indole-5-yl]-4-phenoxybenzenesulfonamide,
[8] N-[1-(2-dimethylaminoethyl)-1H-indole-5-yl]-4-methylbenzenesulfonamide,
[9] N-[1-(2-dimethylaminoethyl)-1H-indole-5-yl]-5-chlorothiophene-2-sulfonamide,
[10] N-[1-(2-dimethylaminoethyl)-1H-indole-5-yl]-benzo[1,2,5]thiadiazole-4-sulfonamide,
[11] N-[1-(2-dimethylaminoethyl)-1H-indole-5-yl]-6-chloroimidazo[2,1-blthiazole-5-sulfonamide,
[12] N-[1-(2-dimethylaminoethyl)-1H-indole-5-yl]-3,5-dichlorobenzenesulfonamide,
[13] N-[1-(2-dimethylaminoethyl)-1H-indole-5-yl]-3-bromobenzenesulfonamide,
[14] N-[1-(2-dimethylaminoethyl)-1H-indole-5-yl]-3-nitrobenzenesulfonamide,
[15] N-[1-(2-dimethylaminoethyl)-1H-indole-5-yl]-1-phenylmethanesulfonamide,
[19] trans-N-[1-(2-dimethylaminoethyl)-1H-indole-5-yl]-2-phenylethenesulfonamide,
[20] N-[1-(2-dimethylaminoethyl)-1H-indole-5-yl]-4,5-dichlorothiophene-2-sulfonamide,
[21] N-[1-(2-dimethylaminoethyl)-1H-indole-5-yl]-4-acetylbenzenesulfonamide,
[22] N-[1-(2-dimethylaminoethyl)-1H-indole-5-yl]-4-bromobenzenesulfonamide,
[23] N-[1-(2-dimethylaminoethyl)-1H-indole-5-yl]-4-methoxybenzenesulfonamide,
[24] N-[3-(2-diethylaminoethyl)-1H-indole-5-yl]-5-chloro-3-methylbenzo[b]thiophene-2-sulfonamide,
[25] N-[1-(2-dimethylaminoethyl)-1H-indole-5-yl]-4-nitrobenzenesulfonamide,
[26] N-[1-(2-dimethylaminoethyl)-1H-indole-5-yl]-4-fluorobenzenesulfonamide,
[27] N-[1-(2-diethylaminoethyl)-1H-indole-5-yl]-6-chloroimidazo[2,1-b]thiazole-5-sulfonamide,
[29] N-(1-(2-(diethylamino)ethyl)-1H-indol-5-yl)-naphthalene-2-sulfonamide,
[30] N-(1-(2-(diethylamino)ethyl)-1H-indol-5-yl)-naphthalene-1-sulfonamide,
[31] N-(1-(2-(diethylamino)ethyl-1H-indol-5-yl)-4-phenylbenzenesulfonamide,
[32] 5-chloro-N-(1-(2-(dimethylamino)ethyl)-2-methyl-1H-indol-5-yl)-3-methylbenzo[b]thiophene-2-sulfonamide,
[33] N-(1-(2-(dimethylamino)ethyl)-2-methyl-1H-indol-5-yl)-naphthalene-2-sulfonamide,
[34] N-(1-(2-(dimethylamino)ethyl)-2-methyl-1H-indol-5-yl)-naphthalene-1-sulfonamide,
[35] 6-chloro-N-(1-(2-(dimethylamino)ethyl)-2-methyl-1H-indol-5-yl)imidazo[2,1-b]thiazole-5-sulfonamide,
[36] N-(1-(2-(dimethylamino)ethyl)-2-methyl-1H-indol-5-yl)-4-phenylbenzenesulfonamide,
[37] N-(1-(2-dimethylamino)ethyl)-2-methyl-1H-indol-5-yl)-2-(naphth-1-yl)-ethanesulfonamide,
[38] N-(1-(2-(dimethylamino)ethyl)-2-methyl-1H-indol-5-yl)-4-phenoxybenzenesulfonamide,
[39] 3,5-dichloro-N-(1-(2-(dimethylamino)ethyl)-2-methyl-1H-indol-5-yl)-benzenesulfonamide,
[40] N-(1-(2-(dimethylamino)ethyl)-2-methyl-1H-indol-5-yl)benzo[b]thiophene-3-sulfonamide,
[41] N-(1-(2-(diethylamino)ethyl)-1H-indol-5-yl)benzo[b]thiophene-3-sulfonamide and
[42] N-(1-(2-(dimethylamino)ethyl)-1H-indol-5-yl)benzo[b]thiophene-3-sulfonamide,
optionally in form of their corresponding salts and their corresponding solvates.

15. A process for obtaining a sulfonamide derivative of general formula (Ia) and/or (Ib), according to one or more of claims 1 - 14, **characterized in that** at least one compound of general formula (II), or one of its suitably protected derivatives, 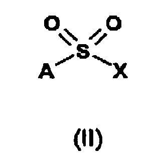 wherein A has the meaning according to one or more of claims 1 - 14, and X is an acceptable leaving group, is reacted with at least one 5-aminoindole of general formula (III), or one of its suitably protected derivatives; 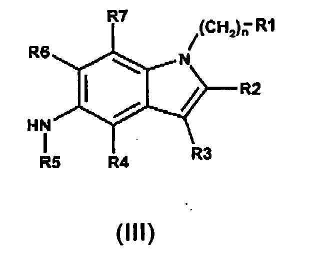 wherein R¹-R⁷ and n have the meaning according to one or more of claims 1 - 14 to obtain the corresponding sulfonamide and optionally, from the latter, the protective groups may be removed if necessary.

16. A process for obtaining a sulfonamide derivative of general formula (Ia) and/or (Ib) according to one or more of claims 1 - 14, wherein R¹-R⁴, R⁶-R⁷, n and A have the meaning according to one or more of claims 1 - 14, and R⁶ represents C₁-C₆ alkyl, **characterized in that** at least one compound of general formula (Ia) and/or at least one compound of general formula (Ib), wherein R¹-R⁴, R⁶-R⁷, n and A have the meaning according to one or more of claims 1 - 14, and R⁵ represents an hydrogen atom, is reacted with an alkyl halogenide or dialkyl sulfate.

17. A process for preparing the salts of the compounds of general formula (Ia) and/or (Ib), according to one or more of claims 1 - 14, consisting in reacting at least one compound of the general formula (Ia) and/or at least one compound of the general formula (Ib) with a mineral acid or an organic acid in a suitable solvent.

18. A medicament comprising at least one compound according to one or more of claims 1 to 8 and optionally at least one or more of pharmacologically acceptable excipients.

19. A medicament according to claim 18, for 5-HT₆ receptor regulation, for the prophylaxis and/or treatment of a disorder or disease related to food intake, for the prophylaxis and/or treatment of obesity, bulimia, anorexia, cachexia or type II diabetes (non insulin dependent diabetes mellitus), for the prophylaxis and/or treatment of gastrointestinal tract disorders, for cognitive enhancement, for the prophylaxis and/or treatment of disorders of the central nervous system, anxiety, panic disorders, depression, bipolar disorders, cognitive memory disorders, senile dementia processes, neurodegenerative disorders, schizophrenia, psychosis or infantile hyperkinesia (ADHD, attention deficit / hyperactivity disorder).

20. A medicament according to claim 19, wherein the disorder or disease related to food intake is selected from the group consisting of regulation of appetite and maintenance, increase or reduction of body weight.

21. A medicament according to claim 19, wherein the type II diabetes is type II diabetes caused by obesity,

22. A medicament according to claim 19, wherein the gastrointestinal tract disorder is irritable bowel syndrome.

23. A medicament according to claim 19, wherein the neurodegenerative disorder is selected from the group consisting of Alzheimer's disease, Parkinson's disease, Huntington's disease and/or multiple sclerosis.

24. The use of at least one compound according to one or more of claims 1 to 8 for the manufacture of a medicament for 5-HT₆ receptor regulation.

25. The use of at least one compound according to one or more of claims 1 to 8 for the manufacture of a medicament for the prophylaxis and/or treatment of a disorder or disease related to food intake.

26. The use of at least one compound according to one or more of claims 1 to 8 for the manufacture of a medicament for the regulation of appetite.

27. The use of at least one compound according to one or more of claims 1 to 8 for the manufacture of a medicament for the maintenance, increase or reduction of body weight.

28. The use of at least one compound according to one or more of claims 1 to 8 for the manufacture of a medicament for the prophylaxis and/or treatment of obesity.

29. The use of at least one compound according to one or more of claims 1 to 8 for the manufacture of a medicament for prophylaxis and/or treatment of bulimia.

30. The use of at least one compound according to one or more of claims 1 to 8 for the manufacture of a medicament for the prophylaxis and/or treatment of anorexia.

31. The use of at least one compound according to one or more of claims 1 to 8 for the manufacture of a medicament for the prophylaxis and/or treatment of cachexia.

32. The use of at least one compound according to one or more of claims 1 to 8 for the manufacture of a medicament for the prophylaxis and/or treatment of type II diabetes (non insulin dependent diabetes mellitus).

33. The use according to claim 32, wherein the type II diabetes is type II diabetes caused by obesity.

34. The use of at least one compound according to one or more of claims 1 to 8 for the manufacture of a medicament for the prophylaxis and/or treatment of gastrointestinal tract disorders.

35. The use of at least one compound according to one or more of claims 1 to 8 for the manufacture of a medicament for the prophylaxis and/or treatment of irritable bowel syndrome.

36. The use of at least one compound according to one or more of claims 1 to 8 for the manufacture of a medicament for the prophylaxis and/or treatment of anxiety.

37. The use of at least one compound according to one or more of claims 1 to 8 for the manufacture of a medicament for the prophylaxis and/or treatment of depression.

38. The use of at least one compound according to one more of claims 1 to 8 for the manufacture of a medicament for the prophylaxis and/or treatment of bipolar disorders.

39. The use of at least one compound according to one or more of claims 1 to 8 for the manufacture of a medicament for the prophylaxis and/or treatment of cognitive memory disorders.

40. The use of at least one compound according to one or more of claims 1 to 8 for the manufacture of a medicament for the prophylaxis and/or treatment of senile dementia processes.

41. The use of at least one compound according to one or more of claims 1 to 8 for the manufacture of a medicament for the prophylaxis and/or treatment of Alzheimer's Disease.

42. The use of at least one compound according to one or more of claims 1 to 8 for the manufacture of a medicament for the prophylaxis and/or treatment of Parkinson's Disease.

43. The use of at least one compound according to one or more of claims 1 to 8 for the manufacture of a medicament for the prophylaxis and/or treatment of Huntington's Disease.

44. The use of at least one compound according to one or more of claims 1 to 8 for the manufacture of a medicament for the prophylaxis and/or treatment of dementias in which a cognitive deficit predominates.

45. The use of at least one compound according to one or more of claims 1 to 8 for the manufacture of a medicament for the prophylaxis and/or treatment of Multiple Sclerosis.

46. The use of at least one compound according to one or more of claims 1 to 8 for the manufacture of a medicament for the prophylaxis and/or treatment of psychosis.

47. The use of at least one compound according to one or more of claims 1 to 8 for the manufacture of a medicament for the prophylaxis and/or treatment of infantile hyperkinesia (ADHD, attention deficit / hyperactivity disorder).

48. The use of at least one compound according to one or more of claims 1 to 8 for the manufacture of a medicament for the prophylaxis and/or treatment of disorders of the central nervous system.

49. The use of at least one compound according to one or more of claims 1 to 8 for the manufacture of a medicament for the prophylaxis and/or treatment of schizophrenia.

50. The use of at least one compound according to one or more of claims 1 to 8 for the manufacture of a medicament for cognitive enhancement.

51. A medicament comprising at least one compound according to one or more of claims 9 to 14 and optionally at least one or more of pharmacologically acceptable excipients.

52. A medicament according to claim 51 for 5-HT₆ receptor regulation, for the prophylaxis and/or treatment of a disorder or disease related to food intake, for the prophylaxis and/or treatment of obesity, bulimia, anorexia, cachexia or type II diabetes (non insulin dependent diabetes mellitus), for the prophylaxis and/or treatment of gastrointestinal tract disorders, for cognitive enhancement, for the prophylaxis and/or treatment of disorders of the central nervous system, anxiety, panic disorders, depression, bipolar disorders, cognitive memory disorders, senile dementia processes, neurodegenerative disorders, schizophrenia, psychosis or infantile hyperkinesia (ADHD, attention deficit / hyperactivity disorder).

53. A medicament according to claim 52, wherein the disorder or disease related to food intake is selected from the group consisting of regulation of appetite and maintenance, increase or reduction of body weight.

54. A medicament according to claim 52, wherein the type II diabetes is type II diabetes caused by obesity,

55. A medicament according to claim 52, wherein the gastrointestinal tract disorder is irritable bowel syndrome.

56. A medicament according to claim 52, wherein the neurodegenerative disorder is selected from the group consisting of Alzheimer's disease, Parkinson's disease, Huntington's disease and/or multiple sclerosis.

57. The use of at least one compound according to one or more of claims 9 to 14 for the manufacture of a medicament for 5-HT₆ receptor regulation.

58. The use of at least one compound according to one or more of claims 9 to 14 for the manufacture of a medicament for the prophylaxis and/or treatment of a disorder or disease related to food intake.

59. The use of at least one compound according to one or more of claims 9 to 14 for the manufacture of a medicament for the regulation of appetite.

60. The use of at least one compound according to one or more of claims 9 to 14 for the manufacture of a medicament for the maintenance, increase or reduction of body weight.

61. The use of at least one compound according to one or more of claims 9 to 14 for the manufacture of a medicament for the prophylaxis and/or treatment of obesity.

62. The use of at least one compound according to one or more of claims 9 to 14 for the manufacture of a medicament for the prophylaxis and/or treatment of bulimia.

63. The use of at least one compound according to one or more of claims 9 to 14 for the manufacture of a medicament for the prophylaxis and/or treatment of anorexia.

64. The use of at least one compound according to one or more of claims 9 to 14 for the manufacture of a medicament for the prophylaxis and/or treatment of cachexia.

65. The use of at least one compound according to one or more of claims 9 to 14 for the manufacture of a medicament for the prophylaxis and/or treatment of type II diabetes (non-insulin-dependent diabetes mellitus).

66. The use according to claim 65, wherein the type II diabetes is type II diabetes caused by obesity.

67. The use of at least one compound according to one or more of claims 9 to 14 for the manufacture of a medicament for the prophylaxis and/or treatment of gastrointestinal tract disorders.

68. The use of at least one compound according to one or more of claims 9 to 14 for the manufacture of a medicament for the prophylaxis and/or treatment of irritable bowel syndrome.

69. The use of at least one compound according to one or more of claims 9 to 14 for the manufacture of a medicament for the prophylaxis and/or treatment of anxiety.

70. The use of at least one compound according to one or more of claims 9 to 14 for the manufacture of a medicament for the prophylaxis and/or treatment of depression.

71. The use of at least one compound according to one or more of claims 9 to 14 for the manufacture of a medicament for the prophylaxis and/or treatment of bipolar disorders.

72. The use of at least one compound according to one or more of claims 9 to 14 for the manufacture of a medicament for the prophylaxis and/or treatment of cognitive memory disorders.

73. The use of at least one compound according to one or more of claims 9 to 14 for the manufacture of a medicament for the prophylaxis and/or treatment of senile dementia processes.

74. The use of at least one compound according to one or more of claims 9 to 14 for the manufacture of a medicament for the prophylaxis and/or treatment of Alzheimer's Disease.

75. The use of at least one compound according to one or more of claims 9 to 14 for the manufacture of a medicament for the prophylaxis and/or treatment of Parkinson's Disease.

76. The use of at least one compound according to one or more of claims 9 to 14 for the manufacture of a medicament for the prophylaxis and/or treatment of Huntington's Disease.

77. The use of at least one compound according to one or more of claims 9 to 14 for the manufacture of a medicament for the prophylaxis and/or treatment of Multiple Sclerosis.

78. The use of at least one compound according to one or more of claims 9 to 14 for the manufacture of a medicament for the prophylaxis and/or treatment of dementias in which a cognitive deficit predominates.

79. The use of at least one compound according to one or more of claims 9 to 14 for the manufacture of a medicament for the prophylaxis and/or treatment of psychosis.

80. The use of at least one compound according to one or more of claims 9 to 14 for the manufacture of a medicament for the prophylaxis and/or treatment of infantile hyperkinesia (ADHD, attention deficit / hyperactivity disorder).

81. The use of at least one compound according to one or more of claims 9 to 14 for the manufacture of a medicament for the prophylaxis and/or treatment of disorders of the central nervous system.

82. The use of at least one compound according to one or more of claims 9 to 14 for the manufacture of a medicament for the prophylaxis and/or treatment of schizophrenia.

83. The use of at least one compound according to one or more of claims 9 to 14 for the manufacture of a medicament for cognitive enhancement.

## Patentansprüche

1. Sulfonamidverbindung der allgemeinen Formel (Ia): 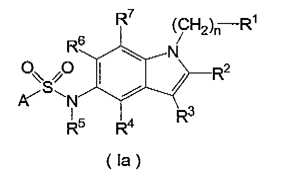 worin
R¹ einen -NR⁸R⁹-Rest, der einen gesättigten oder ungesättigten, optional mindestens mono-substituierten cycloaliphatischen Rest, der optional mindestens ein Heteroatom als ein Ringglied enthalten kann und/oder der mit einem gesättigten oder ungesättigten, optional mindestens mono-substituierten, mono- oder bicyclischen cycloaliphatischen Ringsystem, das optional mindestens ein Heteroatom als Ringglied enthalten kann, kondensiert sein kann, bezeichnet,
R², R³, R⁴, R⁶ und R⁷, gleich oder verschieden, jeweils Wasserstoff, Halogen, Nitro, Alkoxy, Cyano, einen gesättigten oder ungesättigten, linearen oder verzweigten, optional mindestens mono-substituierten aliphatischen Rest oder einen optional mindestens mono-substituierten phenylrest oder einen optional mindestens mono-substituierten Heteroarylrest bezeichnen,
R⁵ Wasserstoff oder einen gesättigten oder ungesättigten, linearen oder verzweigten, optional mindestens mono-substituierten aliphatischen Rest bezeichnet,
R⁸ und R⁹, gleich oder verschieden, jeweils Wasserstoff oder einen gesättigten oder ungesättigten, linearen oder verzweigten, optional mindestens mono-substituierten aliphatischen Rest bezeichnen,
mit der Massgabe, dass R⁸ und R⁹ nicht gleichzeitig Wasserstoff sind und wenn eines von ihnen, R⁸ und R⁹, einen gesättigten oder ungesättigten, linearen oder verzweigten, optional mindestens mono-substituierten, aliphatischen C₁₋₄-Rest bezeichnet, der andere einen gesättigten oder ungesättigten, linearen oder verzweigten, optional mindestens mono-substituierten aliphatischen Rest mit mindestens 5 Kohlenstoffatomen bezeichnet, oder
R⁸ und R⁹ zusammen mit dem Brücken-Stickstoffatom einen gesättigten oder ungesättigten, optional mindestens mono-substituierten heterocyclischen Ring bilden, der mindestens ein zusätzliches Heteroatom als ein Ringglied enthalten kann und/oder der mit einem gesättigten oder ungesättigen, optional mindestens mono-substituierten mono- oder bicyclischen cycloaliphatischen Ringsystem, das optional mindestens ein Heteroatom als ein Ringglied enthalten kann, kondensiert sein kann,
A ein optional mindestens mono-substituiertes mono- oder polycyclisches aromatisches Ringsystem bezeichnet, das über eine optional mindestens mono-substituierte Alkylen-, Alkenylen- oder Alkinylengruppe gebunden sein kann und/oder das mindestens ein Heteroatom als ein Ringglied in einem oder mehreren seiner Ringe enthalten kann,
und
n 0, 1, 2, 3 oder 4 ist;
optional in Form eines ihrer Stereoisomere, vorzugsweise Enantiomere oder Diastereomere, ihres Racemats oder in Form einer Mischung von mindestens zwei ihrer Stereoisomere, vorzugsweise Enantiomere oder Diastereomere, in beliebigem Mischungsverhältnis, oder ein Salz davon, vorzugsweise ein korrespondierendes, physiologisch akzeptables Salz davon oder ein korrespondierendes Solvat davon.

2. Verbindung gemäss Anspruch 1, **dadurch gekennzeichnet, dass** R¹ einen -NR⁸R⁹-Rest oder einen gesättigten oder ungesättigten, optional mindestens mono-substituierten 5- oder 6-gliedrigen cycloaliphatischen Rest bezeichnet, der optional mindestens ein Heteroatom als ein Ringglied enthalten kann und/oder der mit einem gesättigten oder ungesättigten, optional mindestens mono-substituierten mono- oder bicyclischen cycloaliphatischen Ringsystem, das optional mindestens ein Heteroatom als ein Ringglied enthalten kann, kondensiert sein kann, wobei die Ringe des Ringsystems 5- oder 6-gliedrig sind.

3. Verbindung gemäss Anspruch 1 oder 2, **dadurch gekennzeichnet, dass** R², R³, R⁴, R⁶ und R⁷, gleich oder verschieden, jeweils Wasserstoff, einen linearen oder verzweigten, optional mindestens mono-substituierten C₁₋₆-Alkylrest, einen linearen oder verzweigten, optional mindestens mono-substituierten C₂₋₆-Alkenylrest oder einen linearen oder verzweigten, optional mindestens mono-substituierten C₂₋₆-Alkinylrest bezeichnen.

4. Verbindung gemäss einem oder mehreren der Ansprüche 1 bis 3, **dadurch gekennzeichnet, dass** R⁵ Wasserstoff, einen linearen oder verzweigten, optional mindestens mono-substituierten C₁₋₆-Alkylrest, einen linearen oder verzweigten, optional mindestens mono-substituierten C₂₋₆-Alkenylrest oder einen linearen oder verzweigten, optional mindestens mono-substituierten C₂₋₆-Alkinylrest bezeichnet.

5. Verbindung gemäss einem oder mehreren der Ansprüche 1 bis 4, **dadurch gekennzeichnet, dass** R⁸ und R⁹, gleich oder verschieden, jeweils Wasserstoff, einen linearen oder verzweigten, optional mindestens mono-substituierten C₁₋₁₀-Alkylrest, einen linearen oder verzweigten, optional mindestens mono-substituierten C₂₋₁₀-Alkenylrest, einen linearen oder verzweigten, optional mindestens mono-substituierten C₂₋₁₀-Alkinylrest bezeichnen
oder
R⁸ und R⁹, zusammen mit dem Brücken-Stickstoffatom einen gesättigten oder ungesättigten, optional mindestens mono-substituierten 5- oder 6-gliedrigen heterocyclischen Ring bilden, der mindestens ein zusätzliches Heteroatom als ein Ringglied enthalten kann und/oder der mit einem gesättigten oder ungesättigten, optional mindestens mono-substituierten mono- oder bicyclischen cycloaliphatischen Ringsystem, das optional mindestens ein Heteroatom als ein Ringglied enthalten kann, kondensiert sein kann, wobei die Ringe des Ringsystems 5-, 6- oder 7-gliedrig sind.

6. Verbindung gemäss Anspruch 5, **dadurch gekennzeichnet, dass** R⁸ und R⁹, gleich oder verschieden, jeweils Wasserstoff oder einen linearen oder verzweigten, C₁₋₁₀-Alkylrest bezeichnen, oder
R⁸ und R⁹ zusammen mit dem Brücken-Stickstoffatom einen Rest bilden, der ausgewählt ist aus der Gruppe bestehend aus: 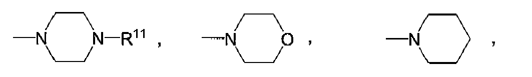 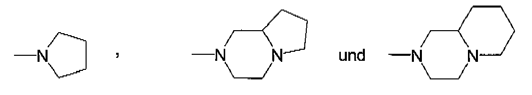 worin R¹¹, wenn es vorliegt, Wasserstoff, einen linearen oder verzweigten C₁₋₆-Alkylrest oder einen Benzylrest bezeichnet.

7. Verbindung gemäss einem oder mehreren der Ansprüche 1 bis 6, **dadurch gekennzeichnet, dass** A ein optional mindestens mono-substituiertes mono- oder polycyclisches aromatisches Ringsystem bezeichnet, in dem der Ring/die Ringe 5- oder 6-gliedrig ist/sind, der/die über eine optional mindestens mono-substituierte C₁₋₆-Alkylengruppe, eine optional mindestens mono-substituierte C₂₋₆-Alkenylengruppe oder eine optional mindestens mono-substituierte C₂₋₆-Alkinylengruppe gebunden sein kann/können und/oder in dem der Ring/die Ringe mindestens ein Heteroatom als ein Ringglied enthalten kann/enthalten können.

8. Verbindung gemäss einem oder mehreren der Ansprüche 1 bis 7, ausgewählt aus der Gruppe bestehend aus:
(16) N-[1-(2-Pyrrolidin-1-yl-ethyl)-1H-indol-5-yl]-naphthalin-2-sulfonamid,
(17) N-[1-(2-Pyrrolidin-1-yl-ethyl)-1H-indol-5-yl]-naphthalin-1-sulfonamid,
(18) N-[1-(2-Pyrrolidin-1-yl-ethyl)-1H-indol-5-yl]-5-chlor-3-methylbenzo[b]thiophen-2-sulfonamid,
(28) N-[1-(2-Pyrrolidin-1-yl-ethyl)-1H-indol-5-yl]-6-chlorimidazo[2,1-b]thiazol-5-sulfonamid,
(43) 5-Chlor-3-methyl-N-(1-(3-(piperidin-1-yl)propyl)-1H-indol-5-yl)benzo[b]thiophen-2-sulfonamid,
(44) N-(1-(3-(Piperidin-1-yl)propyl)-1H-indol-5-yl)naphthalin-2-sulfonamid,
(45) N-(1-(3-(Piperidin-1-yl)propyl)-1H-indol-5-yl)naphthalin-1-sulfonamid,
(46) 6-Chlor-N-(1-(3-piperidin-1-yl)propyl)-1H-indol-5-yl)imidazo[2,1-b]thiazol-5-sulfonamid,
(47) 4-Phenyl-N-(1-(3-(piperidin-1-yl)propyl)-1H-indol-5-yl)benzolsulfonamid,
(48) 2-(Naphth-1-yl)-N-(1-(3-(piperidin-1-yl)propyl)-1H-indol-5-yl)ethansulfonamid,
(49) 4-Phenoxy-N-(1-(3-(piperidin-1-yl)propyl)-1H-indol-5-yl)benzolsulfonamid,
(50) 3,5-Dichlor-N-(1-(3-(piperidin-1-yl)propyl)-1H-indol-5-yl)benzolsulfonamid,
(51) 4,5-Dichlor-N-(1-(3-(piperidin-1-yl)propyl)-1H-indol-5-yl)thiophen-2-sulfonamid und
(52) 5-Chlor-N-(1-(3-(piperidin-1-yl)propyl)-1H-indol-5-yl)naphthalin-1-sulfonamid
optional in Form ihrer korrespondierenden Salze oder ihrer korrespondierenden Solvate.

9. Sulfonamidverbindung der allgemeinen Formel (Ib): 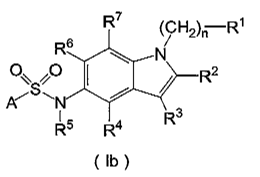 worin
R¹ einen -NR⁸R⁹-Rest bezeichnet,
R², R³, R⁴, R⁶ und R⁷, gleich oder verschieden, jeweils Wasserstoff, Halogen, Nitro, Alkoxy, Cyano, einen gesättigten oder ungesättigten, optional mindestens mono-substituierten, linearen oder verzweigten, aliphatischen Rest oder einen optional mindestens mono-substituierten Phenyl- oder einen optional mindestens mono-substituierten Heteroarylrest bezeichnen,
R⁵ Wasserstoff oder einen gesättigten oder ungesättigten, linearen oder verzweigten, optional mindestens mono-substituierten aliphatischen Rest bezeichnet,
R⁸ und R⁹, gleich oder verschieden, jeweils Wasserstoff oder einen gesättigten oder ungesättigten, linearen oder verzweigten, optional mindestens mono-substituierten aliphatischen C₁₋₄-Rest bezeichnen,
A ein optional mindestens mono-substituiertes mono- oder polycyclisches aromatisches Ringsystem bezeichnet, das über eine optional mindestens mono-substituierte Alkylen-, Alkenylen- oder Alkinylengruppe gebunden sein kann und/oder das mindestens ein Heteroatom als ein Ringglied in einem oder mehreren seiner Ringe enthalten kann,
und n 0, 1, 2, 3 oder 4 ist;
optional in Form eines ihrer Stereoisomere, vorzugsweise Enantiomere oder Diastereomere, ihres Racemats oder in Form einer Mischung von mindestens zwei ihrer Stereoisomere, vorzugsweise Enantiomere oder Diastereomere, in beliebigem Mischungsverhältnis, oder ein Salz davon, vorzugsweise ein korrespondierendes, physiologisch akzeptables Salz davon oder ein korrespondierendes Solvat davon.

10. Verbindung gemäss Anspruch 9, **dadurch gekennzeichnet, dass** R², R³, R⁴, R⁶ und R⁷, gleich oder verschieden, jeweils Wasserstoff, einen linearen oder verzweigten, optional mindestens mono-substituierten C₁₋₆-Alkylrest, einen linearen oder verzweigten, optional mindestens mono-substituierten C₂₋₆-Alkenylrest oder einen linearen oder verzweigten, optional mindestens mono-substituierten C₂₋₆-Alkinylrest bezeichnen.

11. Verbindung gemäss Anspruch 9 oder 10, **dadurch gekennzeichnet, dass** R⁵ Wasserstoff, einen linearen oder verzweigten, optional mindestens mono-substituierten C₁₋₆-Alkylrest, einen linearen oder verzweigten, optional mindestens mono-substituierten C₂₋₆-Alkenylrest oder einen linearen oder verzweigten, optional mindestens mono-substituierten C₂₋₆-Alkinylrest bezeichnet.

12. Verbindung gemäss einem oder mehreren der Ansprüche 9 bis 11, **dadurch gekennzeichnet, dass** R⁸ und R⁹, gleich oder verschieden, jeweils Wasserstoff oder einen linearen oder verzweigten, optional mindestens mono-substituierten C₁₋₄-Alkylrest bezeichnen, mit der Massgabe, dass R⁸ und R⁹ nicht gleichzeitig Wasserstoff, sind.

13. Verbindung gemäss einem oder mehreren der Ansprüche 9 bis 12, **dadurch gekennzeichnet, dass** A ein optional mindestens mono-substituiertes mono- oder polycyclisches aromatisches Ringsystem bezeichnet, in dem der Ring/die Ringe 5- oder 6-gliedrig ist/sind, der/die über eine optional mindestens mono-substituierte C₁₋₆-Alkylengruppe, eine optional mindestens mono-substituierte C₂₋₆-Alkenylengruppe oder eine optional mindestens mono-substituierte C₂₋₆-Alkinylengruppe gebunden sein kann/können und/oder in dem der Ring/die Ringe mindestens ein Heteroatom als ein Ringglied enthalten kann/enthalten können.

14. Verbindung gemäss einem oder mehreren der Ansprüche 9 bis 13, ausgewählt aus der Gruppe bestehend aus:
(1) N-[1-(2-Dimethylaminoethyl)-1H-indol-5-yl]-5-chlor-3-methylbenzo[b]thiophen-2-sulfonamid,
(2) N-[1-(2-Dimethylaminoethyl)-1H-indol-5-yl]-naphthalin-2-sulfonamid,
(3) N-[1-(2-Dimethylaminoethyl)-1H-indol-5-yl]-naphthalin-1-sulfonamid,
(4) N-[1-(2-Dimethylaminoethyl)-1H-indol-5-yl]-5-chlornaphthalin-1-sulfonamid,
(5) N-[1-(2-Dimethylaminoethyl)-1H-indol-5-yl]-benzolsulfonamid,
(6) N-[1-(2-Dimethylaminoethyl)-1H-indol-5-yl]-chinolin-8-sulfonamid,
(7) N-[1-(2-Dimethylaminoethyl)-1H-indol-5-yl]-4-phenoxybenzolsulfonamid,
(8) N-[1-(2-Dimethylaminoethyl)-1H-indol-5-yl]-4-methylbenzolsulfonamid,
(9) N-[1-(2-Dimethylaminoethyl)-1H-indol-5-yl]-5-chlorthiophen-2-sulfonamid,
(10) N-[1-(2-Dimethylaminoethyl)-1H-indol-5-yl]-benzo[1,2,5]thiadiazol-9-sulfonamid,
(11) N-[1-(2-Dimethylaminoethyl)-1H-indol-5-yl]-6-chlorimidazo[2,1-b]thiazol-5-sulfonamid,
(12) N-[1-(2-Dimethylaminoethyl)-1H-indol-5-yl]-3,5-dichlorbenzolsulfonamid,
(13) N-[1-(2-Dimethylaminoethyl)-1H-indol-5-yl]-3-brombenzolsulfonamid,
(14) N-[1-(2-Dimethylaminoethyl)-1H-indol-5-yl]-3-nitrobenzolsulfonamid,
(15) N-[1-(2-Dimethylaminoethyl)-1H-indol-5-yl]-1-phenylmethansulfonamid,
(19) trans-N-[1-(2-Dimethylaminoethyl)-1H-indol-5-yl]-2-phenylethensulfonamid,
(20) N-[1-(2-Dimethylaminoethyl)-1H-indol-5-yl]-4,5-dichlorthiophen-2-sulfonamid,
(21) N-[1-(2-Dimethylaminoethyl)-1H-indol-5-yl]-4-acetylbenzolsulfonamid,
(22) N-[1-(2-Dimethylaminoethyl)-1H-indol-5-yl]-4-brombenzolsulfonamid,
(23) N-[1-(2-Dimethylaminoethyl)-1H-indol-5-yl]-9-methoxybenzolsulfonamid,
(24) N-[3-(2-Diethylaminoethyl)-1H-indol-5-yl]-5-chlor-3-methylbenzo[b]thiophen-2-sulfonamid,
(25) N-[1-(2-Dimethylaminoethyl)-1H-indol-5-yl]-4-nitrobenzolsulfonamid,
(26) N-[1-(2-Dimethylaminoethyl)-1H-indol-5-yl]-4-fluorbenzolsulfonamid,
(27) N-[1-(2-Diethylaminoethyl)-1H-indol-5-yl]-6-chlorimidazo[2,1-b]thiazol-5-sulfonamid,
(29) N-[1-(2-Diethylaminoethyl)-1H-indol-5-yl]-naphthalin-2-sulfonamid,
(30) N-(1-(2-(Diethylamino)ethyl)-1H-indol-5-yl)-naphthalin-1-sulfonamid,
(31) N-(1-(2-(Diethylamino)ethyl)-1H-indol-5-yl)-4-phenylbenzolsulfonamid,
(32) 5-Chlor-N-(1-(2-(dimethylamino)ethyl)-2-methyl-1H-indol-5-yl)-3-methylbenzo[b]thiophen-2-sulfonamid,
(33) N-(1-(2-(Dimethylamino)ethyl)-2-methyl-1H-indol-5-yl)-naphthalin-2-sulfonamid,
(34) N-(1-(2-(Dimethylamino)ethyl)-2-methyl-1H-indol-5-yl)-naphthalin-1-sulfonamid,
(35) 6-Chlor-N-(1-(2-(dimethylamino)ethyl)-2-methyl-1H-indol-5-yl)imidazo[2,1-b]thiazol-5-sulfonamid,
(36) N-(1-(2-(Dimethylamino)ethyl)-2-methyl-1H-indol-5-yl)-4-phenylbenzolsulfonamid,
(37) N-(1-(2-(Dimethylamino)ethyl)-2-methyl-1H-indol-5-yl)-2-(naphth-1-yl)-ethansulfonamid,
(38) N-(1-(2-(Dimethylamino)ethyl)-2-methyl-1H-indol-5-yl)-4-phenoxybenzolsulfonamid,
(39) 3,5-Dichlor-N-(1-(2-(dimethylamino)ethyl)-2-methyl-1H-indol-5-yl)-benzolsulfonamid,
(40) N-(1-(2-(Dimethylamino)ethyl)-2-methyl-1H-indol-5-yl)benzo[b]thiophen-3-sulfonamid,
(41) N-(1-(2-(Diethylamino)ethyl)-1H-indol-5-yl)-benzo[b]thiophen-3-sulfonamid und
(42) N-(1-(2-(Dimethylamino)ethyl)-1H-indol-5-yl)-benzo[b]thiophen-3-sulfonamid,
optional in Form ihrer korrespondierenden Salze und ihrer korrespondierenden Solvate.

15. Verfahren, um ein Sulfonamidderivat der allgemeinen Formel (Ia) und/oder (Ib) gemäss einem oder mehreren der Ansprüche 1 bis 14 zu erhalten, **dadurch gekennzeichnet, dass** mindestens eine Verbindung der allgemeinen Formel (II) oder eines ihrer geeignet geschützten Derivate: 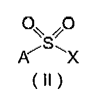 worin A die Bedeutung gemäss einem oder mehreren der Ansprüche 1 bis 14 hat und X eine akzeptable Abgangsgruppe ist, mit mindestens einem 5-Aminoindol der allgemeinen Formel (III) oder einem seiner geeignet geschützten Derivate umgesetzt wird: 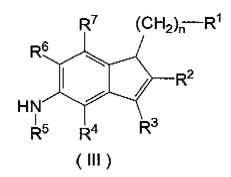 worin R¹ bis R⁷ und n die Bedeutungen gemäss einem oder mehreren der Ansprüche 1 bis 14 haben, um das korrespondierende Sulfonamid zu erhalten, und optional von letzterem die Schutzgruppen, falls notwendig, entfernt werden können.

16. Verfahren, um ein Sulfonamidderivat der allgemeinen Formel (Ia) und/oder (Ib) gemäss einem oder mehreren der Ansprüche 1 bis 14 zu erhalten, bei dem R¹ bis R⁴, R⁶ bis R⁷, n und A die Bedeutungen gemäss einem oder mehreren der Ansprüche 1 bis 14 haben und R⁵ C₁₋₆-Alkyl bezeichnet, **dadurch gekennzeichnet, dass** mindestens eine Verbindung der allgemeinen Formel (Ia) und/oder mindestens eine Verbindung der allgemeinen Formel (Ib), worin R¹ bis R⁴, R⁶ bis R⁷, n und A die Bedeutungen gemäss einem oder mehreren der Ansprüche 1 bis 14 haben und R⁵ ein Wasserstoffatom bezeichnet, mit einem Alkylhalogenid oder Dialkylsulfat umgesetzt wird.

17. Verfahren zur Herstellung der Salze der Verbindungen der allgemeinen Formel (Ia) und/oder (Ib) gemäss einem oder mehreren der Ansprüche 1 bis 14, das aus dem Umsetzen mindestens einer Verbindung der allgemeinen Formel (Ia) und/oder mindestens einer Verbindung der allgemeinen Formel (Ib) mit einer Mineralsäure oder einer organischen Säure in einem geeigneten Lösungsmittel besteht.

18. Medikament, das mindestens eine Verbindung gemäss einem oder mehreren der Ansprüche 1 bis 8 und optional einen oder mehrere pharmakologisch akzeptable Hilfsstoffe umfasst.

19. Medikament gemäss Anspruch 18 für die 5-HT₆-Rezeptorregulation, zur Prophylaxe und/oder Behandlung einer Störung oder Krankheit, die im Zusammenhang mit der Nahrungsaufnahme steht, zur Prophylaxe und/oder Behandlung der Fettleibigkeit, Bulimie, Anorexie, Kachexie oder des Typ II-Diabetes (nicht-insulinabhängiger Diabetes mellitus), zur Prophylaxe und/oder Behandlung von Störungen des Magen-Darm-Trakts, zur kognitiven Steigerung, zur Prophylaxe und/oder Behandlung von Störungen des zentralen Nervensystems, Angst, Panikstörungen, Depression, bipolaren Störungen, kognitiven Gedächtnisstörungen, senilen Demenzprozessen, neurodegenerativen Störungen, Schizophrenie, Psychose oder Hyperkinese bei Kindern (ADHD, Aufmerksamkeits-Defizit-Störung mit Hyperaktivität).

20. Medikament gemäss Anspruch 19, wobei die Störung oder Krankheit, die im Zusammenhang mit der Nahrungsaufnahme steht, ausgewählt ist aus der Gruppe bestehend aus der Regulation des Appetits und der Aufrechterhaltung, Erhöhung oder Verringerung des Körpergewichts.

21. Medikament gemäss Anspruch 19, wobei der Typ II-Diabetes durch Fettleibigkeit hervorgerufener Typ II-Diabetes ist.

22. Medikament gemäss Anspruch 19, wobei die Störung des Magen-Darm-Trakts das Reizdarmsyndrom ist.

23. Medikament gemäss Anspruch 19, wobei die neurodegenerative Störung ausgewählt ist aus der Gruppe bestehend aus Alzheimer-Krankheit, Parkinson-Krankheit, Huntington-Krankheit und/oder Multipler Sklerose.

24. Verwendung mindestens einer Verbindung gemäss einem oder mehreren der Ansprüche 1 bis 8 zur Herstellung eines Medikaments für die 5-HT₆-Rezeptorregulierung.

25. Verwendung mindestens einer Verbindung gemäss einem oder mehreren der Ansprüche 1 bis 8 zur Herstellung eines Medikaments zur Prophylaxe und/oder Behandlung einer Störung oder Krankheit, die im Zusammenhang mit der Nahrungsaufnahme steht.

26. Verwendung mindestens einer Verbindung gemäss einem oder mehreren der Ansprüche 1 bis 8 zur Herstellung eines Medikaments zur Regulierung des Appetits.

27. Verwendung mindestens einer Verbindung gemäss einem oder mehreren der Ansprüche 1 bis 8 zur Herstellung eines Medikaments zur Aufrechterhaltung, Erhöhung oder Verringerung des Körpergewichts.

28. Verwendung mindestens einer Verbindung gemäss einem oder mehreren der Ansprüche 1 bis 8 zur Herstellung eines Medikaments zur Prophylaxe und/oder Behandlung der Fettleibigkeit.

29. Verwendung mindestens einer Verbindung gemäss einem oder mehreren der Ansprüche 1 bis 8 zur Herstellung eines Medikaments zur Prophylaxe und/oder Behandlung der Bulimie.

30. Verwendung mindestens einer Verbindung gemäss einem oder mehreren der Ansprüche 1 bis 8 zur Herstellung eines Medikaments zur Prophylaxe und/oder Behandlung der Anorexie.

31. Verwendung mindestens einer Verbindung gemäss einem oder mehreren der Ansprüche 1 bis 8 zur Herstellung eines Medikaments zur Prophylaxe und/oder Behandlung der Kachexie.

32. Verwendung mindestens einer Verbindung gemäss einem oder mehreren der Ansprüche 1 bis 8 zur Herstellung eines Medikaments zur Prophylaxe und/oder Behandlung von Typ II-Diabetes (nicht-insulinabhängiger Diabetes mellitus).

33. Verwendung gemäss Anspruch 32, wobei der Typ II-Diabetes durch Fettleibigkeit hervorgerufener Typ II-Diabetes ist.

34. Verwendung mindestens einer Verbindung gemäss einem oder mehreren der Ansprüche 1 bis 8 zur Herstellung eines Medikaments zur Prophylaxe und/oder Behandlung von Störungen des Magen-Darm-Trakts.

35. Verwendung mindestens einer Verbindung gemäss einem oder mehreren der Ansprüche 1 bis 8 zur Herstellung eines Medikaments zur Prophylaxe und/oder Behandlung des Reizdarmsyndroms.

36. Verwendung mindestens einer Verbindung gemäss einem oder mehreren der Ansprüche 1 bis 8 zur Herstellung eines Medikaments zur Prophylaxe und/oder Behandlung von Angst.

37. Verwendung mindestens einer Verbindung gemäss einem oder mehreren der Ansprüche 1 bis 8 zur Herstellung eines Medikaments zur Prophylaxe und/oder Behandlung von Depression.

38. Verwendung mindestens einer Verbindung gemäss einem oder mehreren der Ansprüche 1 bis 8 zur Herstellung eines Medikaments zur Prophylaxe und/oder Behandlung von bipolaren Störungen.

39. Verwendung mindestens einer Verbindung gemäss einem oder mehreren der Ansprüche 1 bis 8 zur Herstellung eines Medikaments zur Prophylaxe und/oder Behandlung von kognitiven Gedächtnisstörungen.

40. Verwendung mindestens einer Verbindung gemäss einem oder mehreren der Ansprüche 1 bis 8 zur Herstellung eines Medikaments zur Prophylaxe und/oder Behandlung von senilen Demenzprozessen.

41. Verwendung mindestens einer Verbindung gemäss einem oder mehreren der Ansprüche 1 bis 8 zur Herstellung eines Medikaments zur Prophylaxe und/oder Behandlung der Alzheimer-Krankheit.

42. Verwendung mindestens einer Verbindung gemäss einem oder mehreren der Ansprüche 1 bis 8 zur Herstellung eines Medikaments zur Prophylaxe und/oder Behandlung der Parkinson-Krankheit.

43. Verwendung mindestens einer Verbindung gemäss einem oder mehreren der Ansprüche 1 bis 8 zur Herstellung eines Medikaments zur Prophylaxe und/oder Behandlung der Huntington-Krankheit.

44. Verwendung mindestens einer Verbindung gemäss einem oder mehreren der Ansprüche 1 bis 8 zur Herstellung eines Medikaments zur Prophylaxe und/oder Behandlung von Demenzen, bei denen ein kognitives Defizit dominiert.

45. Verwendung mindestens einer Verbindung gemäss einem oder mehreren der Ansprüche 1 bis 8 zur Herstellung eines Medikaments zur Prophylaxe und/oder Behandlung der Multiplen Sklerose.

46. Verwendung mindestens einer Verbindung gemäss einem oder mehreren der Ansprüche 1 bis 8 zur Herstellung eines Medikaments zur Prophylaxe und/oder Behandlung von Psychose.

47. Verwendung mindestens einer Verbindung gemäss einem oder mehreren der Ansprüche 1 bis 8 zur Herstellung eines Medikaments zur Prophylaxe und/oder Behandlung der Hyperkinese bei Kindern (ADHD, Aufmerksamkeits-Defizit-Störung mit Hyperaktivität).

48. Verwendung mindestens einer Verbindung gemäss einem oder mehreren der Ansprüche 1 bis 8 zur Herstellung eines Medikaments zur Prophylaxe und/oder Behandlung von Störungen des zentralen Nervensystems.

49. Verwendung mindestens einer Verbindung gemäss einem oder mehreren der Ansprüche 1 bis 8 zur Herstellung eines Medikaments zur Prophylaxe und/oder Behandlung der Schizophrenie.

50. Verwendung mindestens einer Verbindung gemäss einem oder mehreren der Ansprüche 1 bis 8 zur Herstellung eines Medikaments zur kognitiven Steigerung.

51. Medikament, das mindestens eine Verbindung gemäss einem oder mehreren der Ansprüche 9 bis 14 und optional einen oder mehrere pharmakologisch akzeptable Hilfsstoffe umfasst.

52. Medikament gemäss Anspruch 51 für die 5-HT₆-Rezeptorregulierung, zur Prophylaxe und/oder Behandlung einer Störung oder Krankheit, die mit der Nahrungsaufnahme im Zusammenhang steht, zur Prophylaxe und/oder Behandlung von Fettleibigkeit, Bulimie, Anorexie, Kachexie oder Typ II-Diabetes (nicht-insulinabhängiger Diabetes mellitus), zur Prophylaxe und/oder Behandlung von Störungen des Magen-Darm-Trakts, zur kognitiven Steigerung, zur Prophylaxe und/oder Behandlung von Störungen des zentralen Nervensystems, Angst, Panikstörungen, Depression, bipolaren Krankheiten, kognitiven Gedächtnisstörungen, senilen Demenzprozessen, neurodegenerativen Störungen, Schizophrenie, Psychose oder Hyperkinese bei Kindern (ADHD, Aufmerksamkeits-Defizit-Störung mit Hyperaktivität).

53. Medikament gemäss Anspruch 52, wobei die Störung oder Krankheit, die im Zusammenhang mit der Nahrungsaufnahme steht, ausgewählt ist aus der Gruppe bestehend aus der Regulierung von Appetit und der Aufrechterhaltung, Erhöhung oder Verringerung des Körpergewichts.

54. Medikament gemäss Anspruch 52, wobei der Typ II-Diabetes durch Fettleibigkeit hervorgerufener Typ II-Diabetes ist.

55. Medikament gemäss Anspruch 52, wobei die Störung des Magen-Darm-Trakts das Reizdarmsyndrom ist.

56. Medikament gemäss Anspruch 52, wobei die neurodegenerative Störung ausgewählt ist aus der Gruppe bestehend aus Alzheimer-Krankheit, Parkinson-Krankheit, Huntington-Krankheit und/oder Multipler Sklerose.

57. Verwendung mindestens einer Verbindung gemäss einem oder mehreren der Ansprüche 9 bis 14 zur Herstellung eines Medikaments für die 5-HT₆-Rezeptorregulierung.

58. Verwendung mindestens einer Verbindung gemäss einem oder mehreren der Ansprüche 9 bis 14 zur Herstellung eines Medikaments zur Prophylaxe und/oder Behandlung einer Störung oder Krankheit, die im Zusammenhang mit der Nahrungsaufnahme steht.

59. Verwendung mindestens einer Verbindung gemäss einem oder mehreren der Ansprüche 9 bis 14 zur Herstellung eines Medikaments zur Regulierung des Appetits.

60. Verwendung mindestens einer Verbindung gemäss einem oder mehreren der Ansprüche 9 bis 14 zur Herstellung eines Medikaments zur Aufrechterhaltung, Erhöhung oder Verringerung des Körpergewichts.

61. Verwendung mindestens einer Verbindung gemäss einem oder mehreren der Ansprüche 9 bis 14 zur Herstellung eines Medikaments zur Prophylaxe und/oder Behandlung der Fettleibigkeit.

62. Verwendung mindestens einer Verbindung gemäss einem oder mehreren der Ansprüche 9 bis 14 zur Herstellung eines Medikaments zur Prophylaxe und/oder Behandlung der Bulimie.

63. Verwendung mindestens einer Verbindung gemäss einem oder mehreren der Ansprüche 9 bis 14 zur Herstellung eines Medikaments zur Prophylaxe und/oder Behandlung der Anorexie.

64. Verwendung mindestens einer Verbindung gemäss einem oder mehreren der Ansprüche 9 bis 14 zur Herstellung eines Medikaments zur Prophylaxe und/oder Behandlung der Kachexie.

65. Verwendung mindestens einer Verbindung gemäss einem oder mehreren der Ansprüche 9 bis 14 zur Herstellung eines Medikaments zur Prophylaxe und/oder Behandlung von Typ II-Diabetes (nicht-insulinabhängiger Diabetes mellitus).

66. Verwendung gemäss Anspruch 65, bei der der Typ II-Diabetes durch Fettleibigkeit hervorgerufener Typ 11-Diabetes ist.

67. Verwendung mindestens einer Verbindung gemäss einem oder mehreren der Ansprüche 9 bis 14 zur Herstellung eines Medikaments zur Prophylaxe und/oder Behandlung von Störungen des Magen-Darm-Trakts.

68. Verwendung mindestens einer Verbindung gemäss einem oder mehreren der Ansprüche 9 bis 14 zur Herstellung eines Medikaments zur Prophylaxe und/oder Behandlung des Reizdarmsyndroms.

69. Verwendung mindestens einer Verbindung gemäss einem oder mehreren der Ansprüche 9 bis 14 zur Herstellung eines Medikaments zur Prophylaxe und/oder Behandlung von Angst.

70. Verwendung mindestens einer Verbindung gemäss einem oder mehreren der Ansprüche 9 bis 14 zur Herstellung eines Medikaments zur Prophylaxe und/oder Behandlung der Depression.

71. Verwendung mindestens einer Verbindung gemäss einem oder mehreren der Ansprüche 9 bis 14 zur Herstellung eines Medikaments zur Prophylaxe und/oder Behandlung von bipolaren Krankheiten.

72. Verwendung mindestens einer Verbindung gemäss einem oder mehreren der Ansprüche 9 bis 14 zur Herstellung eines Medikaments zur Prophylaxe und/oder Behandlung von kognitiven Gedächtnisstörungen.

73. Verwendung mindestens einer Verbindung gemäss einem oder mehreren der Ansprüche 9 bis 14 zur Herstellung eines Medikaments zur Prophylaxe und/oder Behandlung von senilen Demenzprozessen.

74. Verwendung mindestens einer Verbindung gemäss einem oder mehreren der Ansprüche 9 bis 14 zur Herstellung eines Medikaments zur Prophylaxe und/oder Behandlung der Alzheimer-Krankheit.

75. Verwendung mindestens einer Verbindung gemäss einem oder mehreren der Ansprüche 9 bis 14 zur Herstellung eines Medikaments zur Prophylaxe und/oder Behandlung der Parkinson-Krankheit.

76. Verwendung mindestens einer Verbindung gemäss einem oder mehreren der Ansprüche 9 bis 14 zur Herstellung eines Medikaments zur Prophylaxe und/oder Behandlung der Huntington-Krankheit.

77. Verwendung mindestens einer Verbindung gemäss einem oder mehreren der Ansprüche 9 bis 14 zur Herstellung eines Medikaments zur Prophylaxe und/oder Behandlung von Multipler Sklerose.

78. Verwendung mindestens einer Verbindung gemäss einem oder mehreren der Ansprüche 9 bis 14 zur Herstellung eines Medikaments zur Prophylaxe und/oder Behandlung von Demenzen, bei denen ein kognitives Defizit dominiert.

79. Verwendung mindestens einer Verbindung gemäss einem oder mehreren der Ansprüche 9 bis 14 zur Herstellung eines Medikaments zur Prophylaxe und/oder Behandlung der Psychose.

80. Verwendung mindestens einer Verbindung gemäss einem oder mehreren der Ansprüche 9 bis 14 zur Herstellung eines Medikaments zur Prophylaxe und/oder Behandlung der Hyperkinese bei Kindern (ADHD, Aufmerksamkeits-Defizit-Störung mit Hyperaktivität).

81. Verwendung mindestens einer Verbindung gemäss einem oder mehreren der Ansprüche 9 bis 14 zur Herstellung eines Medikaments zur Prophylaxe und/oder Behandlung von Störungen des zentralen Nervensystems.

82. Verwendung mindestens einer Verbindung gemäss einem oder mehreren der Ansprüche 9 bis 14 zur Herstellung eines Medikaments zur Prophylaxe und/oder Behandlung der Schizophrenie.

83. Verwendung mindestens einer Verbindung gemäss einem oder mehreren der Ansprüche 9 bis 14 zur Herstellung eines Medikaments zur kognitiven Steigerung.

## Revendications

1. Composé sulfonamide de formule générale (la), 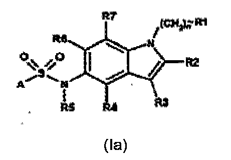 où
R¹ représente un radical -NR⁸R⁹ ou un radical cycloaliphatique facultativement au moins mono-substitué, saturé ou insaturé, qui peut facultativement contenir au moins un hétéroatome en tant que chaînon du cycle et/ou qui peut être condensé avec un système cycloaliphatique mono- ou bi-cyclique, saturé ou insaturé, facultativement au moins mono-substitué, qui peut facultativement contenir au moins un hétéroatome en tant que chaînon du cycle,
R², R³, R⁴, R⁶ et R⁷, identiques ou différents, représentent chacun un hydrogène, un halogène, un nitro, un alcoxy, un cyano, un radical aliphatique facultativement au moins mono-substitué, linéaire ou ramifié, saturé ou insaturé, ou un radical phényle facultativement au moins mono-substitué ou un radical hétéroaryle facultativement au moins mono-substitué,
R⁵ représente un hydrogène ou un radical aliphatique facultativement au moins mono-substitué, linéaire ou ramifié, saturé ou insaturé,
R⁸ et R⁹, identiques ou différents, représentent chacun un hydrogène ou un radical aliphatique facultativement au moins mono-substitué, linéaire ou ramifié, saturé ou insaturé.
à condition que R⁸ et R⁹ ne soient pas un hydrogène en même temps, et si l'un d'entre eux, R⁸ ou R⁹, est un radical aliphatique en C₁ à C₄ facultativement au moins mono-substitué, linéaire ou ramifié, saturé ou insaturé, l'autre représente un radical aliphatique facultativement au moins mono-substitué, linéaire ou ramifié, saturé ou insaturé, possédant au moins cinq atomes de carbone, ou
R⁸ et R⁹ associés à l'atome d'azote de pontage forment un hétérocycle facultativement au moins mono-substitué, saturé ou insaturé, qui peut contenir au moins un hétéroatome supplémentaire en tant que chaînon de cycle et/ou qui peut être condensé avec un système cycloaliphatique mono- ou bi-cyclique facultativement au moins mono-substitué, saturé ou insaturé, qui peut facultativement contenir au moins un hétéroatome en tant que chaînon de cycle,
A représente un système aromatique mono- ou poly-cyclique, facultativement au moins mono-substitué, qui peut être lié par un groupe alkylène, alcénylène ou alcynylène facultativement au moins mono-substitué et/ou qui peut contenir au moins un hétéroatome en tant que chaînon de cycle dans un ou plusieurs de ses cycles,
et
n est égal à 0, 1, 2, 3 ou 4,
facultativement sous la forme de l'un de ses stéréoisomères, de préférence des énantiomères ou diastéréomères, un racémique ou sous la forme d'un mélange constitué d'au moins deux de ses stéréoisomères, de préférence des énantiomères ou diastéréomères, dans un rapport de mélange quelconque, ou l'un de ses sels, de préférence un sel correspondant de celui-ci, physiologiquement acceptable, ou un solvate correspondant de celui-ci.

2. Composé selon la revendication 1, **caractérisé en ce que** R¹ représente un radical -NR⁸R⁹ ou un radical cycloaliphatique à 5 ou 6 chaînons, facultativement au moins mono-substitué, saturé ou insaturé, qui peut facultativement contenir au moins un hétéroatome en tant que chaînon de cycle et/ou qui peut être condensé avec un système cycloaliphatique mono- ou bi-cyclique facultativement au moins mono-substitué, saturé ou insaturé, qui peut facultativement contenir au moins un hétéroatome en tant que chaînon de cycle, de sorte que les cycles du système de cycle comportent 5 ou 6 chaînons.

3. Composé selon la revendication 1 ou 2, **caractérisé en ce que** R², R³, R⁴, R⁶ et R⁷, identiques ou différents, représentent chacun un hydrogène, un radical alkyle en C₁ à C₆ facultativement au moins mono-substitué, linéaire ou ramifié, un radical alcényle en C₂ à C₆ facultativement au moins mono-substitué, linéaire ou ramifié, ou un radical alcynyle en C₂ à C₆ facultativement au moins mono-substitué, linéaire ou ramifié.

4. Composé selon une ou plusieurs des revendications 1 à 3, **caractérisé en ce que** R⁵ représente un hydrogène, un radical alkyle en C₁ à C₆ facultativement au moins mono-substitué, linéaire ou ramifié, un radical alcényle en C₂ à C₆ facultativement au moins mono-substitué, linéaire ou ramifié, ou un radical alcynyle en C₂ à C₆ facultativement au moins mono-substitué, linéaire ou ramifié.

5. Composé selon une ou plusieurs des revendications 1 à 4, **caractérisé en ce que** R⁸ et R⁹, identiques ou différents, représentent chacun un hydrogène, un radical alkyle en C₁ à C₁₀ facultativement au moins mono-substitué, linéaire ou ramifié, un radical alcényle en C₂ à C₁₀ facultativement au moins mono-substitué, linéaire ou ramifié, ou un radical alcynyle en C₂ à C₁₀ facultativement au moins mono-substitué, linéaire ou ramifié
ou
R⁸ et R⁹ associés à l'atome d'azote de pontage forment un hétérocycle à 5 ou 6 chaînons facultativement au moins mono-substitué, saturé ou insaturé, qui peut contenir au moins un hétéroatome supplémentaire en tant que chaînon de cycle et/ou qui peut être condensé avec un système cycloaliphatique mono- ou bi-cyclique, facultativement au moins mono-substitué, saturé ou insaturé, qui peut facultativement contenir au moins un hétéroatome en tant que chaînon de cycle, de sorte que les cycles du système de cycle comportent 5, 6 ou 7 chaînons.

6. Composé selon la revendication 5, **caractérisé en ce que** R⁸ et R⁹, identiques ou différents, représentent chacun un hydrogène ou un radical alkyle en C₁ à C₁₀ linéaire ou ramifié, ou
R⁸ et R⁹ associés à l'atome d'azote de pontage forment un radical choisi parmi le groupe constitué de 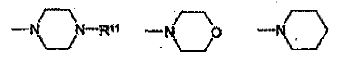 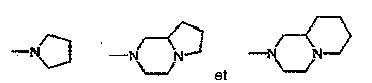 où R¹¹, si présent, représente l'hydrogène, un radical alkyle en C₁ à C₆ linéaire ou ramifié, ou un radical benzyle.

7. Composé selon une ou plusieurs des revendications 1 à 6, **caractérisé en ce que** A représente un système aromatique mono- ou poly-cyclique facultativement au moins mono-substitué, dans lequel le(s) cycle(s) comporte(nt) 5 ou 6 chaînons, qui peut être lié par un groupe alkylène en C₁ à C₆ facultativement au moins mono-substitué, un groupe alcénylène en C₂ à C₆ facultativement au moins mono-substitué ou un groupe alcynylène en C₂ à C₆ facultativement au moins mono-substitué et/ou dans lequel le(s) cycle(s) peut(peuvent) contenir au moins un hétéroatome en tant que chaînon de cycle.

8. Composé selon une ou plusieurs des revendications 1 à 7, choisi parmi le groupe constitué de
[16] N-[1-(2-pyrrolidine-1-yl-éthyl)-1H-indol-5-yl]-naphtalène-2-sulfonamide,
[17] N-[1-(2-pyrrolidine-1-yl-éthyl)-1H-indol-5-yl]-naphtalène-1-sulfonamide,
[18] N-[1-(2-pyrrolidine-1-yl-éthyl)-1H-indol-5-yl]-5-chloro-3-méthylbenzo[b]thiophène-2-sulfonamide,
[28] N-[1-(2-pyrrolidine-1-yl-éthyl)-1H-indol-5-yl]-6-chloroimidazo[2,1-b]thiazole-5-sulfonamide,
[43] 5-chloro-3-méthyl-N-(1-(3-(pipéridin-1-yl)propyl)-1H-indol-5-yl)-benzo[b]thiophène-2-sulfonamide,
[44] N-(1-(3-(pipéridin-1-yl)-propyl)-1H-indol-5-yl)-naphtalène-2-sulfonamide,
[45] N-(1-(3-(pipéridin-1-yl)-propyl)-1H-indol-5-yl)-naphtalène-1-sulfonamide,
[46] 6-chloro-N-(1-(3-(pipéridin-1-yl)propyl)-1H-indol-5-yl)-imidazo[2,1-b]thiazole-5-sulfonamide,
[47] 4-phényl-N-(1-(3-(pipéridin-1-yl)propyl)-1H-indol-5-yl)-benzènesulfonamide,
[48] 2-(napht-1-yl)-N-(1-(3-(pipéridin-1-yl)propyl)-1H-indol-5-yl)-éthanesulfonamide,
[49] 4-phénoxy-N-(1-(3-(pipéridin-1-yl)propyl)-1H-indol-5-yl)-benzènesulfonamide,
[50] 3,5-dichloro-N-(1-(3-(pipéridin-1-yl)propyl)-1H-indol-5-yl)-benzènesulfonylamide,
[51] 4,5-dichloro-N-(1-(3-(pipéridin-1-yl)propyl)-1H-indol-5-yl)-thiophène-2-sulfonamide, et
[52] 5-chloro-N-(1-(3-(pipéridin-1-yl)propyl)-1H-indol-5-yl)-naphtalène-1-sulfonamide,
facultativement sous la forme de leurs sels et solvates correspondants.

9. Composé sulfonamide de formule générale (Ib), 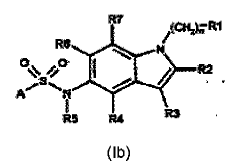 où
R¹ représente un radical -NR⁸R⁹,
R², R³, R⁴, R⁶ et R⁷, identiques ou différents, représentent chacun un hydrogène, un halogène, un nitro, un alcoxy, un cyano, un radical aliphatique facultativement au moins mono-substitué, linéaire ou ramifié, saturé ou insaturé, ou un radical phényle facultativement au moins mono-substitué ou un radical hétéroaryle facultativement au moins mono-substitué,
R⁵ représente un hydrogène ou un radical aliphatique facultativement au moins mono-substitué, linéaire ou ramifié, saturé ou insaturé,
R⁸ et R⁹, identiques ou différents, représentent chacun un hydrogène ou un radical aliphatique en C₁ à C₄ facultativement au moins mono-substitué, linéaire ou ramifié, saturé ou insaturé,
A représente un système aromatique mono- ou poly-cyclique facultativement au moins mono-substitué, qui peut être lié par un groupe alkylène alcénylène ou alcynylène facultativement au moins mono-substitué et/ou qui peut contenir au moins un hétéroatome en tant que chaînon de cycle dans un ou plusieurs de ses cycles,
et n est égal à 0, 1, 2, 3 ou 4,
facultativement sous la forme de l'un de ses stéréoisomères, de préférence des énantiomères ou diastéréomères, son racémique ou sous la forme d'un mélange constitué d'au moins deux de ses stéréoisomères, de préférence des énantiomères ou diastéréomères, dans un rapport de mélange quelconque, ou l'un de ses sels, de préférence un sel correspondant de celui-ci, physiologiquement acceptable, ou un solvate correspondant de celui-ci.

10. Composé selon la revendication 9, **caractérisé en ce que** R², R³, R⁴, R⁶ et R⁷, identiques ou différents, représentent chacun un hydrogène, un radical alkyle en C₁ à C₆ facultativement au moins mono-substitué, linéaire ou ramifié, un radical alcényle en C₂ à C₆ facultativement au moins mono-substitué, linéaire ou ramifié, ou un radical alcynyle en C₂ à C₆ facultativement au moins mono-substitué, linéaire ou ramifié.

11. Composé selon la revendication 9 ou 10, **caractérisé en ce que** R⁵ représente un hydrogène, un radical alkyle en C₁ à C₆ facultativement au moins mono-substitué, linéaire ou ramifié, un radical alcényle en C₂ à C₆ facultativement au moins mono-substitué, linéaire ou ramifié, ou un radical alcynyle en C₂ à C₆ facultativement au moins mono-substitué, linéaire ou ramifié.

12. Composé selon une ou plusieurs des revendications 9 à 11, **caractérisé en ce que** R⁸ et R⁹, identiques ou différents, représentent chacun un hydrogène ou un radical alkyle en C₁ à C₄ facultativement au moins mono-substitué, linéaire ou ramifié, à condition que R⁸ et R⁹ ne soient pas un hydrogène en même temps.

13. Composé selon une ou plusieurs des revendications 9 à 12, **caractérisé en ce que** A représente un système aromatique mono- ou poly-cyclique facultativement au moins mono-substitué, dans lequel le(s) cycle(s) comporte(nt) 5 ou 6 chaînons, qui peut être lié par un groupe alkylène en C₁ à C₆ facultativement au moins mono-substitué, un groupe alcénylène en C₂ à C₆ facultativement au moins mono-substitué ou un groupe alcynylène en C₂ à C₆ facultativement au moins mono-substitué et/ou dans lequel le(s) cycle(s) peut(peuvent) contenir au moins un hétéroatome en tant que chaînon de cycle.

14. Composé selon une ou plusieurs des revendications 9 à 13, choisi parmi le groupe constitué de
[1] N-[1-(2-diméthylaminoéthyl)-1H-indol-5-yl]-5-choloro-3-méthylbenzo[b]thiophène-2-sulfonamide,
[2] N-[1-(2-diméthylaminoéthyl)-1H-indol-5-yl]-naphtalène-2-sulfonamide,
[3] N-[1-(2-diméthylaminoéthyl)-1H-indol-5-yl]-naphtalène-1-sulfonamide,
[4] N-[1-(2-diméthylaminoéthyl)-1H-indol-5-yl]-5-chloronaphtalène-1-sulfonamide,
[5] N-[1-(2-diméthylaminoéthyl)-1H-indol-5-yl]-benzènesulfonamide,
[6] N-[1-(2-diméthylaminoéthyl)-1H-indol-5-yl]-quinoline-8-sulfonamide,
[7] N-[1-(2-diméthylaminoéthyl)-1H-indol-5-yl]-4-phénoxybenzènesulfonamide.
[8] N-[1-(2-diméthylaminoéthyl)-1H-indol-5-yl]-4-méthylbenzènesulfonamide,
[9] N-[1-(2-diméthylaminoéthyl)-1H-indol-5-yl]-5-chloroth iophène-2-sulfonamide,
[10] N-[1-(2-diméthylaminoéthyl)-1H-indol-5-yl]-benzo[1,2,5]thiadiazole-4-sulfonamide,
[11] N-[1-(2-diméthylaminoéthyl)-1H-indol-5-yl]-6-chloroimidazo[2,1-b]thiazole-5-sulfonamide,
[12] N-[1-(2-diméthylaminoéthyl)-1H-indol-5-yl]-3,5-dichlorobenzènesulfonamide,
[13] N-[1-(2-diméthylaminoéthyl)-1H-indol-5-yl]-3-bromobenzènesulfonamide,
[14] N-[1-(2-diméthylaminoéthyl)-1H-indol-5-yl]-3-nitrobenzènesulfonamide,
[15] N-[1-(2-diméthylaminoéthyl)-1H-indoi-5-yl]-1-phényiméthanesuifonamide,
[19] trans-N-[1-(2-diméthylaminoéthyl)-1H-indol-5-yl]-2-phényléthènesulfonamide,
[20] N-[1-(2-diméthylaminoéthyl)-1H-indol-5-yl]-4,5-dichlorothiophène-2-sulfonamide,
[21] N-[1-(2-diméthylaminoéthyl)-1H-indol-5-yl]-4-acétylbenzènesulfonamide,
[22] N-[1-(2-diméthylaminoéthyl)-1H-indol-5-yl]-4-bromobenzènesulfonamide,
[23] N-[1-(2-diméthylaminoéthyl)-1H-indol-5-yl]-4-méthoxybenzènesulfonamide,
[24] N-[3-(2-diéthylaminoéthyl)-1H-indol-5-yl]-5-chloro-3-méthylbenzo[b]thiophène-2-sulfonamide,
[25] N-[1-(2-diméthylaminoéthyl)-1H-indol-5-yl]-4-nitrobenzènesulfonamide,
[26] N-[1-(2-diméthylaminoéthyl)-1H-indol-5-yl]-4-fluorobenzènesulfonamide,
[27] N-[1-(2-diéthylaminoéthyl)-1H-indol-5-yl]-6-chloroimidazo[2,1-b]thiazole-5-sulfonamide,
[29] N-(1-(2-diéthylamino)éthyl)-1H-indol-5-yl)-naphtalène-2-sulfonamide,
[30] N-(1-(2-diéthylamino)éthyl)-1H-indol-5-yl)-naphtalène-1-sulfonamide,
[31] N-(1-(2-diéthylamino)éthyl)-1H-indol-5-yl)-4-phénylbenzènesulfonamide,
[32] 5-chloro-N-(1-(2-diméthylamino)éthyl)-2-méthyl-1H-indol-5-yl)-3-méthylbenzo[b]thiophène-2-sulfonamide,
[33] N-(1-(2-diméthylamino)éthyl)-2-méthyl-1H-indol-5-yl)-naphtalène-2-sulfonamide,
[34] N-(1-(2-diméthylamino)éthyl)-2-méthyl-1H-indol-5-yl)-naphtalène-1-sulfonamide,
[35] 6-chloro-N-(1-(2-diméthylamino)éthyl)-2-méthyl-1H-indol-5-yl)-imidazo[2,1-b]thiazol-5-sulfonamide,
[36] N-(1-(2-diméthylamino)éthyl)-2-méthyl-1H-indol-5-yl)-4-phénylbenzènesulfonamide,
[37] N-(1-(2-diméthylamino)éthyl)-2-méthyl-1H-indol-5-yl)-2-(napht-1-yl)-éthanesulfonamide,
[38] N-(1-(2-diméthylamino)éthyl)-2-méthyl-1H-indol-5-yl)-4-phénoxybenzènesulfonamide,
[39] 3,5-dichloro-N-(1-(2-diméthylamino)éthyl)-2-méthyl-1H-indol-5-yl)-benzènesulfonamide,
[40] N-(1-(2-diméthylamino)éthyl)-2-méthyl-1H-indol-5-yl)-benzo[b]thiophène-3-sulfonamide,
[41] N-(1-(2-diéthylamino)éthyl)-1H-indol-5-yl)-benzo[b]thiophène-3-sulfonamide, et
[42] N-(1-(2-diméthylamino)éthyl)-1H-indol-5-yl)-benzo[b]thiophène-3-sulfonamide,
facultativement sous la forme de leurs sels et solvates correspondants.

15. Procédé pour obtenir un dérivé sulfonamide de formule générale (la) et/ou (Ib), selon une ou plusieurs des revendications 1 à 14, **caractérisé en ce qu'**au moins un composé de formule générale (II), ou l'un de ses dérivés protégés de manière adaptée, 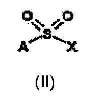 où A a la signification selon une ou plusieurs des revendications 1 à 14, et X représente un groupe partant acceptable, réagit avec au moins un 5-aminoindole de formule générale (III), ou l'un de ses dérivés protégés de manière adaptée, 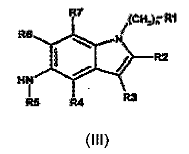 où R¹ à R⁷ et n ont la signification selon une ou plusieurs des revendications 1 à 14 pour obtenir le sulfonamide correspondant et facultativement, à partir de ce dernier, les groupes protecteurs peuvent être éliminés si nécessaire.

16. Procédé pour obtenir un dérivé sulfonamide de formule générale (la) et/ou (Ib), selon une ou plusieurs des revendications 1 à 14, dans lequel R¹ à R⁴, R⁶ à R⁷, n et A ont la signification selon une ou plusieurs des revendications 1 à 14, et R⁵ représente un alkyle en C₁ à C₆, **caractérisé en ce qu'**au moins un composé de formule générale (la) et/ou au moins un composé de formule générale (Ib), où R¹ à R⁴, R⁶ à R⁷, n et A ont la signification selon une ou plusieurs des revendications 1 à 14, et R⁵ représente un atome d'hydrogène, réagit avec un halogénure d'alkyle ou un sulfate de dialkyle.

17. Procédé pour préparer les sels des composés de formule générale (la) et/ou (Ib), selon une ou plusieurs des revendications 1 à 14, consistant à faire réagir au moins un composé ayant la formule générale (la) et/ou au moins un composé ayant la formule générale (Ib) avec un acide minéral ou un acide organique dans un solvant adapté.

18. Médicament comportant au moins un composé selon une ou plusieurs des revendications 1 à 8 et facultativement au moins un ou plusieurs excipients pharmacologiquement acceptables.

19. Médicament selon la revendication 18, pour la régulation des récepteurs 5-HT₆, pour la prophylaxie et/ou le traitement d'une maladie ou d'un trouble lié à la consommation d'aliments, pour la prophylaxie et/ou le traitement de l'obésité, de la boulimie, de l'anorexie, de la cachexie ou du diabète de type II (diabète sucré non insulino-dépendant), pour la prophylaxie et/ou le traitement des troubles du tractus gastro-intestinal, pour l'amélioration cognitive, pour la prophylaxie et/ou le traitement des troubles du système nerveux central, de l'anxiété, de la panique, de la dépression, des troubles bipolaires, des troubles de la mémoire cognitive, des processus de démence sénile, des troubles neurodégénératifs, de la schizophrénie, de la psychose ou de l'hyperkinésie infantile (ADHD, trouble de déficit d'attention/hyperactivité).

20. Médicament selon la revendication 19, dans lequel la maladie ou le trouble lié à la consommation d'aliments est choisi parmi le groupe constitué de la régulation de l'appétit et du maintien, de l'augmentation ou de la diminution du poids corporel.

21. Médicament selon la revendication 19, dans lequel le diabète de type II est un diabète de type II dû à l'obésité.

22. Médicament selon la revendication 19, dans lequel le trouble du tractus gastro-intestinal est le syndrome du côlon irritable.

23. Médicament selon la revendication 19, dans lequel le trouble neurodégénératif est choisi parmi le groupe constitué de la maladie d'Alzheimer, de la maladie de Parkinson, de la maladie de Huntington et/ou de la sclérose en plaques.

24. Utilisation d'au moins un composé selon une ou plusieurs des revendications 1 à 8 pour la fabrication d'un médicament pour la régulation des récepteurs 5-HT₆.

25. Utilisation d'au moins un composé selon une ou plusieurs des revendications 1 à 8 pour la fabrication d'un médicament pour la prophylaxie et/ou le traitement d'une maladie ou d'un trouble lié à la consommation d'aliments.

26. Utilisation d'au moins un composé selon une ou plusieurs des revendications 1 à 8 pour la fabrication d'un médicament pour la régulation de l'appétit.

27. Utilisation d'au moins un composé selon une ou plusieurs des revendications 1 à 8 pour la fabrication d'un médicament pour le maintien, l'augmentation ou la diminution du poids corporel.

28. Utilisation d'au moins un composé selon une ou plusieurs des revendications 1 à 8 pour la fabrication d'un médicament pour la prophylaxie et/ou le traitement de l'obésité.

29. Utilisation d'au moins un composé selon une ou plusieurs des revendications 1 à 8 pour la fabrication d'un médicament pour la prophylaxie et/ou le traitement de la boulimie.

30. Utilisation d'au moins un composé selon une ou plusieurs des revendications 1 à 8 pour la fabrication d'un médicament pour la prophylaxie et/ou le traitement de l'anorexie.

31. Utilisation d'au moins un composé selon une ou plusieurs des revendications 1 à 8 pour la fabrication d'un médicament pour la prophylaxie et/ou le traitement de la cachexie.

32. Utilisation d'au moins un composé selon une ou plusieurs des revendications 1 à 8 pour la fabrication d'un médicament pour la prophylaxie et/ou le traitement du diabète de type II (diabète sucré non insulino-dépendant).

33. Utilisation selon la revendication 32, dans laquelle le diabète de type II est le diabète de type II dû à l'obésité.

34. Utilisation d'au moins un composé selon une ou plusieurs des revendications 1 à 8 pour la fabrication d'un médicament pour la prophylaxie et/ou le traitement des troubles du tractus gastro-intestinal.

35. Utilisation d'au moins un composé selon une ou plusieurs des revendications 1 à 8 pour la fabrication d'un médicament pour la prophylaxie et/ou le traitement du syndrome du côlon irritable.

36. Utilisation d'au moins un composé selon une ou plusieurs des revendications 1 à 8 pour la fabrication d'un médicament pour la prophylaxie et/ou le traitement de l'anxiété.

37. Utilisation d'au moins un composé selon une ou plusieurs des revendications 1 à 8 pour la fabrication d'un médicament pour la prophylaxie et/ou le traitement de la dépression.

38. Utilisation d'au moins un composé selon une ou plusieurs des revendications 1 à 8 pour la fabrication d'un médicament pour la prophylaxie et/ou le traitement des troubles bipolaires.

39. Utilisation d'au moins un composé selon une ou plusieurs des revendications 1 à 8 pour la fabrication d'un médicament pour la prophylaxie et/ou le traitement des troubles de la mémoire cognitive.

40. Utilisation d'au moins un composé selon une ou plusieurs des revendications 1 à 8 pour la fabrication d'un médicament pour la prophylaxie et/ou le traitement des processus de démence sénile.

41. Utilisation d'au moins un composé selon une ou plusieurs des revendications 1 à 8 pour la fabrication d'un médicament pour la prophylaxie et/ou le traitement de la maladie d'Alzheimer.

42. Utilisation d'au moins un composé selon une ou plusieurs des revendications 1 à 8 pour la fabrication d'un médicament pour la prophylaxie et/ou le traitement de la maladie de Parkinson.

43. Utilisation d'au moins un composé selon une ou plusieurs des revendications 1 à 8 pour la fabrication d'un médicament pour la prophylaxie et/ou le traitement de la maladie de Huntington.

44. Utilisation d'au moins un composé selon une ou plusieurs des revendications 1 à 8 pour la fabrication d'un médicament pour la prophylaxie et/ou le traitement des démences dans lesquelles un déficit cognitif prédomine.

45. Utilisation d'au moins un composé selon une ou plusieurs des revendications 1 à 8 pour la fabrication d'un médicament pour la prophylaxie et/ou le traitement de la sclérose en plaques.

46. Utilisation d'au moins un composé selon une ou plusieurs des revendications 1 à 8 pour la fabrication d'un médicament pour la prophylaxie et/ou le traitement de la psychose.

47. Utilisation d'au moins un composé selon une ou plusieurs des revendications 1 à 8 pour la fabrication d'un médicament pour la prophylaxie et/ou le traitement de l'hyperkinésie infantile (ADHD, trouble de déficit d'attention/hyperactivité).

48. Utilisation d'au moins un composé selon une ou plusieurs des revendications 1 à 8 pour la fabrication d'un médicament pour la prophylaxie et/ou le traitement des troubles du système nerveux central.

49. Utilisation d'au moins un composé selon une ou plusieurs des revendications 1 à 8 pour la fabrication d'un médicament pour la prophylaxie et/ou le traitement de la schizophrénie.

50. Utilisation d'au moins un composé selon une ou plusieurs des revendications 1 à 8 pour la fabrication d'un médicament pour l'amélioration cognitive.

51. Médicament comportant au moins un composé selon une ou plusieurs des revendications 9 à 14 et facultativement au moins un ou plusieurs excipients pharmacologiquement acceptables.

52. Médicament selon la revendication 51 pour la régulation des récepteurs 5-HT₆, pour la prophylaxie et/ou le traitement d'une maladie ou d'un trouble lié à la consommation d'aliments, pour la prophylaxie et/ou le traitement de l'obésité, de la boulimie, de l'anorexie, de la cachexie ou du diabète de type II (diabète sucré non insulino-dépendant), pour la prophylaxie et/ou le traitement des troubles du tractus gastro-intestinal, pour l'amélioration cognitive, pour la prophylaxie et/ou le traitement des troubles du système nerveux central, de l'anxiété, de la panique, de la dépression, des troubles bipolaires, des troubles de la mémoire cognitive, des processus de démence sénile, des troubles neurodégénératifs, de la schizophrénie, de la psychose ou de l'hyperkinésie infantile (ADHD, trouble de déficit d'attention/hyperactivité).

53. Médicament selon la revendication 52, dans lequel la maladie ou le trouble lié à la consommation d'aliments est choisi parmi le groupe constitué de la régulation de l'appétit et du maintien, de l'augmentation ou de la diminution du poids corporel.

54. Médicament selon la revendication 52, dans lequel le diabète de type II est un diabète de type II dû à l'obésité.

55. Médicament selon la revendication 52, dans lequel le trouble du tractus gastro-intestinal est le syndrome du côlon irritable.

56. Médicament selon la revendication 52, dans lequel le trouble neurodégénératif est choisi parmi le groupe constitué de la maladie d'Alzheimer, de la maladie de Parkinson, de la maladie de Huntington et/ou de la sclérose en plaques.

57. Utilisation d'au moins un composé selon une ou plusieurs des revendications 9 à 14 pour la fabrication d'un médicament pour la régulation des récepteurs 5-HT₆.

58. Utilisation d'au moins un composé selon une ou plusieurs des revendications 9 à 14 pour la fabrication d'un médicament pour la prophylaxie et/ou le traitement d'une maladie ou d'un trouble lié à la consommation d'aliments.

59. Utilisation d'au moins un composé selon une ou plusieurs des revendications 9 à 14 pour la fabrication d'un médicament pour la régulation de l'appétit.

60. Utilisation d'au moins un composé selon une ou plusieurs des revendications 9 à 14 pour la fabrication d'un médicament pour le maintien, l'augmentation ou la diminution du poids corporel.

61. Utilisation d'au moins un composé selon une ou plusieurs des revendications 9 à 14 pour la fabrication d'un médicament pour la prophylaxie et/ou le traitement de l'obésité.

62. Utilisation d'au moins un composé selon une ou plusieurs des revendications 9 à 14 pour la fabrication d'un médicament pour la prophylaxie et/ou le traitement de la boulimie.

63. Utilisation d'au moins un composé selon une ou plusieurs des revendications 9 à 14 pour la fabrication d'un médicament pour la prophylaxie et/ou le traitement de l'anorexie.

64. Utilisation d'au moins un composé selon une ou plusieurs des revendications 9 à 14 pour la fabrication d'un médicament pour la prophylaxie et/ou le traitement de la cachexie.

65. Utilisation d'au moins un composé selon une ou plusieurs des revendications 9 à 14 pour la fabrication d'un médicament pour la prophylaxie et/ou le traitement du diabète de type II (diabète sucré non insulino-dépendant).

66. Utilisation selon la revendication 65, dans laquelle le diabète de type II est le diabète de type II dû à l'obésité.

67. Utilisation d'au moins un composé selon une ou plusieurs des revendications 9 à 14 pour la fabrication d'un médicament pour la prophylaxie et/ou le traitement des troubles du tractus gastro-intestinal.

68. Utilisation d'au moins un composé selon une ou plusieurs des revendications 9 à 14 pour la fabrication d'un médicament pour la prophylaxie et/ou le traitement du syndrome du côlon irritable.

69. Utilisation d'au moins un composé selon une ou plusieurs des revendications 9 à 14 pour la fabrication d'un médicament pour la prophylaxie et/ou le traitement de l'anxiété.

70. Utilisation d'au moins un composé selon une ou plusieurs des revendications 9 à 14 pour la fabrication d'un médicament pour la prophylaxie et/ou le traitement de la dépression.

71. Utilisation d'au moins un composé selon une ou plusieurs des revendications 9 à 14 pour la fabrication d'un médicament pour la prophylaxie et/ou le traitement des troubles bipolaires.

72. Utilisation d'au moins un composé selon une ou plusieurs des revendications 9 à 14 pour la fabrication d'un médicament pour la prophylaxie et/ou le traitement des troubles de la mémoire cognitive.

73. Utilisation d'au moins un composé selon une ou plusieurs des revendications 9 à 14 pour la fabrication d'un médicament pour la prophylaxie et/ou le traitement des processus de démence sénile.

74. Utilisation d'au moins un composé selon une ou plusieurs des revendications 9 à 14 pour la fabrication d'un médicament pour la prophylaxie et/ou le traitement de la maladie d'Alzheimer.

75. Utilisation d'au moins un composé selon une ou plusieurs des revendications 9 à 14 pour la fabrication d'un médicament pour la prophylaxie et/ou le traitement de la maladie de Parkinson.

76. Utilisation d'au moins un composé selon une ou plusieurs des revendications 9 à 14 pour la fabrication d'un médicament pour la prophylaxie et/ou le traitement de la maladie de Huntington.

77. Utilisation d'au moins un composé selon une ou plusieurs des revendications 9 à 14 pour la fabrication d'un médicament pour la prophylaxie et/ou le traitement de la sclérose en plaques.

78. Utilisation d'au moins un composé selon une ou plusieurs des revendications 9 à 14 pour la fabrication d'un médicament pour la prophylaxie et/ou le traitement des démences dans lesquelles un déficit cognitif prédomine.

79. Utilisation d'au moins un composé selon une ou plusieurs des revendications 9 à 14 pour la fabrication d'un médicament pour la prophylaxie et/ou le traitement de la psychose.

80. Utilisation d'au moins un composé selon une ou plusieurs des revendications 9 à 14 pour la fabrication d'un médicament pour la prophylaxie et/ou le traitement de l'hyperkinésie infantile (ADHD, trouble de déficit d'attention/hyperactivité).

81. Utilisation d'au moins un composé selon une ou plusieurs des revendications 9 à 14 pour la fabrication d'un médicament pour la prophylaxie et/ou le traitement des troubles du système nerveux central.

82. Utilisation d'au moins un composé selon une ou plusieurs des revendications 9 à 14 pour la fabrication d'un médicament pour la prophylaxie et/ou le traitement de la schizophrénie.

83. Utilisation d'au moins un composé selon une ou plusieurs des revendications 9 à 14 pour la fabrication d'un médicament pour l'amélioration cognitive.
